# EUROPEAN PATENT APPLICATION

(11) **EP 0 653 208 A2**
(43) Date of publication of application: **17.05.1995**
(21) Application number: 94203210.3
(22) Date of filing: 03.11.1994
(51) Int. Cl.: A61K 31/435, A61K 31/445, A61K 31/40, A61K 31/55, A61K 31/135

(54) **Pharmaceutical agents for treatment or prevention of sunburn**

(30) Priority: 17.11.1993 US 153682
(71) Applicant: PFIZER INC., New York, N.Y. 10017 (US)
(72) Inventor: Hess, Hans-Jurgen Ernst, Old Lyme, Connecticut 06371 (US); Nagahisa, Atsushi, Mizuho-ku, Nagoya 467 (JP)
(74) Representative: Moore, James William, Dr.

(57) **Abstract**

The present invention relates to the use of certain quinuclidine derivatives, piperidine derivatives, azanorbornane derivatives and related compounds, for the manufacture of a medicament for the treatment or prevention of sunburn in a mammal (including a human being).

## Description

The present invention relates to the use of certain quinuclidine derivatives, piperidine derivatives, azanorbornane derivatives, ethylene diamine derivatives and related compounds, for the manufacture of a medicament for the treatment or prevention of sunburn in a mammal. The pharmaceutically active compounds employed in this invention are substance P receptor antagonists.

The following references refer, collectively, to quinuclidine, piperidine, and azanorbornane derivatives and related compounds that exhibit activity as substance P receptor antagonists: United States Patent 5,162,339, which issued on November 11, 1992; United States Patent 5,232,929, which issued on August 3, 1993; PCT Patent Publication WO91/18899, published December 12, 1992; PCT Patent Publication WO92/01688, published February 6, 1992; PCT Patent Publication WO92/06079, published April 16, 1992; PCT Patent Publication WO92/15585 published September 17, 1992; PCT Patent Publication WO93/00331 published January 7, 1993; PCT Patent Publication WO92/21677 published December 10, 1992; PCT Patent Publication WO93/00330 published January 7, 1993; PCT Patent Publication WO93/06099 published April 1, 1993; PCT Patent Publication WO93/10073 published May 27,1993; PCT Patent Publication WO92/20676 published November 26,1992; PCT Patent Application PCT/US93/01810 filed March 5, 1993; PCT Patent Application PCT/US93/04063 filed May 5, 1993; PCT Patent Application PCT/US93/01429 filed February 23, 1993; PCT Patent Application PCT/US93/05077 filed June 3, 1993; United States Patent Application 988,653, filed December 10, 1992; United States Patent Application 026,382, filed March 4, 1993; and United States Patent Application 123,306, filed September 17, 1993.

F. Gillardon et al., 5th Interscience World Conference on Inflammation, Antirheumatics, Analgesics, Immunomodulators, Abstract 129, published April 25, 1993 refers to the use of the substance P antagonist (2S,3S)-N-(2-methoxyphenyl)methyl-2-diphenylmethyl-1-azabicyclo[2.2.2] octan-3-amine to inhibit ear swelling following exposure to ultra-violet radiation.

### Summary of the Invention

This invention relates to the use of a compound that is a substance P receptor antagonist, except (2S,3S)-N-(2-methoxyphenyl)methyl-2-diphenylmethyl-1-azabicyclo[2.2.2]octan-3-amine, or a pharmaceutically acceptable salt thereof, for the manufacture of a medicament for the treatment or prevention of sunburn in a mammal (including a human being).

This invention also relates, for the said use, to a compound of the formula 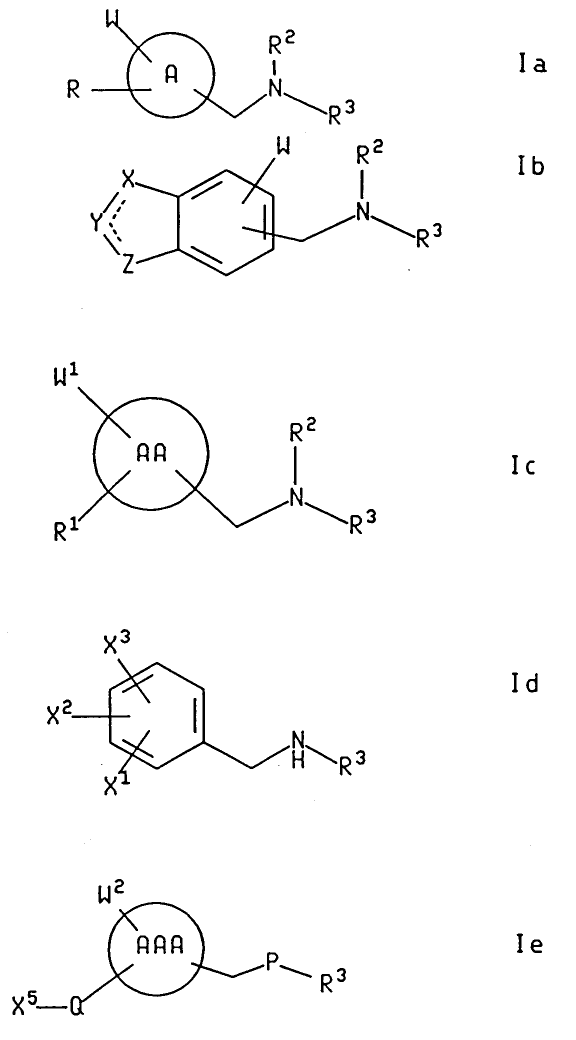
wherein A is a ring system selected from phenyl, naphthyl, thienyl, quinolinyl and indolinyl, and wherein the sidechain containing NR²R³ is attached to a carbon atom of ring system A;
AA is an aryl group selected from phenyl, naphthyl, thienyl, dihydroquinolinyl and indolinyl, and wherein the sidechain containing NR²R³ is attached to a carbon atom of AA;
AAA is an aryl group selected from phenyl, naphthyl, thienyl, dihydroquinolinyl and indolinyl, and wherein the - CH₂PR³ sidechain is attached to a carbon atom of ring A;
P is NR², O, S, SO or SO₂;
Q is SO₂, NH, 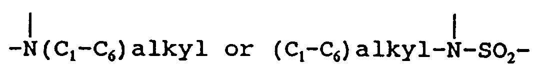
wherein the point of attachment of said 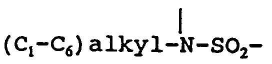
to ring A is the nitrogen atom and the point of attachment to R¹ is the sulfur atom;
W² is hydrogen, (C₁-C₆)alkyl, S-(C₁-C₃)alkyl, halo or (C₁-C₆)alkoxy optionally substituted with from one to three fluorine atoms;
W¹ is hydrogen, halo, (C₁-C₆) alkyl, S-(C₁-C₃)alkyl, halo or (C₁-C₆) alkoxy optionally substituted with from one to three fluorine atoms;
W is hydrogen, (C₁-C₆)alkyl optionally substituted with from one to three fluorine atoms, -S(O)ᵥ-(C₁-C₆) alkyl wherein v is zero, one or two, halo or (C₁-C₆)alkoxy optionally substituted with from one to three fluorine atoms;
R is a 4, 5 or 6 membered heterocyclic ring containing from one to three heteroatoms selected from oxygen, nitrogen and sulfur (e.g., thiazolyl, azetidinyl, pyrrolyl, pyrazolyl, 1,2,3-triazolyl, 1,2,4-triazolyl, isothiazolyl, imidazolyl, isoxazolyl, or oxazolyl) wherein said heterocyclic ring may contain from zero to three double bonds and may optionally be substituted with one or more substituents, preferably one or two substituents, independently selected from (C₁-C₆) alkyl optionally substituted with from one to three fluorine atoms and (C₁-C₆) alkoxy optionally substituted with from one to three fluorine atoms;
R¹ is selected from amino, (C₁-C₆) alkylamino, di-(C₁-C₆)alkylamino, -S(O)ᵥ-(C₁-C₁₀)-alkyl wherein v is zero, one or two, -S(O)ᵥ-aryl wherein v is zero, one or two, -O-aryl, -SO₂NR⁴R⁵ wherein each of R⁴ and R⁵ is, independently, (C₁-C₆)alkyl, or R⁴ and R⁵, together with the nitrogen to which they are attached, form a saturated ring containing one nitrogen and from 3 to 6 carbons, 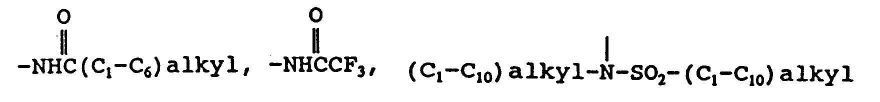
wherein one or both of the alkyl moieties may optionally be substituted with from one to three fluorine atoms, -N(SO₂-(C₁-C₁₀)alkyl)₂ and 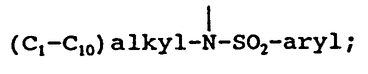
and wherein the aryl moieties of said -S(O)ᵥ-aryl, -O-aryl and 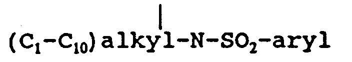
are independently selected from phenyl and benzyl and may optionally be substituted with from one to three substituents independently selected from (C₁-C₄)alkyl, (C₁-C₄)alkoxy and halo;
or R¹ is a group having the formula 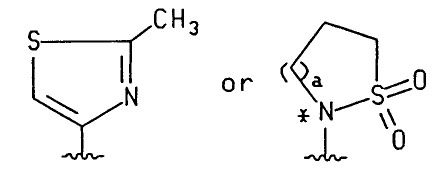
wherein a is 0, 1 or 2 and the asterisk represents a position meta to the R²R³NCH₂ side chain;
the dotted lines in formula Ib represent that one of the X-Y and Y-Z bonds may optionally be a double bond;
X is selected from =CH-, -CH₂-, -O-, -S-, -SO-, -SO₂-, -N(R⁴)-, -NH-, =N-, -CH[(C₁-C₆)alkyl]-, =C[(C₁-C₆)alkyl]-, -CH(C₆H₅)- and =C(C₆H₅)-;
X¹ is hydrogen, (C₁-C₁₀) alkoxy optionally substituted with from one to three fluorine atoms or (C₁-C₁₀)alkyl optionally subsituted with from one to three fluorine atoms;
X² and X³ are independently selected from hydrogen, halo, nitro, (C₁-C₁₀)alkyl optionally substituted with from one to three fluorine atoms, (C₁-C₁₀)alkoxy optionally substituted with from one to three fluorine atoms, trifluoromethyl, hydroxy, phenyl, cyano, amino, (C₁-C₆) alkylamino, di-(C₁-C₆)alkylamino, 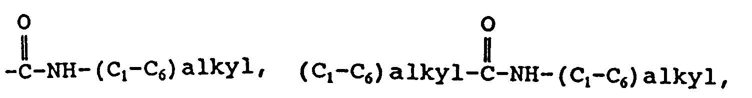
hydroxy (C₁-C₄)alkyl, (C₁-C₄)alkoxy, (C₁-C₄)alkyl, 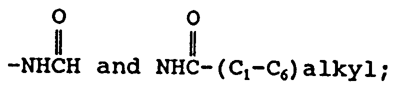
X⁵ is a four to six membered heterocyclic ring containing from one to three heteroatoms selected from sulfur, nitrogen and oxygen (e.g., thiazolyl, pyrrolyl, thienyl, triazolyl, oxazolyl, oxadiazolyl, thiadiazolyl or imidazolyl), wherein said heterocyclic ring may optionally be susbtituted with from one to three substituents, preferably with from zero to two substituents, independently selected from phenyl, (C₁-C₆)alkyl optionally substituted with from one to three fluorine atoms, (C₁-C₆)alkoxy optionally substituted with from one to three fluorine atoms and halo;
Y is selected from >C=O, >C=NR⁴, >C=S, =CH-, -CH₂-, =C[(C₁-C₆)alkyl]-, -CH[(C₁-C₆)alkyl]-, =C(C₆H₅)-, -CH(C₆H₅)-, =N-, -NH-, -N(R⁴)-, =C(halo)-, =C(OR⁴)-, =C(SR⁴)-, =C(NR⁴)-, - O-, -S- and SO₂, wherein the phenyl moieties of said =C(C₆H₅)-and -CH(C₆H₅)- may optionally be substituted with from one to three substituents independently selected from trifluoromethyl and halo, and wherein the alkyl moieties of said =[(C₁-C₆)alkyl]- and -CH[C₁-C₆)alkyl]- may optionally be substituted with from one to three fluorine atoms;
Z is selected from =CH-, -CH₂-, =N-, -NH-, -S-, -N(R⁴)-, =C(C₆H₅)-, -CH(C₆H₅)-, =C[(C₁-C₆) alkyl]- and -CH[(C₁-C₆)alkyl]-;
or X, Y and Z, together with the two carbon atoms shared between the benzo ring and the XYZ ring, form a fused pyridine or pyrimidine ring;
R⁴ is (C₁-C₆) alkyl or phenyl;
R² is hydrogen or -CO₂(C₁-C₁₀)alkyl;
R³ is selected from 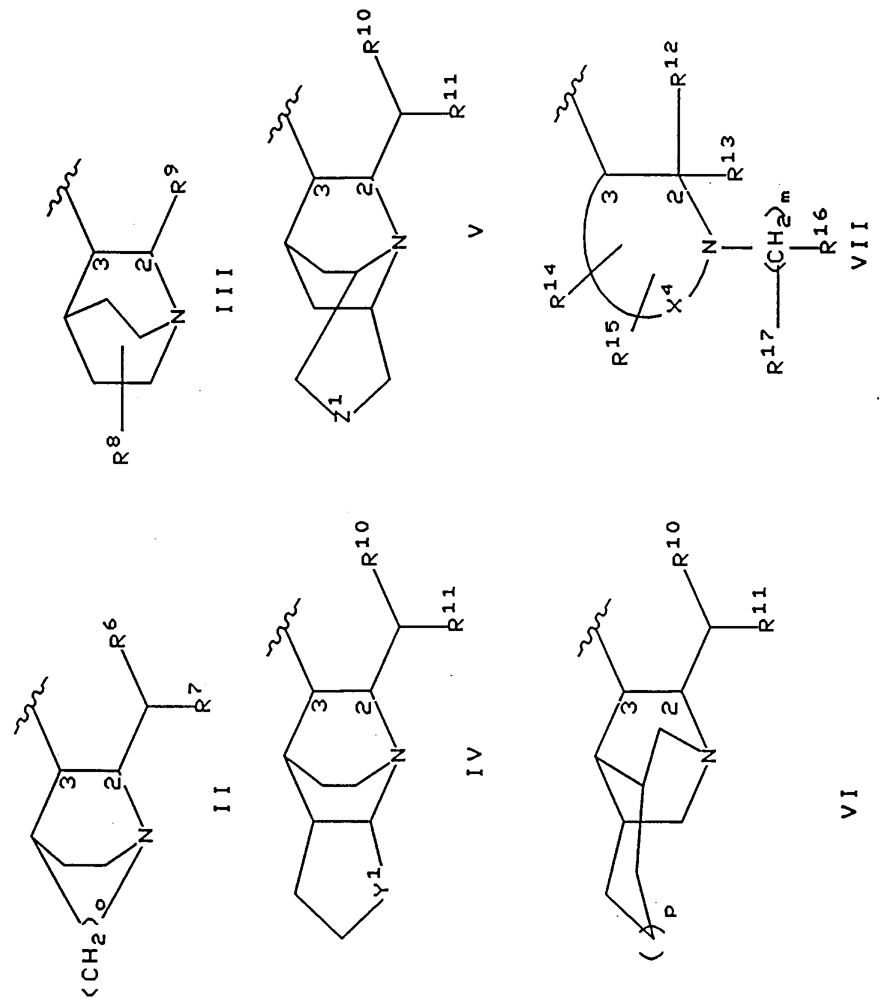 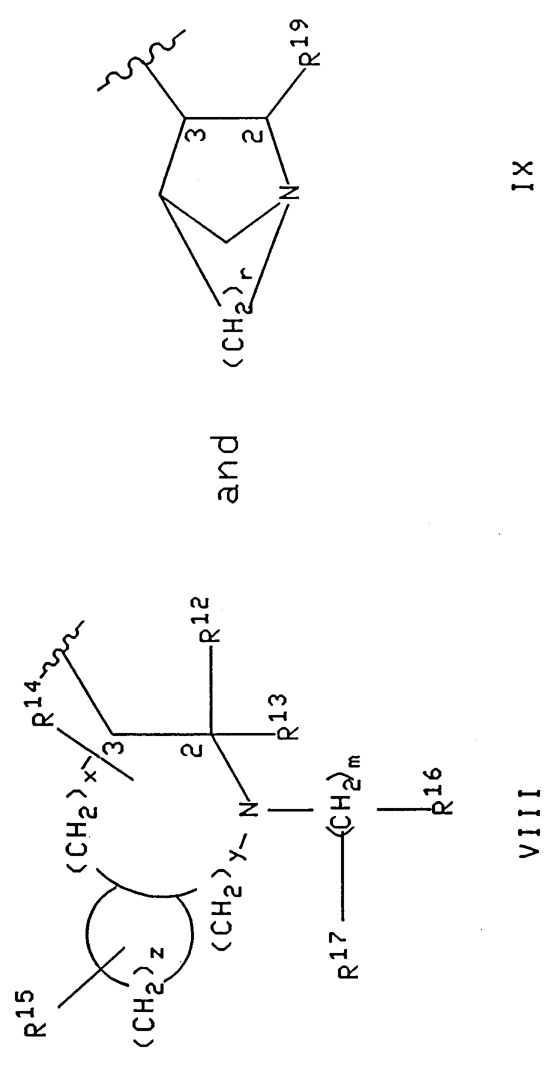
wherein R⁶ and R¹⁰ are independently selected from furyl, thienyl, pyridyl, indolyl, biphenyl and phenyl, wherein said phenyl may optionally be substituted with one or two substituents independently selected from halo, (C₁-C₁₀) alkyl optionally substituted with from one to three fluorine atoms, (C₁-C₁₀) alkoxy optionally substituted with from one to three fluorine atoms, carboxy, benzyloxycarbonyl and (C₁-C₃) alkoxy-carbonyl;
R⁷ is selected from (C₃-C₄) branched alkyl, (C₅-c₆) branched alkenyl, (C₅-C₇) cycloalkyl, and the radicals named in the definition of R⁶;
R⁸ is hydrogen or (C₁-C₆) alkyl;
R⁹ and R¹⁹ are independently selected from phenyl, biphenyl, naphthyl, pyridyl, benzhydryl, thienyl and furyl, and R⁹ and R¹⁹ may optionally be substituted with from one to three substituents independently selected from halo, (C₁-C₁₀) alkyl optionally substituted with from one to three fluorine atoms and (C₁-C₁₀) alkoxy optionally substituted with from one to three fluorine atoms;
Y¹ is (CH₂)ₗ wherein l is an integer from one to three, or Y¹ is a group of the formula 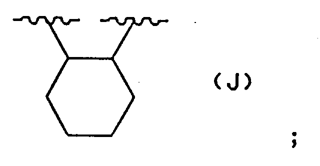
Z¹ is oxygen, sulfur, amino, (C₁-C₃)alkylamino or (CH₂)ₙ wherein n is zero, one or two;
x is an integer from zero to four;
y is an integer from zero to four;
z is an integer from one to six, wherein the ring containing (CH₂)_{z} may contain from zero to three double bonds, and one of the carbons of (CH₂)_{z} may optionally be replaced by oxygen, sulfur or nitrogen;
o is two or three;
p is zero or one;
r is one, two or three;
R¹¹ is thienyl, biphenyl or phenyl optionally substituted with one or two substituents independently selected from halo, (C₁-C₁₀) alkyl optionally substituted with from one to three fluorine atoms and (C₁-C₁₀) alkoxy optionally substituted with from one to three fluorine atoms;
X⁴ is (CH₂)_{q} wherein q is an integer from 1 to 6, and wherein any one of the carbon-carbon single bonds in said (CH₂)_{q} may optionally be replaced by a carbon-carbon double bond, and wherein any one of the carbon atoms of said (CH₂)_{q} may optionally be substituted with R¹⁴, and wherein any one of the carbon atoms of said (CH₂)_{q} may optionally be substituted with R¹⁵;
m is an integer from 0 to 8, and any one of the carbon-carbon single bonds of (CH₂)ₘ, wherein both carbon atoms of such bond are bonded to each other and to another carbon atom in the (CH₂)ₘ chain, may optionally be replaced by a carbon-carbon double bond or a carbon-carbon triple bond, and any one of the carbon atoms of said (CH₂)ₘ may optionally be substituted with R¹⁷;
R¹² is a radical selected from hydrogen, (C₁-C₆) straight or branched alkyl, (C₃-C₇)cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl-(C₂-C₆)alkyl, benzhydryl and benzyl, wherein the point of attachment on R¹² is a carbon atom unless R¹² is hydrogen, and wherein each of said aryl and heteroaryl groups and the phenyl moieties of said benzyl, phenyl-(C₂-C₆) alkyl and benzhydryl may optionally be substituted with one or more substituents independently selected from halo, nitro, (C₁-C₁₀) alkyl optionally substituted with from one to three fluorine atoms, (C₁-C₁₀)alkoxy optionally substituted with
from one to three fluorine atoms, amino, hydroxy-(C₁-C₆)alkyl, (C₁-C₆)alkoxy-(C₁-C₆)alkyl, (C₁-C₆)-alkylamino, 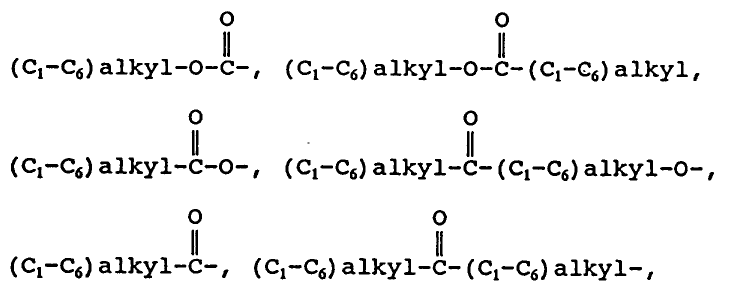
di-(C₁-C₆)alkylamino, 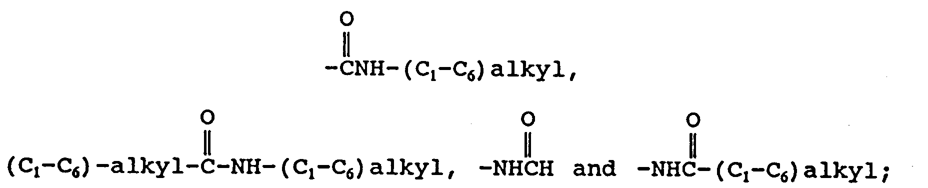
and wherein one of the phenyl moieties of said benzhydryl may optionally be replaced by naphthyl, thienyl, furyl or pyridyl;
R¹³ is hydrogen, phenyl or (C₁-C₆)alkyl;
or R¹² and R¹³, together with the carbon to which they are attached, form a saturated carbocyclic ring having from 3 to 7 carbon atoms wherein one of said carbon atoms that is neither the point of attachment of the spiro ring nor adjacent to it may optionally be replaced by oxygen, nitrogen or sulfur;
R¹⁴ and R¹⁵ are each independently selected from hydrogen, hydroxy, halo, amino, oxo (=O), cyano, hydroxy-(C₁-C₆)alkyl, (C₁-C₆)alkoxy-(C₁-C₆)alkyl, (C₁-C₆)alkylamino, di-(C₁-C₆)alkylamino, (C₁-C₆)alkoxy, 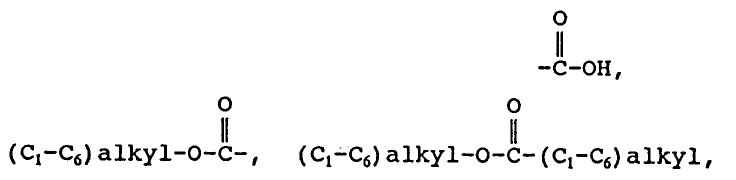 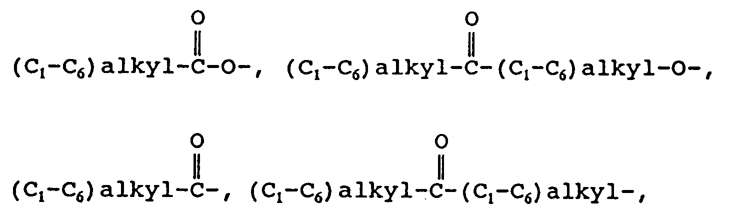
and the radicals set forth in the definition of R¹²;
R¹⁶ is 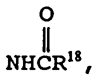
NHCH₂R¹⁸, SO₂R¹⁸, GR²⁰ CO₂H or one of the radicals set forth in any of the definitions of R¹², R¹⁴ and R¹⁵;
R¹⁷ is oximino (=NOH) or one of the radicals set forth in any of the definitions of R¹², R¹⁴ and R¹⁵; and
R¹⁸ is (C₁-C₆) alkyl, hydrogen, phenyl or phenyl (C₁-C₆)alkyl;
G is selected from the group consisting of CH₂, nitrogen, oxygen, sulfur and carbonyl;
R²⁰ is a monocyclic or bicyclic heterocycle selected from the group consisting of pyrimidinyl, benzoxazolyl, 2,3-dihydro-3-oxobenzisosulfonazol-2-yl, morpholin-1-yl, thiomorpholin-1-yl, benzofuranyl, benzothienyl, indolyl, isoindolyl, isoquinolinyl, furyl, pyridyl, isothiazolyl, oxazolyl, triazolyl, tetrazolyl, quinolyl, thiazolyl, thienyl, and groups of the formulae 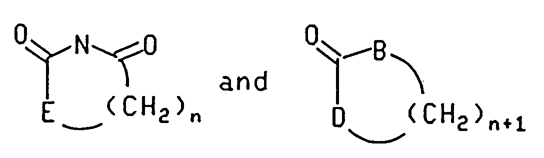
wherein B and D are selected from carbon, oxygen, and nitrogen, and at least one of B and D is other than carbon; E is carbon or nitrogen; n is an integer from 1 to 5; and any one of the carbons of the (CH₂)n or (CH₂)ₙ₊₁ may be optionally substituted with (C₁-C₆) alkyl or (C₂-C₆) spiroalkyl, and either any two of the carbon atoms of said (CH₂)ₙ and (CH₂)ₙ₊₁ may be bridged by a one or two carbon atom linkage, or any one pair of adjacent carbons of said (CH₂)ₙ and (CH₂)ₙ₊₁ may form, together with from one to three carbon atoms that are not members of the carbonyl containing ring, a (C₃-C₅) fused carbocyclic ring;
with the proviso that (a) when m is 0, one of R¹⁶ and R¹⁷ is absent and the other is hydrogen, (b) when R³ is a group of the formula VIII, R¹⁴ and R¹⁵ cannot be attached to the same carbon atom, (c) when R¹⁴ and R¹⁵ are attached to the same carbon atom, then either each of R¹⁴ and R¹⁵ is independently selected from hydrogen, fluoro, (C₁-C₆)alkyl, hydroxy-(C₁-C₆)alkyl and (C₁-C₆)alkoxy-(C₁-C₆)alkyl, or R¹⁴ and R¹⁵, together with the carbon to which they are attached, form a (C₃-C₆) saturated carbocyclic ring that forms a spiro compound with the nitrogen-containing ring to which they are attached; (d) R¹² and R¹³ cannot both be hydrogen; (e) when R¹⁴ or R¹⁵ is attached to a carbon atom of X⁴ or (CH₂)_{y} that is adjacent to the ring nitrogen, then R¹⁴ or R¹⁵, respectively, must be a substituent wherein the point of attachment is a carbon atom; (f) neither R¹⁴, R¹⁵, R¹⁶ nor R¹⁷ can form a ring with R¹³; and (g) the compound of formula Ia cannot be (2S,3S)-N-(2-methoxyphenyl)methyl-2-diphenylmethyl-1-azabicyclo[2.2.2]octan-3-amine;
or a pharmaceutically acceptable salt thereof.

The fused bicyclic nucleus of compounds of the formula Ib to which W and the -CN₂NR²R³ sidechain are attached may be, but is not limited to one of the following groups: benzoxazolyl, benzthiazolyl, benzimidazolyl, benzisoxazolyl, benzoisothiazolyl, indazolyl, indolyl, isoquinolinyl, benzofuryl, benzothienyl, oxindolyl, benzoxazolinonyl, benzthiazolinonyl, benzimidazolinonyl, benzimidazoliniminyl, dihydrobenzothienyl-S,S-dioxide, benztriazolyl, benzthiadiazolyl, benzoxadiazolyl, and quinazolinyl.

Preferred embodiments of this invention include, for the said use,
a compound as defined in paragraphs (1) through (47-A) below, or a pharmaceutically acceptable salt of such compound.
(1) A compound of the formula Ia or Ib wherein the substituents at positions "2" and "3" of the nitrogen containing ring of R³ are in a cis configuration. (When R³ is a group of the formula VII or VIII, "a cis configuration", as used herein, means that the non-hydrogen substituent at position "3" is cis to R¹²).
(2) A compound of the formula Ia wherein R³ is a group of the formula III, VII or IX; R² is hydrogen; A is phenyl or indolinyl; W is (C₁-C₃)alkoxy optionally substituted with from one to five fluorine atoms; and R is thiazolyl, imidazolyl, thiadiazolyl, pyrrolyl or oxazolyl, and R may optionally be substituted with one or two (C₁-C₃) alkyl moieties.
(3) A compound of the formula Ib wherein R³ is a group of the formula III, VII or IX; R² is hydrogen; the fused bicyclic ring system to which W and the -CH₂NR²R³ sidechain are attached is benzoxazolyl, benzisoxazolyl, benzthiazolyl or benzimidazolyl; and W is (C₁-C₆)alkoxy optionally substituted with from one to five fluorine atoms.
(4) A compound as defined in paragraph 1, 2 or 3 above wherein: (a) R³ is a group of the formula III and R⁹ is benzhydryl; (b) R³ is a group of the formula VII, R¹² is phenyl, each of R¹³, R¹⁴, R¹⁵ and R¹⁶ is hydrogen, m is zero and X⁴ is -(CH₂)₃-; or (c) R³ is a group of the formula IX, r is two and R¹⁹ is benzhydryl.
(5) A compound of the formula Ia wherein: (a) R³ is a group of the ,formula III wherein the substituents at positions "2" and "3" of the nitrogen containing ring are in the cis configuration, R⁹ is benzhydryl and A is phenyl; or (b) R³ is a group of the formula VII wherein R¹² and the substituent at position "3" of the nitrogen containing ring are in the cis configuration, A is phenyl, R¹² is phenyl, each of R², R¹³, R¹⁴, R¹⁵ and R¹⁶ is hydrogen, m is zero, W is methoxy or isopropoxy, X⁴ is -(CH₂)₃- and R is thiazolyl, imidazolyl, pyrrolyl, oxazolyl or thiadiazolyl.
(6) A compound of the formula Ib wherein R³ is a group of the formula IX wherein the substituents at positions "2" and "3" of the nitrogen containing ring are in the cis configuration, R¹⁹ is benzhydryl, r is two and the fused bicyclic ring system to which W and the -CH₂NR²R³ sidechain are attached is benzisoxazolyl or benzthiazolyl.
(7) A compound of the formula Ib wherein R³ is a group of the formula IX, R¹⁹ is benzhydryl, the fused bicyclic ring system to which W and the -CH₂NR²R³ sidechain are attached is benzisoxazolyl, and W is methoxy.
(8) A compound of the formula Ib wherein R³ is a group of the formula VII, R¹² is phenyl, each of R¹³, R¹⁴, R¹⁵ and R¹⁶ is hydrogen, m is zero, X⁴ is -(CH₂)₃-, and the fused bicyclic ring system to which W and the -CH₂NR²R³ sidechain are attached is benzothiazolyl, benzoxazolyl or benzimidazolyl.
(9) A compound of the formula Ia wherein R³ is a group of the formula VII, each of R¹³, R¹⁴, R¹⁵ and R¹⁶ is hydrogen, m is zero, X⁴ is -(CH₂)₃-, A is phenyl, W is methoxy, and R is selected from thiazolyl, imidazolyl, thiadiazolyl and isoxazolyl.
(10) A compound of the formula Ia or Ib that is selected from:
   (2S,3S)-3-[2-methoxy-5-(2-thiazolyl)benzyl]amino-2-phenylpiperidine;
   (2S,3S)-3-[5-(2-imidazolyl)-2-methoxybenzyl]amino-2-phenylpiperidine;
   (2S,3S)-3-[2-methoxy-5-(2-oxopyrrolidinyl)benzyl]amino-2-phenylpiperidine;
   (2S,3S)-3-[2-methoxy-5-(4-methyl-2-thiazolyl)benzyl]amino-2-phenylpiperidine;
   (2S,3S)-3-[2-methoxy-5-(1,2,3-thiadiazol-4-yl)benzyl]amino-2-phenylpiperidine;
   (2S,3S)-(6-methoxy-2-methyl-benzothiazol-5-ylmethyl)-(2-phenylpiperidin-3-yl)amine;
   (2S,3S)-[5-(2,5-dimethyl-pyrrol-1-yl)-2-methoxybenzyl]-(2-phenylpiperidin-3-yl)amine;
   (2S,3S)-3-[2-methoxy-5-(5-oxazolyl)benzyl]amino-2-phenylpiperidine;
   (2S,3S)-(6-methoxy-2-methyl-benzoxazol-5-ylmethyl)-(2-phenyl-piperidin-3-yl)-amine; and
   (1SR,2SR,3SR,4RS)-3-[6-methoxy-3-methylbenzisoxazol-5-yl]methylamino-2-benzhydrylazanorbornane.
(11) A compound of the formula Ic, wherein R³ is a group of the formula II, III, VII or IX; R² is hydrogen; ring AA is phenyl or indolinyl; W¹ is (C₁-C₃) alkoxy optionally substituted with from one to three fluorine atoms; and R¹ is S(O)ᵥ-(C₁-C₁₀)alkyl wherein v is zero, one or two, S(O)ᵥ-aryl wherein v is zero, one or two, -O-aryl, 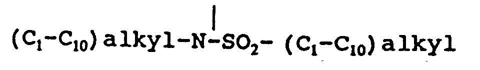 wherein one or both of the alkyl moieties may optionally be substituted with from one to three fluorine atoms, -N(SO₂-(C₁-C₁₀)alkyl)₂ or 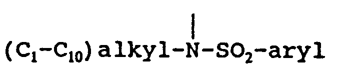 wherein said aryl is phenyl or benzyl and may optionally be substituted with from one to three substituents independently selected from (C₁-C₄)alkyl, (C₁-C₄)alkoxy and halo.
(12) A compound as defined in paragraph 11 above, wherein R³ is a group of the formula II, o is two, and each R⁶ and R⁷ is phenyl.
(13) A compound as defined in paragraph 11 above, wherein R³ is a group of the formula VII, each of R¹³, R¹⁴, R¹⁵ and R¹⁶ is hydrogen, R¹² is phenyl, m is zero and X⁴ is -(CH₂)₃-.
(14) A compound as defined in paragraph 11 above, wherein R³ is a group of the formula IX, R¹⁹ is benzhydryl and r is two.
(15) A compound as defined in paragraph 11 above, wherein R³ is a group of the formula III, R⁸ is other than hydrogen and R⁹ is benzyhydryl.
(16) A compound to the formula Ic wherein the substituents at positions "2" and "3" of the nitrogen containing ring are in the cis configuration.
(17) A compound of the formula 1c wherein R³ is a group of the formula II wherein the substituents at positions "2" and "3" of the nitrogen containing ring are in the cis configuration, o is two, each of R⁶ and R⁷ is phenyl and ring AA is phenyl or indolinyl.
(18) A compound of the formula Ic wherein R³ is a group of the formula III wherein the substituents at positions "2" and "3" of the nitrogen containing ring are in the cis configuration, R⁸ is other than hydrogen, R⁹ is benzhydryl and ring AA is phenyl.
(19) A compound of the formula Ic wherein R³ is a group of the formula VII wherein R¹² and the substituent at position "3" of the nitrogen containing ring are in the cis configuration, ring AA is phenyl, R¹² is phenyl, each of R², R¹³, R¹⁴, R¹⁵ and R¹⁶ is hydrogen, m is zero, X⁴ is -(CH₂)₂- or -(CH₂)₃- and R¹ is selected from S(O)ᵥ-(C₁-C₁₀)alkyl wherein v is zero, one or two, and 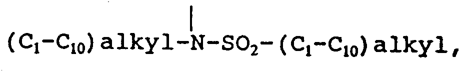 and di-(C₁-C₆)alkylamino.
(20) A compound as defined in paragraph 19 above, wherein X⁴ is -(CH₂)₂- and W¹ is (C₁-C₆) alkoxy optionally substituted with from one to three fluorine atoms.
(21) A compound as defined in paragraph 19 above, wherein X⁴ is -(CH₂)₃- and W¹ is (C₁-C₆) alkoxy optionally substituted with from one to three fluorine atoms.
(22) A compound of the formula Ic, wherein R³ is a group of the formula IX wherein the substituents at positions "2" and "3" of the nitrogen containing ring are in the cis configuration, r is two and R¹⁹ is benzhydryl.
(23) A compound as defined in paragraph 22 above, wherein ring AA is phenyl, W¹ is (C₁-C₅) alkoxy optionally substituted with from one to three fluorine atoms and R¹ is selected from -S(O)ᵥ-(C₁-C₁₀) alkyl wherein v is zero, one or two, di-(C₁-C₆)alkylamino and 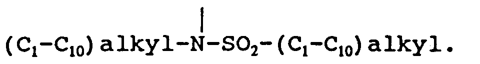
(24) A compound as defined in paragraph 15 above, wherein ring AA is phenyl, W¹ is (C₁-C₆)alkoxy optionally substituted with from one to three fluorine atoms, and R¹ is selected from -S(O)ᵥ-(C₁-C₁₀) alkyl wherein v is zero, one or two, and 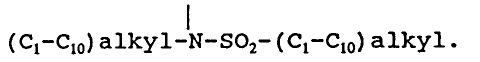
(25) A compound as defined in paragraph 15 above, wherein ring AA is phenyl, W¹ is (C₁-C₆)alkoxy optionally substituted with from one to three fluorine atoms, and R¹ is selected from amino, (C₁-C₆)alkylamino or di-(C₁-C₆)alkylamino.
(26) A compound as defined in paragraph 12 above, wherein ring AA is phenyl, W¹ is (C₁-C₆) alkoxy optionally substituted with from one to three fluorine atoms, and R¹ is selected from -S(O)ᵥ-(C₁-C₁₀)alkyl wherein v is zero, one or two, and 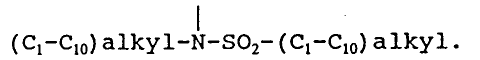
(27) A compound as defined in paragraph 12 above, wherein ring AA is phenyl, W¹ is (C₁-C₆) alkoxy optionally substituted with from one to three fluorine atoms, and R¹ is selected from amino, (C₁-C₆)alkylamino or di-(C₁-C₆)alkylamino.
(28) A compound as defined in paragraph 24 above, wherein W¹ is attached at the "2" position of ring AA and R¹ is attached at the "5" position of ring AA, relative to the point of attachment of the NR²R³ containing side chain.
(29) A compound as defined in paragraph 25 above, wherein W¹ is attached at the "2" position of ring AA and R¹ is attached at the "5" position of ring AA, relative to the point of attachment of the NR²R³ containing side chain.
(30) A compound as defined in paragraph 26 above, wherein W¹ is attached at the "2" position of ring AA and R¹ is attached at the "5" position of ring AA, relative to the point of attachment of the NR²R³ containing side chain.
(31) A compound as defined in paragraph 27 above, wherein W¹ is attached at the "2" position of ring AA and R¹ is attached at the "5" position of ring AA, relative to the point of attachment of the NR²R³ containing side chain.
(32) A compound as defined in paragraph 13 above, wherein ring AA is phenyl, W¹ is selected from isopropoxy, OCF₃, OCH₃, OCHF₂ and OCH₂CF₃, and R¹ is selected from -S(O)ᵥ-(C₁-C₁₀)alkyl wherein v is zero, one or two, and (C₁-C₁₀)alkyl-N-SO₂-(C₁-C₁₀)alkyl.
(33) A compound selected from the group consisting of:
   (2S,3S)-N-(2-methoxy-5-methylsulfonylphenyl)-methyl-2-diphenylmethyl-1-azabicyclo[2.2.2]octan-3-amine;
   (2S,3S)-N-(2-methoxy-5-methylthiophenyl)methyl-2-diphenylmethyl-1-azabicyclo[2.2.2]octan-3-amine;
   (2S,3S)-N-(2-methoxy-5-dimethylaminophenyl)methyl-2-diphenylmethyl-1-azabicyclo[2.2.2]octan-3-amine; and
   (2S,3S)-N-(5-trifluoroacetylamino-2-methoxyphenyl)methyl-2-diphenylmethyl-1-azabicyclo[2.2.2]octan-3-amine.
(34) A compound of the formula Ic, wherein R³ is a group of the formula VII, m is zero, each of R¹³, R¹⁵, R¹⁶ and R¹⁷ is hydrogen, R¹² is phenyl, R¹⁴ is 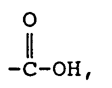 ring AA is phenyl, W¹ is (C₁-C₃)alkoxy and R¹ is selected from (C₁-C₅)alkyl, -SCH₃, SO₂CH₃, SOCH₃, (C₁-C₆)alkylamino and di-(C₁-C₆)alkyl-amino.
(35) A compound of the formula Ic, having the formula 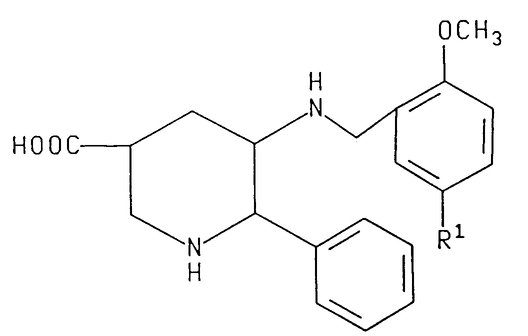
(36) A compound of the formula Id wherein R⁶, R¹⁰, R¹¹ and R¹³ are phenyl, R⁸ is hydrogen, R⁹ is phenyl optionally substituted with chlorine, fluorine, (C₁-C₆) alkyl optionally substituted with from one to three fluorine atoms or (C₁-C₆) alkoxy optionally substituted with from one to three fluorine atoms, m is 0 and n is 3 or 4.
(37) A compound of the formula Id that is selected from the group consisting of:
   (2S,3S)-3-(5-tert-butyl-2-methoxybenzyl)amino-2-(3-trifluoromethoxyphenyl)piperidine;
   (2S,3S)-3-(2-isopropoxy-5-trifluoromethoxybenzyl)amino-2-phenyl-piperidine;
   (2S,3S)-3-(2-ethoxy-5-trifluoromethoxybenzyl)amino-2-phenyl-piperidine;
   (2S,3S)-3-(2-methoxy-5-trifluoromethoxybenzyl)-amino-2-phenylpiperidine;
   (2S,3S)-3(-5-tert-butyl-2-trifluoromethoxybenzyl)amino-2-phenylpiperidine;
   2-(diphenylmethyl)-N-(2-methoxy-5-trifluoromethoxyphenyl)methyl-1-azabicyclo[2.2.2]octan-3-amine;
   (2S,3S)-3-[5-chloro-2-(2,2,2-trifluoroethoxy)benzyl]amino-2-phenylpiperidine;
   (2S,3S)-3-(5-tert-butyl-2-trifluoromethoxybenzyl)amino-2-phenylpiperidine;
   (2S,3S)-3-(2-isopropoxy-5-trifluoromethoxybenzyl)amino-2-phenylpiperidine;
   (2S,3S)-3-(2-difluoromethoxy-5-trifluoromethoxybenzyl)-amino-2-phenylpiperidine;
   (2S,3S)-2-phenyl-3-[2-(2,2,2-trifluoroethoxybenzyl)-aminopiperidine; and
   (2S,3S)-2-phenyl-3-(2-trifluoromethoxybenzyl)]aminopiperidine.
(38) A compound of the formula Id, wherein R³ is a group of the formula II wherein o is two or three and each of R⁶ and R⁷ is phenyl or substituted phenyl.
(39) A compound of the formula Id, wherein R³ is a group of the formula III, R⁸ is hydrogen and R⁹ is phenyl or substituted phenyl.
(40) A compound of the formula Id, wherein R³ is a group of the formula IV wherein l is one or two and each of R¹⁰ and R¹¹ is phenyl or substituted phenyl.
(41) A compound of the formula Id, wherein R³ is a group of the formula V wherein n is zero or one and each of R¹⁰ and R¹¹ is phenyl or substituted phenyl.
(42) A compound of the formula Id, wherein R³ is a group of the formula VI wherein p is one and each of R¹⁰ and R¹¹ are phenyl or substituted phenyl.
(43) A compound of the formula Id, wherein R³ is a group of the formula VII wherein q is two, three or four, m is zero and R¹² is phenyl or substituted phenyl.
(44) A compound of the formula Id, wherein R³ is a group of the formula VIII wherein y is zero, x is zero or one, z is three or four, m is zero and R¹² is phenyl or substituted phenyl.
(45) A compound of the formula Id wherein R³ is a group of the, formula VII, R⁶, R¹⁴, R¹³ R¹⁶ and R¹⁵ are hydrogen, R¹² is phenyl, X¹ is, 2-methoxy, X² and X³ are independently selected from hydrogen, chlorine, fluorine, methyl, (C₁-C₆)alkoxy and trifluoromethane, m is 0 and q is 3 or 4.
(46) A compound of the formula Id wherein R³ is a group of the formula VII and said compound is selected from the group consisting of:
   cis-3-(2-chlorobenzylamino)-2-phenylpiperidine;
   cis-3-(2-trifluoromethylbenzylamino)-2-phenyl-piperidine;
   cis-3-(2-methoxybenzylamino)-2-(2-fluorophenyl)-piperidine;
   cis-3-(2-methoxybenzylamino)-2-(2-chlorophenyl)-piperidine;
   cis-3-(2-methoxybenzylamino)-2-(2-methylphenyl)-piperidine;
   cis-3-(2-methoxybenzylamino)-2-(3-methoxyphenyl)-piperidine;
   cis-3-(2-methoxybenzylamino)-2-(3-fluorophenyl)-piperidine;
   cis-3-(2-methoxybenzylamino)-2-(3-chlorophenyl)-piperidine;
   cis-3-(2-methoxybenzylamino)-2-phenylpiperidine;
   cis-3-(2-methoxybenzylamino)-2-(3-methylphenyl)-piperidine;
   cis-3-(2-methoxybenzylamino)-2-(4-fluorophenyl)-piperidine;
   cis-3-(2-methoxybenzylamino)-2-(3-thienyl)-piperidine;
   cis-3-(2-methoxybenzylamino)-2-phenylazacyclo-heptane;
   3-(2-methoxybenzylamino)-4-methyl-2-phenyl-piperidine;
   3-(2-methoxbenzylamino)-5-methyl-2-phenyl-piperidine;
   3-(2-methoxybenzylamino)-6-methyl-2-phenyl-piperidine;
   (2S,3S)-3-(2-methoxybenzylamino)-2-phenylpiperidine;
   (2S,3S)-1-(5-carboethoxypent-1-yl)-3-(2-methoxybenzylamino)-2-phenylpiperidine;
   (2S,3S)-1-(6-hydroxy-hex-1-yl)-3-(2-methoxybenzylamino)-2-phenylpiperidine;
   (2S,3S)-1-(4-hydroxy-4-phenylbut-1-yl)-3-(2-methoxybenzylamino)-2-phenylpiperidine;
   (2S,3S)-1-(4-oxo-4-phenylbut-1-yl)-3-(2-methoxybenzylamino)-2-phenylpiperidine;
   (2S,3S)-1-(5,6-dihydroxyhex-1-yl)-3-(2-methoxybenzylamino)-2-phenylpiperidine;
   cis-3-(5-fluoro-2-methoxybenzylamino)-2-phenyl-piperidine;
   (2S,3S)-1-[4-(4-fluorophenyl)-4-oxobut-1-yl]-3-(2-methoxybenzylamino)-2-phenylpiperidine;
   (2S,3S)-1-[4-(4-fluorophenyl)-4-hydroxobut-1-yl]-3-(2-methoxybenzylamino)-2-phenylpiperidine;
   cis-3-(2-methoxy-5-methylbenzylamino)-2-phenyl-piperidine;
   (2S,3S)-1-(4-benzamidobut-1-yl)-3-(2-methoxybenzylamino)-2-phenylpiperidine;
   cis-3-(2-methoxynaphth-1-ylmethylamino)-2-phenylpiperidine;
   (2S,3S)-3-(2-methoxybenzylamino)-1-(5-N-methylcarboxamidopent-1-yl)-2-phenylpiperidine;
   (2S,3S)-1-(4-cyanobut-1-yl)-3-(2-methoxybenzylamino)-2-phenylpiperidine;
   (2S,3S)-1-[4-(2-naphthamido)but-1-yl]-3-(2-methoxybenzylamino)-2-phenylpiperidine;
   (2S,3S)-1-(5-benzamidopent-1-yl)-3-(2-methoxybenzylamino)-2-phenylpiperidine;
   (2S,3S)-1-(5-aminopent-1-yl)-3-(2-methoxybenzylamino)-2-phenylpiperidine;
   (2S,3S)-3-(5-chloro-2-methoxybenzylamino)-2-phenylpiperidine;
   (2S,3S)-3-(2,5-dimethoxybenzylamino)-2-phenylpiperidine;
   cis-3-(3,5-difluoro-2-methoxybenzylamino)-2-phenylpiperidine;
   cis-3-(4,5-difluoro-2-methoxybenzylamino)-2-phenylpiperidine;
   cis-3-(2,5-dimethoxybenzylamino)-1-[4-(4-fluorophenyl)-4-oxobut-1-yl]-2-phenylpiperidine;
   cis-3-(5-chloro-2-methoxybenzylamino)-1-(5,6-dihydroxyhex-1-yl)-2-phenylpiperidine;
   cis-1-(5,6-dihydroxyhex-1-yl)-3-(2,5-dimethoxybenzylamino)-2-phenylpiperidine;
   cis-2-phenyl-3-[-2(prop-2-yloxy)benzylamino]piperidine;
   cis-3-(2,5-dimethoxybenzyl)amino-2-(3-methoxyphenyl)piperidine hydrochloride;
   cis-3-(5-chloro-2-methoxybenzyl)amino-2-(3-methoxyphenyl)piperidine dihydrochloride;
   cis-3-(5-chloro-2-methoxybenzyl)amino-2-(3-chlorophenyl)piperidine dihydrochloride;
   3-(2-methoxybenzylamino)-2,4-diphenylpiperidine;
   cis-3-(2-methoxybenzylamino)-2-phenylpyrrolidine;
   (2S,3S)-3-(5-ethyl-2-methoxybenzyl)amino-2-phenylpiperidine;
   (2S,3S)-3-(5-n-butyl-2-methoxybenzyl)amino-2-phenylpiperidine;
   (2S,3S)-3-(2-methoxy-5-n-propylbenzyl)amino-2-phenylpiperidine;
   (2S,3S)-3-(5-isopropyl-2-methoxybenzyl)amino-2-phenylpiperidine;
   (2S,3S)-3-(5-s-butyl-2-methoxybenzyl)amino-2-phenylpiperidine;
   (2S,3S)-3-(5-t-butyl-2-methoxybenzyl)amino-2-phenylpiperidine; and
   (2S,3S)-3-(2-methoxy-5-phenylbenzyl)amino-2-phenylpiperidine.
(47) A compound of the formula Id, wherein R³ is a group of the formula II or III and said compound is selected from the group consisting of:
   (2S,3S)-N-(5-isopropyl-2-methoxyphenyl)methyl-2-diphenylmethyl-1-azabicyclo[2.2.2]octan-3-amine;
   (2S,3S)-N-(5-tert-butyl-2-methoxyphenyl)methyl-2-diphenylmethyl-1-azabicyclo[2.2.2]octan-3-amine;
   (2S,3S)-N-(5-methyl-2-methoxyphenyl)methyl-2-diphenylmethyl-1-azabicyclo[2.2.2]octan-3-amine;
   (2S,3S)-N-(5-ethyl-2-methoxyphenyl)methyl-2-diphenylmethyl-1-azabicyclo[2.2.2]octan-3-amine;
   (2S,3S)-N-(5-isopropyl-2-methoxyphenyl)methyl-2-diphenylmethyl-1-azabicyclo[2.2.2]octan-3-amine;
   (2S,3S)-N-(5-sec-butyl-2-methoxyphenyl)methyl-2-diphenylmethyl-1-azabicyclo[2.2.2]octan-3-amine; and
   (2S,3S)-N-(5-n-propyl-2-methoxyphenyl)methyl-2-diphenylmethyl-1-azabicyclo[2.2.2]octan-3-amine.
(47-A) A compound of the formula Ie, wherein R³ is a group of the formula VII and said compound is selected from the group consisting of:
   2,4-dimethylthiazole-5-sulfonic acid [4-methoxy-3-((2S,3S)-2-phenylpiperidin-3-ylaminomethyl)phenyl]-methylamide;
   N-(4,5-dimethylthiazol-2-yl)-N-[4-methoxy-3-((2S,3S)-2-phenylpiperidin-3-yl-aminomethyl)phenyl]-methanesulfonamide;
   {5-[(4,5-dimethylthiazol-2-yl)methylamino]-2-methoxybenzyl}-((2S,3S)-2-phenylpiperidin-3-yl)amine;
   {5-(4,5-dimethylthiazol-2-ylamino)-2-methoxybenzyl}-((2S,3S)-2-phenylpiperidin-3-ylamine;
   4,5-dimethylthiazole-2-sulfonic acid methyl-[3-((2S,3S)-2-phenylpiperidin-3-ylaminomethyl)-4-trifluoromethoxyphenyl]-amide;
   2,4-dimethylthiazole-5-sulfonic acid [4-isopropoxy-3-((2S,3S)-2-phenylpiperidin-3-ylaminomethyl)phenyl]-methylamide;
   2,4-dimethylthiazole-5-sulfonic acid [4-isopropoxy-3-((2S,3S)-2-phenylpiperidin-3-ylaminomethyl)phenyl]-isopropylamide;
   2,4-dimethylthiazole-5-sulfonic acid [4-methoxy-3-((2S,3S)-2-phenylpiperidin-3-ylaminomethyl)phenyl]-isopropylamide;
   2,4-dimethylthiazole-5-sulfonic acid [4-methoxy-3-((2S,3S)-2-phenylpiperidin-3-ylaminomethyl)phenyl]-isobutylamide; and
   2,4-dimethylthiasole-5-sulfonic acid [4-isopropoxy-3-((2S,3S)-2-phenylpiperidin-3-ylaminomethyl)phenyl]-isobutylamide.
   This invention also relates, for the said use, to a compound having the formula 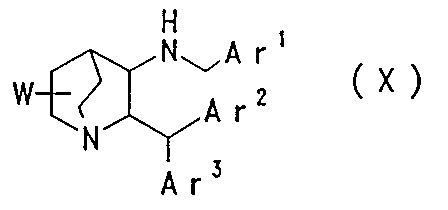 wherein W is Y or X(CH₂)ₙ;
   Y is optionally substituted (C₁-C₆)alkyl, optionally substituted (C₂-C₆)alkenyl or optionally substituted (C₃-C₈)cycloalkyl;
   X is optionally substituted (C₁-C₆) alkoxy, hydroxy, CONR¹R², CO₂R¹, CHR¹OR², CHR¹NR²R³, COR¹, CONR¹OR² or optionally substituted aryl, wherein said aryl is selected from phenyl, naphthyl, pyridyl, quinolyl, thienyl, furyl, phenoxyphenyl, oxazolyl, tetrazolyl, thiazolyl, imidazolyl and pyrazolyl; and n is an integer from zero to six;
   Ar¹, Ar² and Ar³ are each, independently, optionally substituted aryl, wherein said aryl is selected from phenyl, naphthyl, pyridyl, quinolyl, thienyl, furyl, phenoxyphenyl, oxazolyl, tetrazolyl, thiazolyl, imidazolyl and pyrazolyl;
   and R¹, R² and R³ are independently selected from hydrogen, optionally substituted (C₁-C₆)alkyl, optionally substituted (C₁-C₆)alkoxy, optionally substituted (C₃-C₈) cycloalkyl, optionally substituted aryl, wherein said aryl is selected from phenyl, naphthyl, pyridyl, quinolyl, thienyl, furyl, phenoxyphenyl, oxazolyl, tetrazolyl, thiazolyl, imidazolyl and pyrazolyl; and optionally substituted (C₁-C₅)heterocyclic groups, wherein said heterocyclic groups are selected from pyrrolidino, piperidino, morpholino, piperazinyl and thiamorpholino;
   and wherein the substituents on the foregoing substituted alkyl, alkenyl, cycloalkyl and alkoxy groups are independently selected from halo, nitro, amino, (C₁-C₄)alkyl, (C₁-C₄)alkoxy, trifluoromethyl and trifluoromethoxy;
   and wherein the substituents on the foregoing substituted (C₁-C₅) heterocyclic groups are attached to a sulfur or nitrogen atom on the ring and are independently selected from oxygen, di-oxygen and (C₁-C₄) alkyl when attached to a ring sulfur atom and are independently selected from oxygen and (C₁-C₄)alkyl when attached to a ring nitrogen atom;
   and wherein the substituents on said substituted Ar¹ groups are independently selected from (C₁-C₆)alkyl optionally substituted with from one to three halo groups, (C₁-C₆)alkoxy optionally substituted with from one to three halo groups, (C₁-C₆)alkylsulfinyl, (C₂-C₆)alkenyl, (C₁-C₆)alkylthio, (C₁-C₆)alkylsulfonyl, (C₁-C₆)alkylsulfonylamino, and di-(C₁-C₆)alkylamino wherein one or both of the alkyl groups may be optionally substituted with a (C₁-C₆)alkylsulfonyl, or (C₁-C₆)alkylsulfinyl group;
   and wherein the substituents on said substituted Ar² and Ar³ groups are independently selected from (C₁-C₄)alkyl, (C₁-C₄)alkoxy, (C₁-C₄)alkylthio, (C₁-C₄)alkylsulfinyl, di-(C₁-C₄)alkylamino, trifluoromethyl and trifluoromethoxy; with the proviso that when Y is unsubstituted or is substituted with (C₁-C₄)alkyl, it is attached to the 4- or 6-position of the quinuclidine ring;
   or a pharmaceutically acceptable salt of such compound.
   Preferred embodiments of this invention include, for the said use,
   a compound as defined in paragraphs (48) through (53) below, or a pharmaceutically acceptable salt of such compound.
(48) A compound of the formula X, wherein W is X(CH₂)ₙ.
(49) A compound of the formula X, wherein W is Y.
(50) A compound of the formula X, wherein Ar¹ is substituted aryl and W is Y.
(51) A compound of the formula X, wherein Ar¹ is mono-, di- or tri-substituted phenyl and W is Y.
(52) A compound of the formula X, wherein Ar¹ is phenyl disubstituted at the 2- and 5-positions and W is Y.
(53) A compound of the formula X, wherein Ar¹ is paramethoxyphenyl, each of Ar² and Ar³ is phenyl and W is Y.
(54) A compound of the formula X, or a pharmaceutically acceptable salt thereof, that is selected from the group consisting of:
   (3R,4S,5S,6S)-N,N-diethyl-5-(5-isopropyl-2-methoxybenzylamino)-6-diphenylmethyl-1-azabicyclo[2.2.2]octane-3-carboxamide;
   (3R,4S,5S,6S)-N,N-diethyl-5-(2,5-dimethoxybenzylamino)-6-diphenylmethyl-1-azabicyclo[2.2.2]octane-3-carboxamide;
   (3R,4S,5S,6S)-5-(5-isopropyl-2-methoxybenzylamino)-6-diphenylmethyl-1-azabicyclo[2.2.2]octane-3-carboxylic acid;
   (3R,4S,5S,6S)-5-(2-methoxy-2-methylthiobenzylamino)-6-diphenylmethyl-1-azabicyclo[2.2.2]octane-3-carboxylic acid;
   (3R,4S,5S,6S)-5-(2,5-dimethoxybenzylamino)-6-diphenylmethyl-1-azabicyclo-[2.2.2]octane-3-carboxylic acid;
   (3R,4S,5S,6S)-5-(2-methoxy-5-methylbenzylamino)-6-diphenylmethyl-1-azabicyclo[2.2.2]octane-3-carboxylic acid;
   (3R,4S,5S,6S)-5-(5-ethyl-2-methoxybenzylamino)-6-diphenylmethyl-1-azabicyclo[2.2.2]octane-3-carboxylic acid;
   (3R,4S,5S,6S)-5-(2-methoxyl-5-n-propylbenzylamino)-6-diphenylmethyl-1-azabicyclo[2.2.2]octane-3-carboxylic acid;
   (3R,4S,5S,6S)-5-(5-sec-butyl-2-methoxybenzylamino)-6-diphenylmethyl-1-azabicyclo[2.2.2]octane-3-carboxylic acid;
   (3R,4S,5S,6S)-5-(5-N-methyl-methanesulfonylamino-2-methoxy-benzylamino)-6-diphenylmethyl-1-azabicyclo[2.2.2]octane-3-carboxylic acid;
   (3R,4S,5S,6S)-5-(2-methoxy-5-methylsulfinylbenzylamino)-6-diphenylmethyl-1-azabicyclo[2.2.2]octane-3-carboxylic acid;
   (3R,4S,5S,6S)-5-(2-methoxy-5-trifluoromethoxybenzylamino)-6-diphenylmethyl-1-azabicyclo[2.2.2]octane-3-carboxylic acid;
   (3R,4S,5S,6S)-5-(2-methoxy-5-methylsulfonylbenzylamino)-6-diphenylmethyl-1-azabicyclo[2.2.2]octane-3-carboxylic acid;
   (3R,4S,5S,6S)-5-(5-dimethoxylamino-2-methoxybenzylamino-6-diphenylmethyl-1-azabicyclo[2.2.2]octane-3-carboxylic acid;
   (3R,4S,5S,6S)-5-(5-isopropyl-2-methoxybenzylamino)-6-diphenylmethyl-1-azabicyclo[2.2.2]octane-2-carboxylic acid;
   (3R,4S,5S,6S)-5-(2-methoxy-5-methylthiobenzylamino)-6-diphenylmethyl-1-azabicyclo[2.2.2]octane-2-carboxylic acid;
   (3R,4S,5S,6S)-5-(2,5-dimethoxybenzylamino)-6-diphenylmethyl-1-azabicyclo[2.2.2]octane-2-carboxylic acid;
   (3R,4S,5S,6S)-5-(2-methoxy-5-methylbenzylamino)-6-diphenylmethyl-1-azabicyclo[2.2.2]octane-2-carboxylic acid;
   (3R,4S,5S,6S)-5-(5-ethyl-2-methoxybenzylamino)-6-diphenylmethyl-1-azabicyclo[2.2.2]octane-2-carboxylic acid;
   (3R,4S,5S,6S)-5-(2-methoxyl-5-n-propylbenzylamino)-6-diphenylmethyl-1-azabicyclo[2.2.2]octane-2-carboxylic acid;
   (3R,4S,5S,6S)-5-(5-sec-butyl-2-methoxybenzylamino)-6-diphenylmethyl-1-azabicyclo[2.2.2]octane-2-carboxylic acid;
   (3R,4S,5S,6S)-5-(5-N-methylmethanesulfonylamino-2-methoxybenzyl-amino)-6-diphenylmethyl-1-azabicyclo[2.2.2]octane-2-carboxylic acid;
   (3R,4S,5S,6S)-5-(2-methoxy-5-methylsulfinylbenzylamino)-6-diphenylmethyl-1-azabicyclo[2.2.2]octane-2-carboxylic acid;
   (3R,4S,5S,6S)-5-(2-methoxy-5-trifluoromethoxybenzylamino)-6-diphenylmethyl-1-azabicyclo[2.2.2]octane-2-carboxylic acid;
   (3R,4S,5S,6S)-5-(2-methoxy-5-methylsulfonylbenzylamino)-6-diphenylmethyl-1-azabicyclo[2.2.2]octane-2-carboxylic acid; and
   (3R,4S,5S,6S)-5-(5-dimethylamino-2-methoxybenzylamino)-6-diphenylmethyl-1-azabicyclo[2.2.2]octane-2-carboxylic acid.
   This invention also relates, for the said use, to a compound having the formula 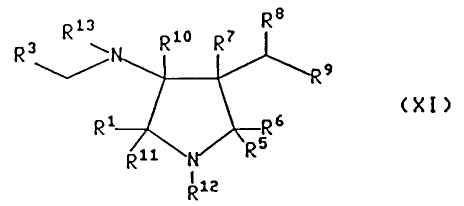 wherein R¹ is selected from hydrogen, (C₁-C₆) straight or branched alkyl, (C₃-C₇) cycloalkyl wherein one of the carbon atoms may optionally be replaced by nitrogen, oxygen or sulfur; aryl selected from phenyl, biphenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents independently selected from halo, nitro, (C₁-C₆) alkyl optionally substituted with from one to three fluorine atoms, (C₁-C₆) alkoxy, amino, trihaloalkoxy (e.g., trifluoromethoxy), (C₁-C₆)alkylamino, 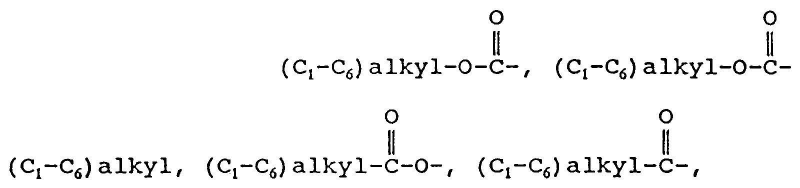 (C₁-C₆)alkyl-O-, 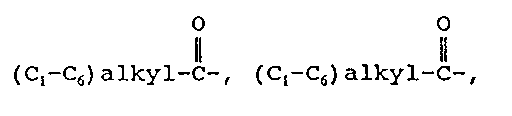 (C₁-C₆)alkyl-, di-(C₁-C₆)alkylamino, 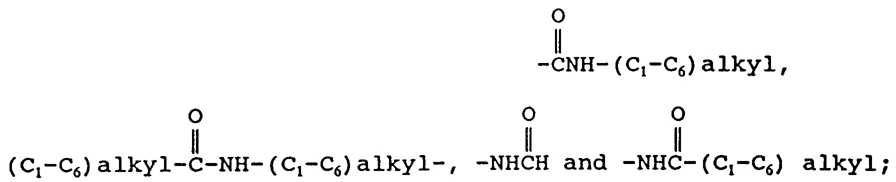 and wherein one of the phenyl moieties of said benzhydryl may optionally be replaced by naphthyl, thienyl, furyl or pyridyl;
   R³ is aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; and cycloalkyl having 3 to 7 carbon atoms wherein one of said carbon atoms may optionally be replaced by nitrogen, oxygen or sulfur; wherein each of said aryl and heteroaryl groups may optionally be substituted with one or more substituents, and said (C₃-C₇) cycloalkyl may optionally be substituted with one or two substituents, each of said substituents being independently selected from halo, nitro, (C₁-C₆) alkyl optionally substituted with from one to three fluorine atoms, (C₁-C₆) alkoxy optionally substituted with from one to three fluorine atoms, amino, phenyl, trihaloalkoxy (e.g., trifluoromethoxy), (C₁-C₆) alkylamino, 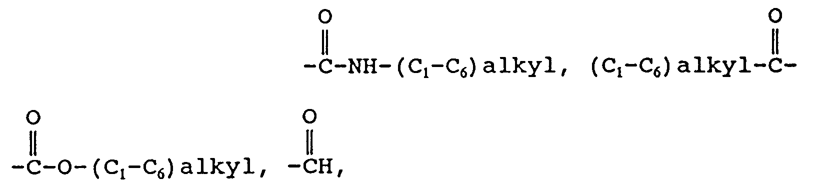 -CH₂OR¹³, NH(C₁-C₆)alkyl-, 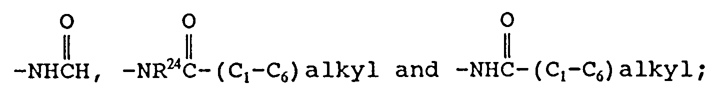 one of R⁵ and R⁶ is hydrogen and the other is selected from hydroxymethyl, hydrogen, (C₁-C₃)alkyl, (C₁-C₈)acyloxy-(C₁-C₃)alkyl, (C₁-C₈)alkoxymethyl and benzyloxymethyl;
   R⁷ and R⁸ are independently selected from hydrogen, (C₁-C₃)alkyl and phenyl;
   R⁹ is selected from methyl, hydroxymethyl, 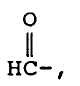 R¹⁴R¹⁵NCO₂CH₂-, R¹⁶OCO₂CH₂-, (C₁-C₄)alkyl-CO₂CH₂-, -CONR¹⁷R¹⁸, R¹⁷R¹⁸NCO₂-, R¹⁹OCO₂-, C₆H₅CH₂CO₂CH₂-, C₆H₅CO₂CH₂-, (C₁-C₄)alkyl-CH(OH)-, C₆H₅CH(OH)-, C₆H₅CH₂CH(OH)-, CH₂halo, R²⁰SO₂OCH₂, -CO₂R¹⁶ and R²¹CO₂-;
   R¹⁰ and R¹¹ are independently selected from hydrogen, (C₁-C₃) alkyl and phenyl;
   R¹² is hydrogen, benzyl or a group of the formula 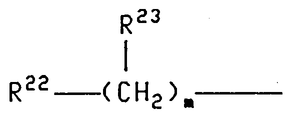 wherein m is an integer from zero to twelve, and any one of the carbon-carbon single bonds of (CH₂)ₘ, wherein both carbon atoms of such bond are bonded to each other and to another carbon atom in the (CH₂)ₘ chain, may optionally be replaced by a carbon-carbon double or triple bond, and any one of the carbon atoms of (CH₂)ₘ may optionally be substituted with R²³;
   R¹³, R¹⁴, R¹⁵, R¹⁶, R¹⁷, R¹⁸, R¹⁹, R²⁰, R²¹ and R²⁴ are independently selected from hydrogen, (C₁-C₃)alkyl and phenyl;
   R²² and R²³ are independently selected from hydrogen, hydroxy, halo, amino, carboxy, carboxy (C₁-C₆) alkyl, (C₁-C₆)alkylamino, di-(C₁-C₆)alkylamino, (C₁-C₆)alkoxy, 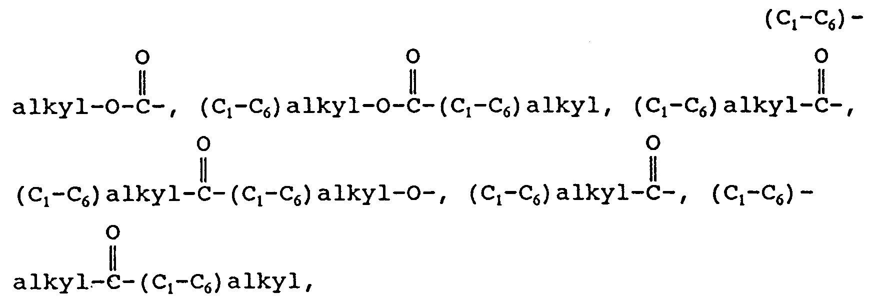 (C₁-C₆) straight or branched alkyl, (C₃-C₇) cycloalkyl wherein one of the carbon atoms may optionally be replaced by nitrogen, oxygen or sulfur; aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; phenyl-(C₂-C₆)alkyl, benzhydryl and benzyl, wherein each of said aryl and heteroaryl groups and the phenyl moieties of said benzyl, phenyl-(C₂-C₆)alkyl and benzhydryl may optionally be substituted with one or two substituents independently selected from halo, nitro, (C₁-C₆)alkyl optionally substituted with from one to three fluorine atoms, (C₁-C₆)alkoxy optionally substituted with from one to three fluorine atoms,
   trifluoromethyl, amino, (C₁-C₆)-alkylamino, 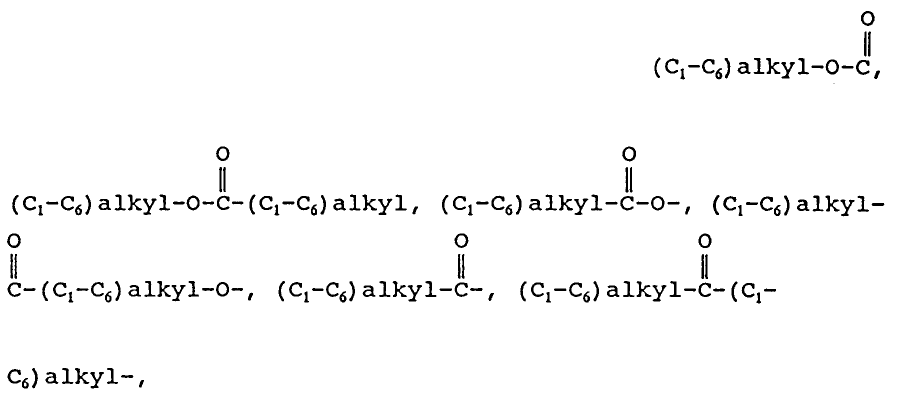 di-(C₁-C₆)alkylamino, 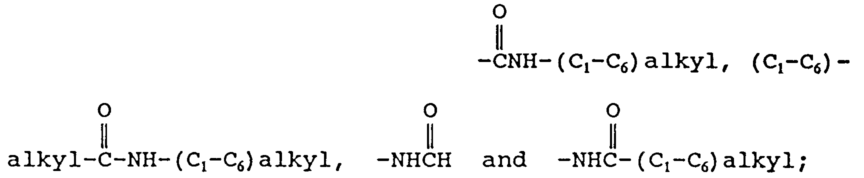 and wherein one of the phenyl moieties of said benzhydryl may optionally be replaced by naphthyl, thienyl, furyl or pyridyl;
   or R⁹, together with the carbon to which it is attached, the nitrogen of the pyrrolidine ring, the carbon to which R⁷ is attached and the carbon to which R⁵ and R⁶ are attached form a second pyrrolidine ring; with the proviso that when R⁹, together with the carbon to which it is attached, the nitrogen of the pyrrolidine ring, the carbon to which R⁷ is attached and the carbon to which R⁵ and R⁶ are attached, form a second pyrrolidine ring (thus forming a bicyclic structure containing a bridgehead nitrogen), either R¹² is absent or R¹² is present and the nitrogen of the second pyrrolidine ring is positively charged; or a pharmaceutically acceptable salt of such compound.
   Compounds of the formula XI that contain two pyrrolidine rings may be represented by one of the following two structures, depending on whether R¹² is present or absent. 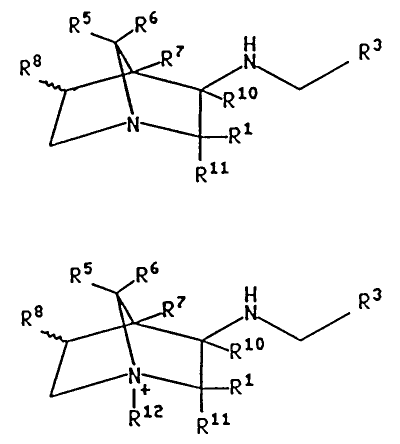 Preferred embodiments of this invention include, for the said use,
   a compound as defined in paragraphs (55) through (59) below, or a pharmaceutically acceptable salt of such compound.
(55) A compound of the formula XI wherein R¹ is benzhydryl.
(56) A compound of the formula XI wherein R¹ is diphenylmethyl, R³ is aryl selected from phenyl or indanyl wherein each of said aryl groups may be optionally substituted with one, two or three substituents, each of R⁵, R⁶, R⁷, R⁸, R¹⁰, and R¹¹ is hydrogen, R⁹ is selected from hydroxymethyl, methoxymethyl, -CO₂R¹⁶, -CONR¹⁷R¹⁸, R¹⁴R¹⁵NCO₂CH₂-, R¹⁶OCO₂CH₂-, (C₁-C₄)alkyl-CO₂CH₂-, C₆H₅CH₂CO₂CH₂-, -CH₂halo and R²⁰SO₂OCH₂-, and R¹² is hydrogen or benzyl.
(57) A compound of the formula XI wherein R¹ is phenyl, R³ is aryl selected from phenyl or indanyl wherein each of said aryl groups may be optionally substituted with one, two or three substiuents, each of R⁵, R⁶, R⁷, R⁸, R¹⁰, and R¹¹ is hydrogen, R⁹ is selected from hydroxymethyl, methoxymethyl, - CO₂R¹⁸, -CONR¹⁷R¹⁸, R¹⁴R¹⁵NCO₂CH₂CH₂-, R¹⁶OCO₂CH₂-, (C₁-C₄) alkyl-CO₂CH₂-, -CH₂halo, R²⁰SO₂OCH-, and R¹² is hydrogen or benzyl.
(58) A compound of the formula XI wherein R¹ is diphenylmethyl, R³ is aryl selected from phenyl or indanyl wherein each of said aryl groups may be optionally substituted with one, two or three substituents, each of R⁵, R⁶, R⁷, R⁸, R¹⁰, R¹¹ and R¹³ is hydrogen, and wherein R⁹, together with the carbon to which it is attached, the nitrogen of the pyrrolidine ring, the carbon to which R⁷ is attached and the carbon to which R⁵ and R⁶ are attached, form a second pyrrolidine ring (thus forming a bicyclic structure containing a bridgehead nitrogen).
(59) A compound of the formula XI that is selected from the group consisting of:
   (2S, 3S, 4R)-2-diphenylmethyl-3-[(2-methoxy-4,5-dimethylphenyl)methylamino]-4-(2-hydroxyethyl)pyrrolidine;
   (2SR, 3SR, 4RS)-2-diphenylmethyl-3-[(2-methoxy-4,5-dimethylphenyl)methylamino]-4-(2-hydroxyethyl)pyrrolidine;
   (2SR, 3SR, 4RS)-2-diphenylmethyl-3-[(2-methoxy-5-(methylethyl)phenyl)methylamino]-4-(carbomethoxymethyl)-pyrrolidine;
   (2SR, 3SR, 4RS)-2-diphenylmethyl-3-[(2-methoxy-5-(methylethyl)phenyl)methylamino]-4-(carboxymethyl)-pyrrolidine;
   (2SR, 3SR, 4RS)-2-diphenylmethyl-3-[(2-methoxy-5-(methylethyl)phenyl)methylamino]-4-(2-dimethylaminocarbamoylethyl)pyrrolidine;
   (2SR, 3SR, 4RS)-2-diphenylmethyl-3-[(2-trifluoromethoxyphenyl)methylamino]-4-(2-hydroxyethyl)-pyrrolidine;
   (2S, 3S, 4R)-2-diphenylmethyl-3-[(2-methoxy-5-(1,1-dimethylethyl)phenyl)methylamino]-4-(2-hydroxyethyl)-pyrrolidine;
   (2SR, 3SR, 4RS)-2-diphenylmethyl-3-[(2-methoxy-5-(1,1 dimethylethyl)phenyl)methylamino]-4-(2-methoxyethyl)-pyrrolidine;
   (2S, 3S, 4R)-2-diphenylmethyl-3-[(2-methoxy-5-methylethyl)phenyl)methylamino]-4-(2-hydroxyethyl)-pyrrolidine;
   (2SR, 3SR, 4RS)-2-diphenylmethyl-3-[(2-methoxy-5-methylethyl)phenyl)methylamino]-4-(2-methoxyethyl)-pyrrolidine;
   (2SR, 3SR, 4RS)-2-diphenylmethyl-3-[(2-methyl-5-(1,1-dimethylethyl)phenyl)methylamino]-4-(2-hydroxyethyl)-pyrrolidine;
   (1SR, 2SR, 3SR, 4RS)-1-aza-2-diphenylmethyl-3-[(2-methoxy-4,5-dimethylphenyl)-methylamino]-bicyclo[2.2.1]heptane;
   (1SR, 2SR, 3SR, 4RS)-1-aza-2-diphenylmethyl-3-[(2-methoxyphenyl)methylamino]bicyclo[2.2.1]heptane;
   (1SR, 2SR, 3SR, 4RS)-1-aza-2-diphenylmethyl-3-[(2-methoxy-5-(1,1-dimethylethyl)phenyl)methylamino]bicyclo[2.2.1]heptane;
   (1SR, 2SR, 3SR, 4RS)-1-aza-2-diphenylmethyl-3-[(2-methoxy-5-trifluoromethoxyphenyl)methylamino]bicyclo[2.2.1]heptane;
   (1SR, 2SR, 3SR, 4RS)-1-aza-2-diphenylmethyl-3-[(2-methoxy-5-(1-methylethyl)phenyl)methylamino]bicyclo[2.2.1]heptane;
   (1SR, 2SR, 3SR, 4RS)-1-aza-2-diphenylmethyl-3-[(2-methoxy-5-propylphenyl)methylamino]bicyclo[2.2.1]heptane;
   (1SR, 2SR, 3SR, 4RS)-1-aza-2-diphenylmethyl-3-[(2-methoxy-5-(1-methylpropyl)phenyl)methylamino]bicyclo[2.2.1]heptane;
   (1SR, 2SR, 3SR, 4RS)-1-aza-2-phenyl-3-[(2-methoxyphenyl)methylamino]bicyclo[2.2.1]heptane;
   (1SR, 2SR, 3RS, 4RS)-1-aza-2-phenyl-3-[(2-methoxy-5-trifluoromethoxyphenyl)methylamino]bicyclo[2.2.1]heptane;
   (2SR, 3SR, 4RS)-N-1-phenylmethyl-2-diphenylmethyl-3-[(2-methoxyphenyl)methylamino]-4-(2-hydroxyethyl)-pyrrolidine;
   (2SR, 3SR, 4RS)-2-diphenylmethyl-3-[(2-methoxyphenyl)methylamino]-4-(2-hydroxyethyl)pyrrolidine;
   (2SR, 3SR, 4RS)-2-diphenylmethyl-3-[(2-methoxy-5-(1,1-dimethylethyl)phenyl)methylamino]-4-(2-hydroxyethyl)-pyrrolidine;
   (2SR, 3SR, 4RS)-2-diphenylmethyl-3-[(2-methoxy-5-trifluoromethoxyphenyl)methylamino]-4-(2-hydroxyethyl)-pyrrolidine;
   (2SR, 3SR, 4RS)-2-diphenylmethyl-3-[(2-methoxy-5-(1-methylethyl)phenyl)methylamino]-4-(2-hydroxyethyl)-pyrrolidine;
   (2SR, 3SR, 4RS)-2-diphenylmethyl-3-[(2-methoxy-5-propylphenyl)methylamino]-4-(2-hydroxyethyl)pyrrolidine;
   (2SR, 3SR, 4RS)-2-diphenylmethyl-3-[(2-methoxy-5-(1-methyl-1-propyl)phenyl)methylamino]-4-(2-hydroxyethyl)pyrrolidine;
   (2SR, 3SR, 4RS)-2-diphenylmethyl-3-[(2-trifluoromethoxy-5-(1,1-dimethylethyl)phenyl)methylamino]-4-(2-hydroxyethyl)pyrrolidine;
   (2SR, 3SR, 4RS)-2-diphenylmethyl-3-[(2-methoxy-5-chlorophenyl)methylamino]-4-(2-hydroxyethyl)pyrrolidine;
   (2SR, 3SR, 4RS)-2-phenyl-3-[(2-methoxyphenyl)methylamino]-4-(2-hydroxyethyl)pyrrolidine;
   (2SR, 3SR, 4RS)-2-phenyl-3-[(2-methoxy-5-(1,1-dimethylethyl)phenyl)methylamino]-4-(2-hydroxyethyl)pyrrolidine; and
   (2SR, 3SR, 4RS)-2-phenyl-3-[(2-methoxy-5-trifluoromethoxyphenyl)methylamino]-4-(2-hydroxyethyl)pyrrolidine.
   This invention also relates, for the said use, to a compound of the formula 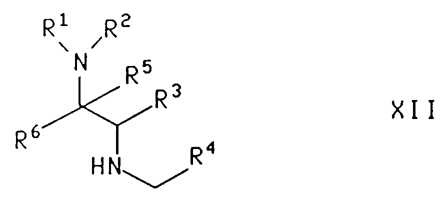 wherein R¹ is hydrogen, (C₁-C₈) alkyl, a saturated (C₆-C₁₀) carbocyclic ring system containing two fused rings, a saturated (C₆-C₁₀) carbocyclic bridged ring system containing two rings, or benzyl wherein the phenyl moiety of said benzyl may optionally be substituted with one or more substituents independently selected from halo, (C₁-C₆) alkyl optionally substituted with from one to three fluorine atoms and (C₁-C₈) alkoxy optionally substituted with from one to three fluorine atoms;
   R² is hydrogen, benzyl or a group of the formula 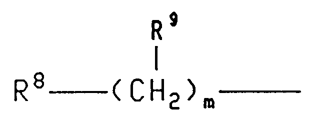 wherein m is an integer from zero to twelve, and any one of the carbon-carbon single bonds of (CH₂)ₘ, wherein both carbon atoms of such bond are bonded to each other and to another carbon atom of the (CH₂)ₘ chain, may optionally be replaced by a carbon-carbon double or triple bond, and any one of the carbon atoms of (CH₂)ₘ may optionally be substituted with R⁹;
   R⁸ and R⁹ are independently selected from hydrogen, hydroxy, halo, amino, carboxy, carboxy(C₁-C₆)alkyl, (C₁-C₆)alkylamino, di-(C₁-C₆)alkylamino, (C₁-C₆)alkoxy, 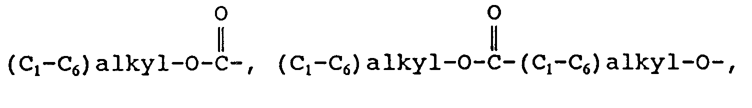 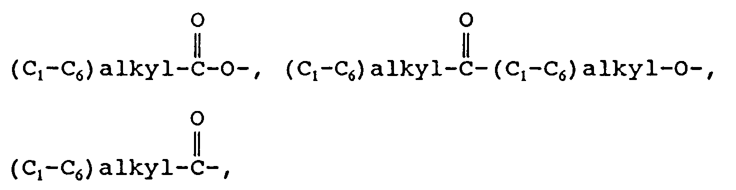 (C₁-C₆) straight or branched alkyl, (C₃-C₇) cycloalkyl wherein one of the carbon atoms may optionally be replaced by nitrogen, oxygen or sulfur; aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; phenyl-(C₂-C₆)alkyl, benzhydryl and benzyl, wherein each of said aryl and heteroaryl groups and the phenyl moieties of said benzyl, phenyl-(C₂-C₆)alkyl and benzhydryl may optionally be substituted with one or two substituents independently selected from halo, nitro, (C₁-C₆)alkyl optionally substituted with from one to three fluorine atoms, (C₁-C₆)alkoxy optionally substituted with from one to three fluorine atoms,
   trifluoromethyl, amino, (C₁-C₆)-alkylamino, 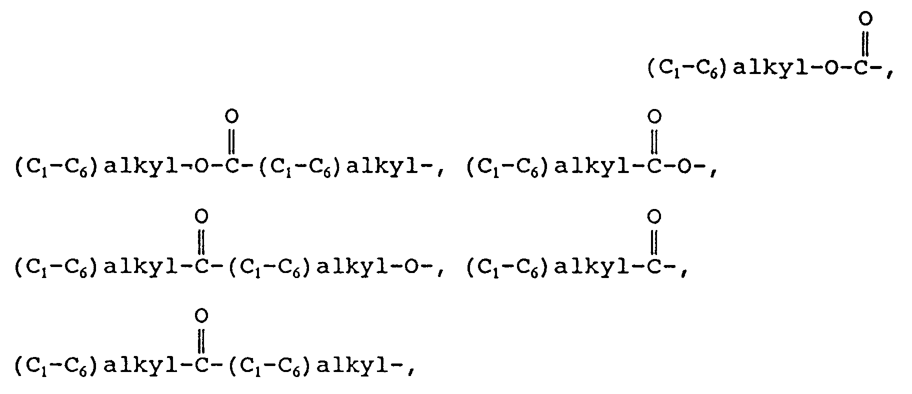 di-(C₁-C₆)alkylamino, 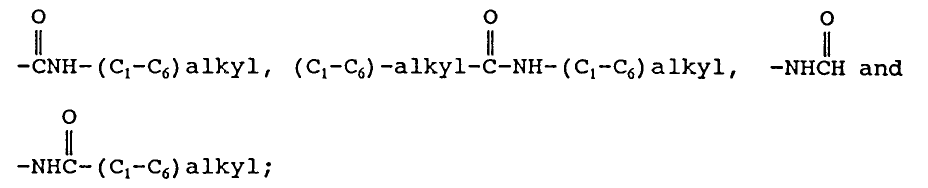 and wherein one of the phenyl moieties of said benzhydryl may optionally be replaced by naphthyl, thienyl, furyl or pyridyl;
   or R¹ and R², together with the nitrogen to which they are attached, form a saturated or unsaturated monocyclic ring containing from three to eight carbon atoms, a fused bicyclic ring containing from six to ten carbon atoms, or a saturated bridged ring system containing from six to ten carbon atoms;
   R⁴ is aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; and cycloalkyl having from three to seven carbon atoms wherein one of said carbon atoms may optionally be replaced by nitrogen, oxygen or sulfur; wherein each of said aryl and heteroaryl groups may optionally be substituted with one or more substituents, and said (C₃-C₇) cycloalkyl may optionally be substituted with one, two or three substituents, each of said substituents being independently selected from halo, nitro, (C₁-C₆) alkyl optionally substituted with from one to three fluorine atoms, (C₁-C₆) alkoxy optionally substituted with from one to three fluorine atoms, phenyl,
   amino, (C₁-C₆) alkylamino, 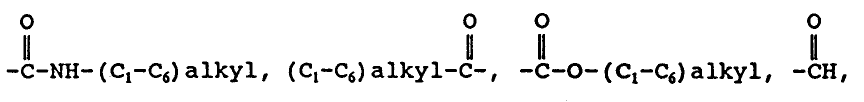 -CH₂OR¹², NH₂(C₁-C₆) alkyl-, 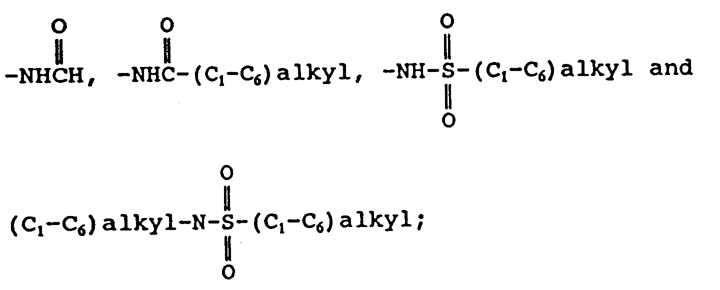 R³ is hydrogen, (C₃-C₈)cycloalkyl, (C₁-C₆) straight or branched alkyl or phenyl optionally substituted with one or more substituents independently selected from halo, (C₁-C₆)alkyl optionally substituted with from one to three fluorine atoms, and (C₁-C₆)alkoxy optionally substituted with from one to three fluorine atoms;
   R⁵ is hydrogen, (C₁-C₆)alkyl, or phenyl optionally substituted with one or more substituents independently selected from halo, (C₁-C₆)alkyl optionally substituted with from one to three fluorine atoms and (C₁-C₆) alkoxy optionally substituted with from one to three fluorine atoms;
   R⁶ is selected from hydrogen, (C₁-C₆) straight or branched alkyl, (C₃-C₇) cycloalkyl wherein one of the carbon atoms may optionally be replaced by nitrogen, oxygen or sulfur; aryl selected from phenyl, biphenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents independently selected from halo, nitro, (C₁-C₆) alkyl optionally substituted with from one to three fluorine atoms, (C₁-C₆) alkoxy, trifluoromethyl, amino, trihaloalkoxy (e.g.,trifluoromethoxy), (C₁-C₆)alkylamino, 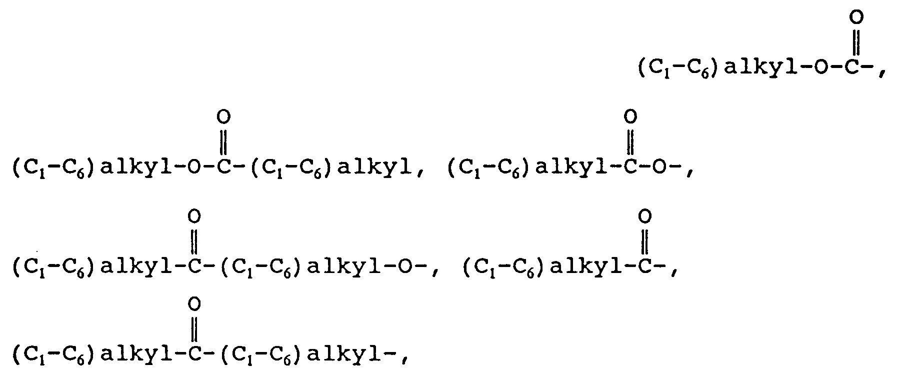 di-(C₁-C₆)alkylamino, 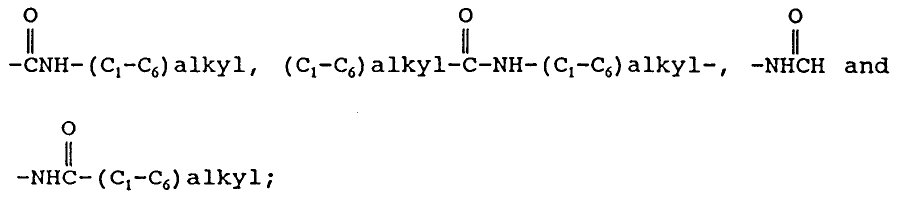 and wherein one of the phenyl moieties of said benzhydryl may optionally be replaced by naphthyl, thienyl, furyl or pyridyl; and
   R¹² is hydrogen, (C₁-C₃)alkyl or phenyl;
   or a pharmaceutically acceptable salt of such compound.
   Preferred embodiments of this invention include, for the said use,
   a compound as defined in paragraphs (60) through (62) below, or a pharmaceutically acceptable salt of such compound.
(60) A compound of the formula XII wherein R² is hydrogen, or R² and R¹, together with the nitrogen to which they are attached, form a monocyclic ring containing five to seven carbon atoms; R³ is hydrogen, methyl or phenyl; R⁵ is hydrogen; R⁴ is phenyl or indanyl, wherein said phenyl or indanyl may optionally be substituted with from one to three substituents independently selected from halo, nitro, (C₁-C₆) alkyl optionally substituted with from one to three fluorine atoms, (C₁-C₆) alkoxy, trihaloalkoxy (e.g., trifluoromethoxy), (C₁-C₆) alkylamino, -C(O)NH-(C₁-C₈)alkyl, (C₁-C₆)alkyl-C(O)-, -C(O)-O-(C₁-C₆)alkyl, -C(O)H, -CH₂OR¹², -NH(C₁-C₆)alkyl, -NHC(O)H, -NHC(O)-(C₁-C₆)alkyl, -NHSO₂(C₁-C₆)alkyl and (C₁-C₆)alkyl-N-SO₂-(C₁-C₆)alkyl; and R⁶ is phenyl.
(61) A compound of the formula XII wherein R¹ is alkyl, R⁶ is unsubstituted phenyl, R⁴ is a monosubstituted or disubstituted aryl group that is substituted at the C-2 position with an alkoxy group or substituted at the C-5 position with an alkyl, alkoxy or trihaloalkoxy group, or substituted in such manner at both C-2 and C-5 positions (i.e., with an alkoxy group at the C-2 position and an alkyl, alkoxy or trihaloalkoxy group at the C-5 position), and each of R², R³ and R⁵ is hydrogen.
(62) A compound of the formula XII that is selected from the group consisting of:
   1-N-cyclohexyl-1-phenyl-2-N'-[(2-methoxyphenyl)methyl]-1,2-ethanediamine;
   1-N-cyclohexyl-1-phenyl-2-N'-[(2-methoxy-5-trifluoromethoxyphenyl)methyl]-1,2-ethanediamine;
   1-N-pyrrolidyl-1-phenyl-2-N'-[(2-methoxyphenyl)methyl]-1,2-ethanediamine;
   1-N-methyl-1-phenyl-2-N'-[(2-methoxyphenyl)methyl]-1,2-ethanediamine;
   1-N-cyclopentyl-1-phenyl-2-N'-[(2-methoxyphenyl) methyl]-1,2-ethanediamine;
   1-N-propyl-1-phenyl-2-N'-[(2-methoxyphenyl)methyl]-1,2-ethanediamine;
   1-N-phenylmethyl-1-phenyl-2-N'-[(2-methoxyphenyl) methyl]-1,2-ethanediamine;
   1-N-cyclooctyl-1-phenyl-2-N'-[(2-methoxyphenyl)methyl]-1,2-ethanediamine;
   1-N-cyclobutyl-1-phenyl-2-N'-[(2-methoxyphenyl)methyl]-1,2-ethanediamine;
   1-N-(2-adamantyl)-1-phenyl-2-N'-[(2-methoxyphenyl) methyl]-1,2-ethanediamine;
   1-N-(1,1-dimethylethyl)-1-phenyl-2-N'-[(2-methoxyphenyl)methyl]-1,2-ethanediamine;
   1-N-cyclopropyl-1-phenyl-2-N'-[(2-methoxyphenyl) methyl]-1,2-ethanediamine;
   1-N-isopropyl-1-phenyl-2-N'-[(2-methoxyphenyl)methyl]-1,2-ethanediamine;
   1-N-(1-phenylethyl)-1-phenyl-2-N'-[(2-methoxyphenyl)methyl]-1,2-ethanediamine;
   1-N-(2-norbornyl)-1-pheny-1-2-N'-[(2-methoxyphenyl)methyl]-1,2-ethanediamine;
   1-N-cyclohexyl-1-phenyl-2-N'-[(2-methoxy-5-tert-butylphenyl)methyl]-1,2-ethanediamine;
   1-N-cyclohexyl-1-phenyl-2-N'-[(2-methoxy-5-isopropylphenyl)methyl]-1,2-ethanediamine;
   1-N-cyclohexyl-1-phenyl-2-N'-[(2-methoxy-4,5-dimethylphenyl)methyl]-1,2-ethanediamine; and
   1-N-cyclohexyl-1-N-(6-hydroxyhexyl)-1-phenyl-2-N'-[(2-methoxyphenyl)methyl]-1,2-ethanediamine.
   This invention also relates, for the said use, to a compound of the formula 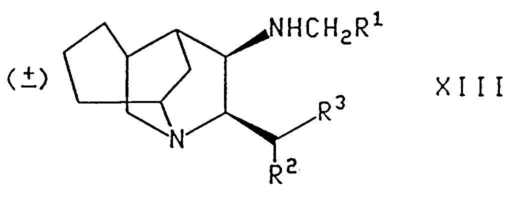 wherein R¹ is cycloalkyl having from five to seven carbon atoms, pyrrolyl, thienyl, pyridyl, phenyl or substituted phenyl, wherein said substituted phenyl is substituted with from one to three substituents independently selected from fluorine, chlorine, bromine, trifluoromethyl, alkyl having from one to three carbon atoms, alkoxy having from one to three carbon atoms, carboxy, alkoxycarbonyl having from one to three carbon atoms in the alkoxy moiety and benzyloxycarbonyl;
   R² is furyl, thienyl, pyridyl, indolyl, biphenyl, phenyl or substituted phenyl, wherein said substituted phenyl is substituted with one or two substituents independently selected from fluorine, chlorine, bromine, trifluoromethyl, alkyl having from one to three carbon atoms, alkoxy having from one to three carbon atoms, carboxy, alkoxycarbonyl having from one to three carbon atoms in the alkoxy moiety and benzyloxycarbonyl; and
   R³ is thienyl, phenyl, fluorophenyl, chlorophenyl or bromophenyl, or a pharmaceutically acceptable salt of such compound.
   Preferred embodiments of this invention include, for the said use,
   a compound as defined in paragraphs (63) through (65) below, or a pharmaceutically acceptable salt of such compound.
(63) A compound of the formula XIII, wherein R¹ is phenyl or substituted phenyl.
(64) A compound of the formula XIII, wherein R¹ is methoxyphenyl.
(65) A compound of the formula XIII, wherein said compound is (±)-cis-9-diphenylmethyl-N-((2-methoxyphenyl)methyl)-10-azatricyclo[4.4.1.0^{5,7}]undecan-8-amine.
   This invention also relates, for the said use, to a compound of the formula 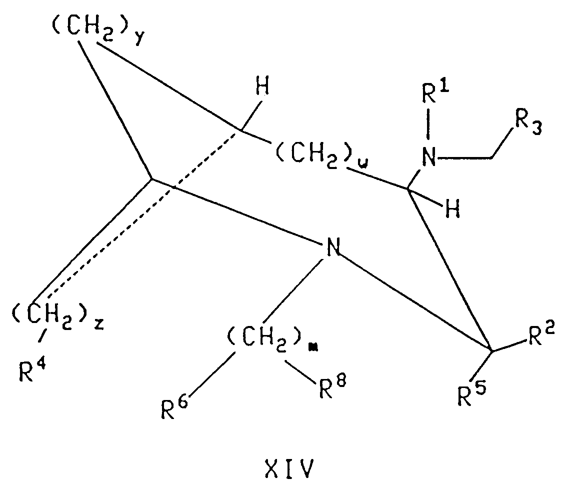 wherein m is an integer from 0 to 8, and any one of the carbon-carbon single bonds of (CH₂)ₘ, wherein both carbon atoms of such bond are bonded to each other and to another carbon atom in the (CH₂)ₘ chain, may optionally be replaced by a carbon-carbon double bond or a carbon-carbon triple bond, and any one of the carbon atoms of said (CH₂)ₘ may optionally be substituted with R⁸;
   w is an integer from 0 to 2;
   y is an integer from 1 to 4;
   z is an integer from 1 to 4, and wherein any one of the carbon atoms of said (CH₂)_{z} may optionally be substituted with R⁴;
   R¹ is hydrogen or (C₁-C₈) alkyl optionally substituted with hydroxy, alkoxy or fluoro;
   R² is a group selected from hydrogen, (C₁-C₆)straight or branched alkyl, (C₃-C₇)cycloalkyl wherein one of the carbon atoms may optionally be replaced by nitrogen, oxygen or sulfur; aryl selected from phenyl, indanyl, and naphthyl; heteroaryl selected from thienyl, furyl, pyridyl, thiazolyl, isothiazolyl, oxazolyl, isoxazolyl, triazolyl, tetrazolyl and quinolyl; phenyl(C₂-C₆)alkyl, benzhydryl and benzyl, wherein one of the phenyl moieties of said benzhydryl may optionally be replaced by naphthyl, thienyl, furyl or pyridyl and wherein each of said aryl and heteroaryl groups and the phenyl moieties of said benzyl, phenyl(C₂-C₆)alkyl and benzhydryl may optionally be substituted with one or more substituents independently selected from halo, nitro, (C₁-C₆)alkyl, (C₁-C₆)alkoxy, trifluoromethyl, amino, (C₁-C₆)-alkylamino, 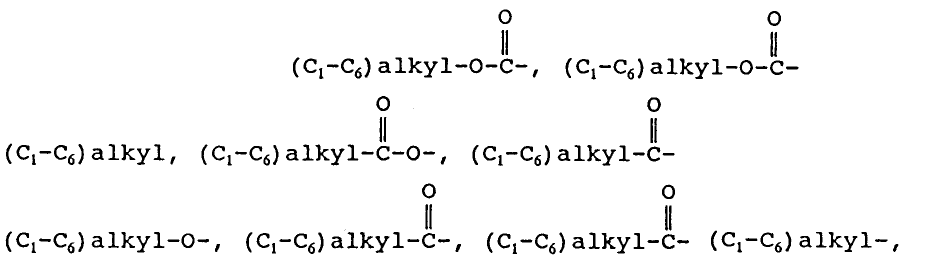 di-(C₁-C₆)alkylamino, 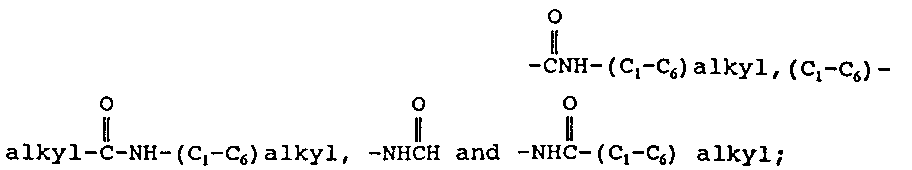 R⁵ is hydrogen, phenyl or (C₁-C₆)alkyl;
   or R² and R⁵, together with the carbon to which they are attached, form a saturated carbocyclic ring having from 3 to 7 carbon atoms wherein one of said carbon atoms may optionally be replaced by oxygen, nitrogen or sulfur;
   R³ is aryl selected from phenyl, indanyl, and naphthyl; heteroaryl selected from thienyl, furyl, pyridyl, thiazolyl, isothiazolyl, oxazolyl, isoxazolyl, triazolyl, tetrazolyl and quinolyl; and cycloalkyl having 3 to 7 carbon atoms wherein one of said carbon atoms may optionally be replaced by nitrogen, oxygen or sulfur; wherein each of said aryl and heteroaryl groups may optionally be substituted with one or more substituents, and said (C₃-C₇) cycloalkyl may optionally be substituted with one or two substituents, each of said substituents being independently selected from halo, nitro, (C₁-C₆)alkyl optionally substituted with from one to three fluorine atoms, (C₁-C₆)alkoxy optionally substituted with from one to three fluorine atoms, trifluoromethyl, phenyl, amino, (C₁-C₆)alkylamino, (C₁-C₆)dialkyl amino, 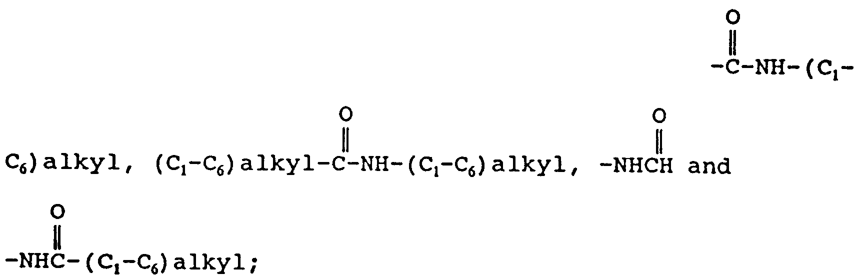 R⁴ is independently selected from hydrogen, hydroxy, halo, amino, oxo (=O), nitrile,
   (C₁-C₆)alkylamino, di-(C₁-C₆)alkylamino, (C₁-C₆)alkoxy, 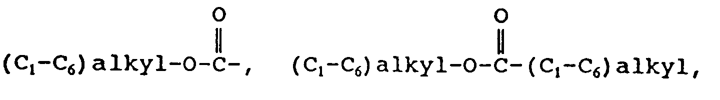 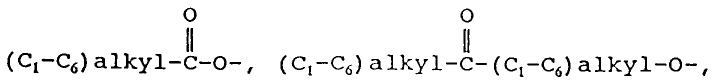 hydroxy-(C₁-C₆)alkyl, (C₁-C₆)alkoxy(C₁-C₆)alkyl, 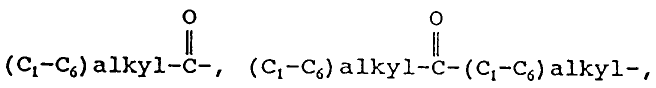 and the groups set forth in the definition of R²;
   R⁶ is 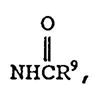 NHCH₂R⁹, NHSO₂R⁹ or one of the groups set forth in any of the definitions of R², and R⁴;
   R⁸ is oximino (=NOH) or one of the groups set forth in any of the definitions of R², and R⁴;
   R⁹ is (C₁-C₆) alkyl, hydrogen, phenyl or phenyl (C₁-C₆)alkyl;
   with the proviso that (a) when m is 0, R⁸ is absent and R⁶ is hydrogen, (b) neither R⁴, R⁶, nor R⁸ can form, together with the carbon to which it is attached, a ring with R⁵, (c) the sum of y and z must be less than 7; or a pharmaceutically acceptable salt thereof.
   Preferred embodiments of this invention include, for the said use,
   a compound as defined in paragraphs (66) through (68) below, or a pharmaceutically acceptable salt of such compound.
(66) A compound of the formula XIV, wherein R² is a radical selected from hydrogen, phenyl, naphthyl and benzhydryl; wherein each of said phenyl, naphthyl and benzhydryl may optionally be substituted with one or more substituents independently selected from halo, nitro, (C₁-C₆) alkyl, (C₁-C₆)alkoxy, trifluoromethyl, amino, (C₁-C₆)-alkylamino, 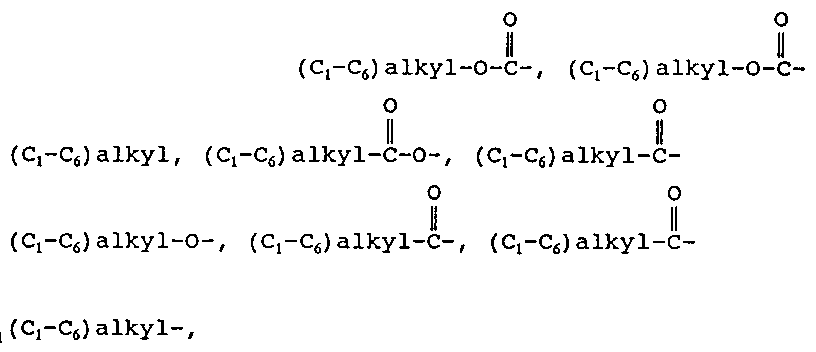 di-(C₁-C₆)alkylamino, 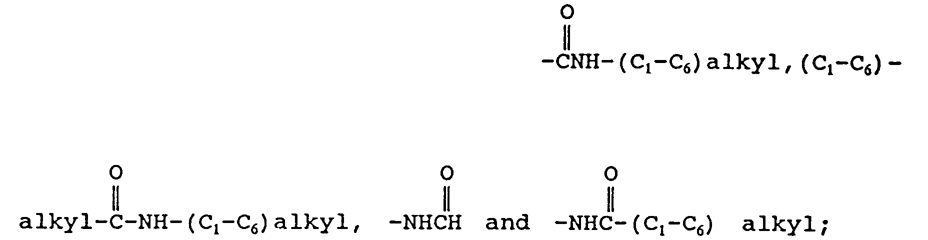 and wherein one of the phenyl moieties of said benzhydryl may optionally be replaced by naphthyl, thienyl, furyl or pyridyl.
(67) A compound of the formula XIV, wherein R² is a group selected from hydrogen, phenyl, naphthyl and benzhydryl; wherein each of said phenyl, naphthyl and benzhydryl may optionally be substituted with one or more substituents independently selected from halo, nitro, (C₁-C₆) alkyl, (C₁-C₆) alkoxy, trifluoromethyl, amino, (C₁-C₆)-alkylamino, 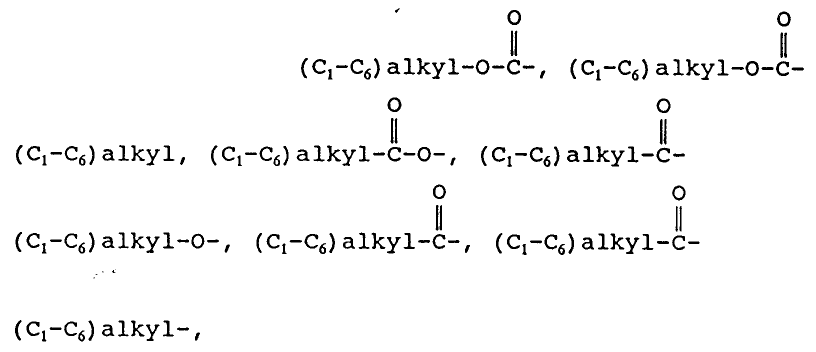 di-(C₁-C₆)alkylamino, 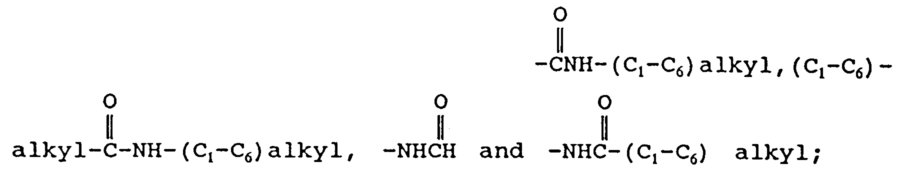 and wherein one of the phenyl moieties of said benzhydryl may optionally be replaced by naphthyl, thienyl, furyl or pyridyl; and
   R⁴ is independently selected from hydrogen, hydroxy, halo, amino, oxo (=O), nitrile,
   (C₁-C₆)alkylamino, di-(C₁-C₆)alkylamino, (C₁-C₆)alkoxy, 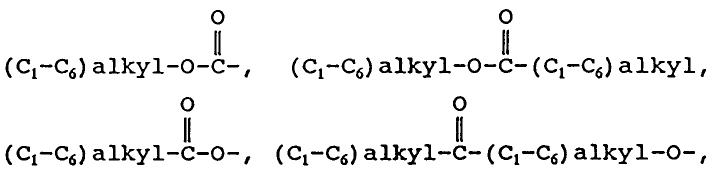 hydroxy-(C₁-C₆)alkyl, (C₁-C₆)alkoxy(C₁-C₆)alkyl 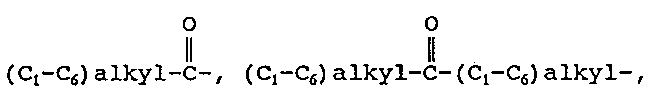 (C₁-C₆)alkyl and phenyl.
(68) A compound of the formula XIV, wherein said compound is (3RS,4RS)-3-phenyl-4-(2-methoxybenzyl)amino-2-azabicyclo[3.3.1]nonane.
   This invention also relates, for the said use, to a compound of the formula 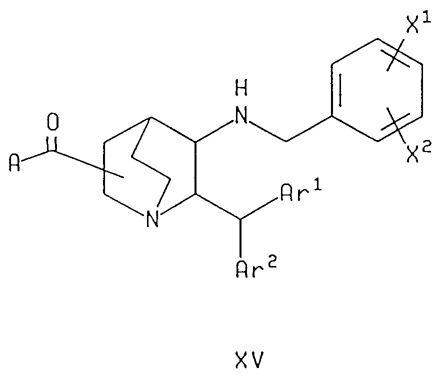 wherein X¹ is (C₁-C₅)alkoxy or halosubstituted (C₁-C₅) alkoxy;
   X² is hydrogen, halogen, (C₁-C₅)alkyl, (C₂-C₅)alkenyl, (C₂-C₅)alkynyl, (C₁-C₅)alkoxy, (C₁-C₅)alkylthio, (C₁-C₅) alkylsulfinyl, (C₁-C₅) alkylsulfonyl, halosubstituted (C₁-C₅) alkyl, halosubstituted (C₁-C₅) alkoxy, (C₁-C₅)alkylamino, dialkylamino having from 1 to 5 carbon atoms in each alkyl moiety, (C₁-C₅)alkylsulfonylamino (which may be substituted by halogen), 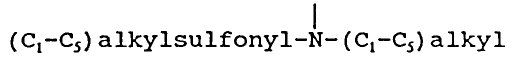 (which may be substituted by halogen in the alkylsulfonyl moiety), (C₁-C₅)alkanoylamino (which may be substituted by halogen) or 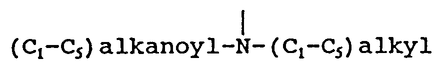 (which may be substituted by halogen in the alkanoyl moiety);
   Ar¹ and Ar₂ are each, independently, thienyl, phenyl, fluorophenyl, chlorophenyl or bromophenyl;
   A is Y-(CH₂)ₘ-CH(R²)-(CH₂)ₙ-NR¹-;
   R¹ is hydrogen, (C₁-C₅)alkyl, benzyl or -(CH₂)ₚ-Y;
   R² is hydrogen, (C₁-C₅)alkyl (which may be substituted by a substituent selected from the group consisting of hydroxy, amino, methylthio and mercapto), benzyl, 4-hydroxybenzyl, 3-indolylmethyl or -(CH₂)ₚ-Y;
   Y is -CN, -CH₂Z or -COZ;
   Z is hydroxy, amino, (C₁-C₅)alkoxy, (C₁-C₅) alkylamino or dialkylamino having from 1 to 5 carbon atoms in each alkyl moiety;
   m, n and p are each, independently, 0, 1, 2 or 3; and
   R¹ and R² may be connected to form a ring;
   or a pharmaceutically acceptable salt of such compound.
   Preferred embodiments of this invention include, for the said use,
   a compound as defined in paragraph (69) below, or a pharmaceutically acceptable salt of such compound.
(69) A compound of the formula XV, wherein said compound is selected from the group consisting of:
   (3R,4S,5S,6S)-N-carbamoylmethyl-5-(5-isopropyl-2-methoxybenzylamino)-6-diphenylmethyl-1-azabicyclo[2.2.2]octane-3-carboxamide;
   (3R,4S,5S,6S)-N-carboxymethyl-5-(5-isopropyl-2-methoxybenzylamino)-6-diphenylmethyl-1-azabicyclo[2.2.2]octane-3-carboxamide;
   (3R,4S,5S,6S)-3-(2-carbamoylpyrrolidin-1-yl)carbonyl-5-(5-isopropyl-2-methoxybenzylamino)-6-diphenylmethyl-1-azabicyclo[2.2.2]octane;
   (3R*,4S*,5S*,6S*)-N-(1-carbamoylethyl)-5-(5-isopropyl-2-methoxybenzylamino)-6-diphenylmethyl-1-azabicyclo[2.2.2]octane-3-carboxamide;
   (3R,4S,5S,6S)-N-(1-carbamoyl-3-methylbutyl)-5-(5-isopropyl-2-methoxybenzylamino)-6-diphenylmethyl-1-azabicyclo[2.2.2]octane-3-carboxamide; and
   (3R,4S,5S,6S)-N-(2-carbamoylethyl)-5-(5-isopropyl-2-methoxybenzylamino)-6-diphenylmethyl-1-azabicyclo[2.2.2]octane-3-carboxamide.
   This invention also relates, for the said use, to a compound of the formula 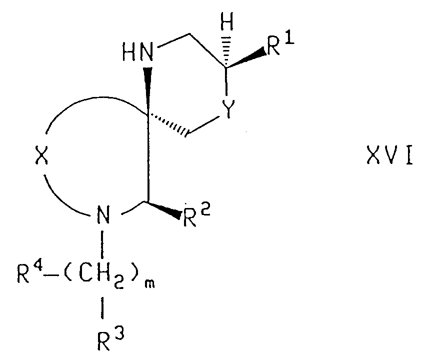 wherein R¹ is phenyl optionally substituted with one or more substituents, preferably with from one to three substituents, independently selected from hydrogen, halo, nitro, (C₁-C₁₀) alkyl optionally substituted with from one to three fluorine atoms, (C₁-C₁₀) alkoxy optionally substituted with from one to three fluorine atoms, trifluoromethyl, hydroxy, phenyl, cyano, amino, (C₁-C₆)-alkylamino, di-(C₁-C₆)alkylamino, 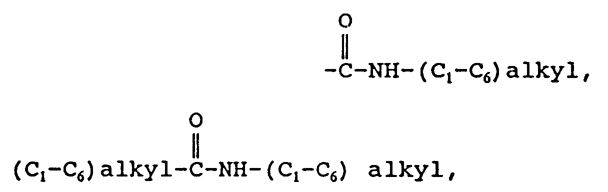 hydroxy(C₁-C₄)alkyl, 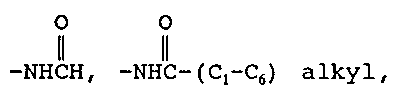 (C₁-C₄)alkoxy(C₁-C₄)alkyl, -S(O)ᵥ-(C₁-C₁₀)-alkyl wherein v is zero, one or two, -S(O)ᵥ-aryl wherein v is zero, one or two, -O-aryl, -SO₂NR⁴R⁵ wherein each of R⁴ and R⁵ is, independently, (C₁-C₆)alkyl, or R⁴ and R⁵, together with the nitrogen to which they are attached, form a saturated ring containing one nitrogen and from 3 to 6 carbons, 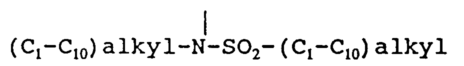 wherein one or both of the alkyl moieties may optionally be substituted with from one to three fluorine atoms, -N(SO₂-(C₁-C₁₀)alkyl)₂ and 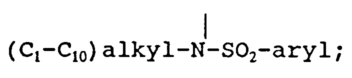 and wherein the aryl moieties of said -S(O)ᵥ-aryl, -O-aryl and 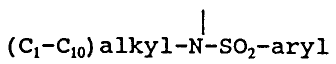 are independently selected from phenyl and benzyl and may optionally be substituted with from one to three substituents independently selected from (C₁-C₄)alkyl, (C₁-C₄)alkoxy and halo;
   or R¹ is phenyl substituted with a group having the formula 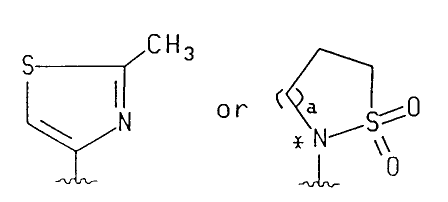 wherein a is 0, 1 or 2 and the asterisk represents a position meta to the point of attachment of R¹;
   R² is selected from (C₁-C₆) straight or branched alkyl, (C₃-C₇) cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents, preferably with from one to three substituents, independently selected from halo, nitro, (C₁-C₁₀) alkyl optionally substituted with from one to three fluorine atoms, (C₁-C₁₀) alkoxy optionally substituted with from one to three fluorine atoms, amino, hydroxy-(C₁-C₆)alkyl, (C₁-C₆)alkoxy-(C₁-C₆)alkyl, (C₁-C₆)-alkylamino, 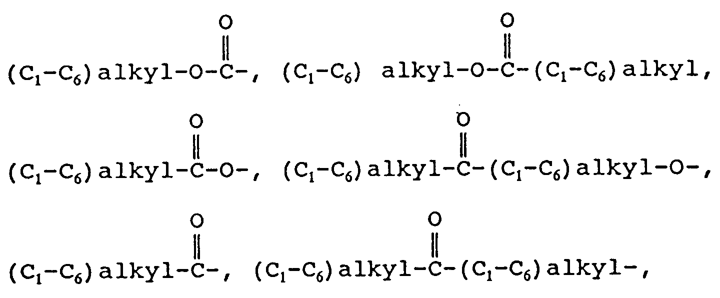 di-(C₁-C₆)alkylamino, 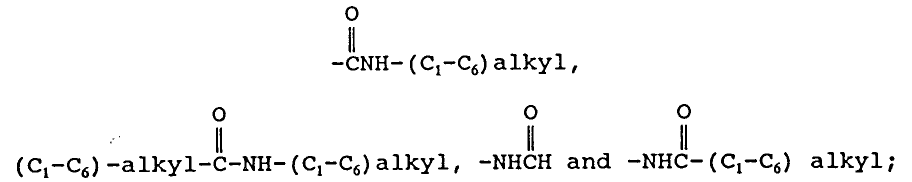 and wherein one of the phenyl moieties of said benzhydryl may optionally be replaced by naphthyl, thienyl, furyl or pyridyl;
   m is an integer from 0 to 8, and any one of the carbon-carbon single bonds of (CH₂)ₘ, wherein both carbon atoms of such bond are bonded to each other and to another carbon atom in the (CH₂)ₘ chain, may optionally be replaced by a carbon-carbon double bond or a carbon-carbon triple bond, and any one of the carbon atoms of said (CH₂)ₘ may optionally be substituted with R⁴;
   R³ is selected from 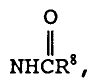 NHCH₂R⁸, SO₂R⁸, AR⁹, CO₂H and the radicals set forth in the definitions of R², R⁶ and R⁷;
   A is CH₂, nitrogen, oxygen, sulfur or carbonyl;
   R⁸ is (C₁-C₆)alkyl, hydrogen, phenyl or phenyl (C₁-C₆)alkyl;
   R⁴ is selected from oximino (=NOH) and the radicals set forth in the definitions of R², R⁶ and R⁷;
   R⁹ is a monocyclic or bicyclic heterocycle selected from the group consisting of pyrimidinyl, benzoxazolyl, 2,3-dihydro-3-oxobenzisosulfonazol-2-yl, morpholin-1-yl, thiomorpholin-1-yl, benzofuranyl, benzothienyl, indolyl, isoindolyl, isoquinolinyl, furyl, pyridyl, isothiazolyl, oxazolyl, triazolyl, tetrazolyl, quinolyl, thiazolyl, thienyl, and groups of the formulae 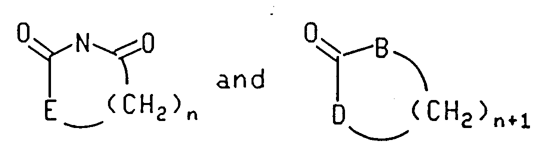 wherein B and D are selected from carbon, oxygen and nitrogen, and at least one of B and D is other than carbon; E is carbon or nitrogen; n is an integer from 1 to 5; any one of the carbon atoms of said (CH₂)ₙ and (CH₂)ₙ₊₁ may be optionally substituted with (C₁-C₆)alkyl or (C₂-C₆) spiroalkyl; and either any one pair of the carbon atoms of said (CH₂)ₙ and (CH₂)ₙ₊₁ may be bridged by a one or two carbon atom linkage, or any one pair of adjacent carbon atoms of said (CH₂)ₙ and (CH₂)ₙ₊₁ may form, together with from one to three carbon atoms that are not members of the carbonyl containing ring, a (C₃-C₅) fused carbocyclic ring;
   X is (CH₂)_{q} wherein q is two or three and wherein one of the carbon-carbon single bonds in said (CH₂)_{q} may optionally be replaced by a carbon-carbon double bond, and wherein any one of the carbon atoms of said (CH₂)_{q} may optionally be substituted with R⁶, and wherein any one of the carbon atoms of said (CH₂)_{q} may optionally be substituted with R⁷;
   R⁶ and R⁷ are independently selected from hydrogen, hydroxy, halo, amino, oxo (=O), cyano, hydroxy-(C₁-C₆)alkyl, (C₁-C₆)alkoxy-(C₁-C₆)alkyl, (C₁-C₆)alkylamino, di-(C₁-C₆)alkylamino, (C₁-C₆)alkoxy, 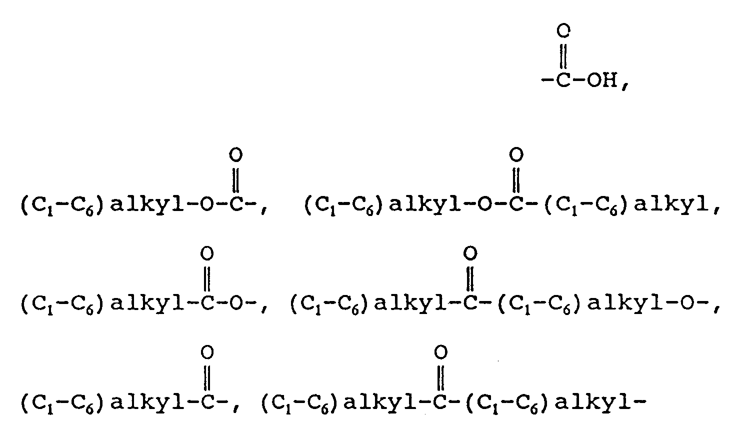 and the radicals set forth in the definition of R²; and
   Y is (CH₂)_{z} wherein z is zero or one;
   with the proviso that: (a) when A is -(CH₂)- or carbonyl, R⁹ cannot be furyl, pyridyl, isothiazolyl, oxazolyl, triazolyl, tetrazolyl, quinolyl, thiazolyl or thienyl; (b) when m is zero, one of R³ and R⁴ is absent and the other is hydrogen; and (c) when R⁶ or R⁷ is attached to a carbon atom of X that is adjacent to the ring nitrogen, then R⁶ or R⁷, respectively, must be a substituent wherein the point of attachment is a carbon atom;
   or a pharmaceutically acceptable salt of such compound.
   Preferred embodiments of this invention include, for the said use,
   a compound as defined in paragraph (70-75) below, or a pharmaceutically acceptable salt of such compound.
(70) A compound of the formula XVI wherein z is one.
(71) A compound of the formula XVI wherein q is three.
(72) A compound of the formula XVI wherein q is three, m is zero, R³ is hydrogen and R⁴ is absent.
(73) A compound of the formula XVI wherein R¹ is phenyl substituted with from one to three substituents independently selected from (C₁-C₆)alkyl optionally substituted with from one to three fluorine atoms and (C₁-C₆)alkoxy optionally substituted with from one to three flourine atoms.
(74) A compound of the formula XVI wherein z is one, m is zero, R⁴ is absent, and each of R³, R⁶ and R⁷ is hydrogen.
(75) A compound of the formula XVI that is selected from the group consisting of:
   (±)-[3R-[3α, 6α (R*)]]-3-phenyl-7-phenyl-1,8-diazaspiro[5.5]undecane; and
   (±)-[3R-[3α, 6α (R*)]]-3-(2-methoxyphenyl)-7-phenyl-1,8-diazaspiro[5.5]undecane.
   Other compounds of the formula I include the following: (±)-[3R-[3α,6α(R*)]]-3-(2-methoxy-5-trifluoromethoxyphenyl)-7-phenyl-1,8-diazaspiro[5.5]undecane;
   (±)-[3R-[3α, 6α(R*)]]-3-(5-chloro-2-methoxyphenyl)-7-phenyl-1,8-diazaspiro[5.5]undecane;
   (±)-[3R-[3α,6α(R*)]]-3-(5-isopropyl-2-methoxyphenyl)-7-phenyl-1,8-diazaspiro[5.5]undecane;
   (±)-[3R-[3α, 6α (R*)]]-3-(5-tert.butyl-2-methoxyphenyl)-7-phenyl-1,8-diazaspiro[5.5]undecane;
   (±)-[3R-[3α, 6α (R*)]]-3-(2-methoxy-5-(N-methyl-N-methylsulfonylaminophenyl)-7-phenyl-1,8-diazaspiro[5.5]undecane;
   (±)-[3R-[3α, 6α (R*)]]-3-(2-iodophenyl)-7-phenyl-1,8-diazaspiro[5.5]undecane;
   (±)-[3R-[3α, 6α (R*)]]-3-(2-methoxy-4-methylphenyl)-7-phenyl-1,8-diazaspiro[5.5]undecane;
   (±)-[3R-[3α,6α(R*)]]-3-(2-isopropoxyphenyl)-7-phenyl-1,8-diazaspiro[5.5]undecane;
   (±)-[3R-[3α, 6α (R*)]]-3-(2-difluoromethoxy-5-trifluoromethoxyphenyl)-7-phenyl-1,8-diazaspiro[5.5]undecane;
   (±)-[3R-[3α, 5α (R*)]]-3-(2-methoxyphenyl)-6-phenyl-1,7-diazaspiro[4.5]decane;
   (±)-[3R-[3α, 5α (R*)]]-3-(2-methoxy-5-trifluoromethoxyphenyl)-6-phenyl-1,7-diazaspiro[4.5]decane;
   (±)-[3R-[3α, 5α (R*)]]-3-(5-chloro-2-methoxyphenyl)-6-phenyl-1,7-diazaspiro[4.5]decane;
   (±)-[3R-[3α,5α(R*)]]-3-(5-isopropyl-2-methoxyphenyl)-6-phenyl-1,7-diazaspiro[4.5]decane; and
   (±)-[3R-[3α, 5α (R*)]]-3-(5-tert.butyl-2-methoxyphenyl)-6-phenyl-1,7-diazaspiro[4.5]decane.

This invention also relates to a topical pharmaceutical composition for the said use, said composition comprising a compound of the formula Ia, Ib, Ic, Id, Ie, X, XI, XII, XIII, XIV, XV or XVI and a pharmaceutically acceptable carrier.

This invention also relates to a topical pharmaceutical composition for the said use, said composition comprising a substance P receptor antagonist and a pharmaceutically acceptable carrier.

This invention also relates to a combination topical pharmaceutical composition for the said use, said composition comprising a compound of the formula Ia, Ib, Ic, Id, Ie, X, XI, XII, XIII, XIV, XV or XVI and a second therapeutic agent effective in preventing sunburn and a pharmaceutically acceptable carrier.

This invention also relates to a combination topical pharmaceutical composition for the said use, said composition comprising a substance P receptor antagonist and a second therapeutic agent effective in treating or preventing sunburn and a pharmaceutically acceptable carrier.

The term "halo", as used herein, unless otherwise indicated, includes chloro, fluoro, bromo and iodo.

The term "alkyl", as used herein, unless otherwise indicated, includes saturated monovalent hydrocarbon radicals having straight, branched or cyclic moieties or combinations thereof.

The term "alkenyl", as used herein, unless otherwise indicated, refers to straight or branched hydrocarbon chain radicals having one double bond including, but not limited to, ethenyl, 1- and 2-propenyl, 2-methyl-1-propenyl, 1- and 2-butenyl.

The term "alkoxy", as used herein, unless otherwise indicated, refers to -O-alkyl, wherein alkyl is defined as above, and includes, but is not limited to methoxy, ethoxy, propoxy, isopropoxy, n-butoxy, isobutoxy and t-butoxy.

The term "alkylthio", as used herein, unless otherwise indicated, refers to -S-alkyl, wherein alkyl is defined as above, and includes, but is not limited to methylthio, ethylthio, n-propylthio, isopropylthio, n-butylthio, isobutylthio, and t-butylthio.

The term "cycloalkyl", as used herein, unless otherwise indicated, refers to cyclic hydrocarbon radicals including, but not limited to cyclopropyl, cyclopropyl, cyclobutyl, cyclopentyl and cyclohexyl.

The term "one or more substituents," as used herein, includes from one to the maximum number of substituents possible based on the number of available bonding sites.

Compounds of the formulae I, X, XI, XII, XIII, XIV, XV and XVI contain chiral centers and therefore exist in different enantiomeric forms. The above definitions of these compounds include all optical isomers and all stereoisomers of such compounds, and mixtures thereof.

The therapeutic agents used in the invention are active in the treatment and prevention of sunburn. Prevention of sunburn refers to minimizing the deleterious effects on human skin caused by excessive exposure to ultraviolet B (290 to 320 nm) and ultraviolet A (320 to 400 nm) radiation by administration of a composition of the invention before erythema can be detected. Treatment of sunburn refers to minimizing the response to excessive exposure to ultraviolet B (290 to 320 nm) and ultraviolet A (320 to 400 nm) radiation after exposure has occurred.

The treatment of sunburn also includes the treatment of any ultraviolet (UV) radiation induced erythema. Ultaviolet will be understood by those skilled in the art to include both UVA and UVB wavelength radiation. Sunburn includes acute sunburn as well as the hazards associated with long term UV radiation exposure such as: premature aging of the skin, characterized by wrinkling and yellowing of the skin, telangiectasis, solar keratoses, ecchymoses and loss of skin elasticity. Substance P receptor antagonists are useful in the treatment of sunburn, however induced. For example, sunburn may be induced by exposure to natural sunlight, or artificial UV sources such as tanning lamps.

Other substance P receptor antagonists that are expected to exhibit activity for the treatment or prevention of sunburn are those compounds described in the following references: European Patent Publication 499,313 published August 19, 1992; European Patent Publication 520,555 published December 30, 1992; European Patent Publication 522,808 published January 13, 1993, European Patent Publication 528,495 published February 24, 1993, PCT Patent Publication WO 93/14084 published July 22, 1993, PCT Patent Publication WO 93/01169 published January 21, 1993, PCT Patent Publication WO93/01165 published January 21, 1993, PCT Patent Publication WO 93/01159 published January 21, 1993, PCT Patent Publication WO 92/20661 published November 26, 1992, European Patent Publication 517,589 published December 12, 1992, European Patent Publication 428,434 published May 22, 1991, and European Patent Publication 360,390 published March 28, 1990.

### Detailed Description of the Invention

The compounds of the formulae Ia, Ib, Ic, Id, Ie, X, XI, XII, XIII, and XIV may be prepared as described below. Unless otherwise indicated, in the discussion that follows, structural formulae Ia, Ib, Ic, Id, Ie, X, XI XII, XIII, and XIV, and groups II, III, IV, V, VI, VII, VIII and IX are defined as above.

Compounds of the formula Ia and Ib may be prepared as described in United States Patent Application of 988,653, which was filed on December 10, 1992. This application is incorporated herein by reference in its entirety.

Compounds of the formula Ic may be prepared as described in United States Patent Application 932,392, which was filed on August 19, 1992 and PCT Patent Application PCT/US93/04063 filed on May 5, 1993. These applications are incorporated herein by reference in its entirety.

Compounds of the formula Id may be prepared as described in PCT Patent Application PCT/US 92/03571, which designates the United States and was filed in the United States Receiving Office on May 5, 1992 and was published as WO93/00331 on January 7, 1993. This application is incorporated herein by reference in its entirety.

Compounds of the formula Ie may be prepared as described in United States Patent Application 123,306, which was filed on September 17, 1993. This application is incorporated herein by reference in its entirety.

When R³ is a group of the formula II, the starting materials of the formula NH₂R³ that are used in the preparation of compounds of the formulae Ia, Ib, Ic, Id and Ie may be prepared as described in United States Patent 5,162,339, which issued on November 11, 1992. This patent is incorporated herein by reference in its entirety.

When R³ is a group of the formula III, the starting materials of the formula NH₂R³ that are used in the preparation of compounds of the formulae Ia, Ib, Ic, Id and Ie may be prepared as described in PCT Patent Application PCT/US 91/02853, which designates the United States, was filed in the United States Receiving Office on April 25, 1991 and was published as WO 91/18899 on December 12, 1992. This application is incorporated herein by reference in its entirety.

When R³ is a group of the formula IV, V or VI, the starting materials of the formula NH₂R³ that are used in the preparation of compounds of the formulae Ia, Ib, Ic, Id and Ie may be prepared as described in PCT Patent Application PCT/US 91/03369, which designates the United States, was filed on in the United States Receiving Office May 14, 1991 and was published as WO 92/01688 on February 6, 1992. This application is incorporated herein by reference in its entirety.

When R³ is a group of the formula VII, the starting materials of the formula NH₂R³ that are used in the preparation of compounds of the formulae Ia, Ib, Ic, Id and Ie may be prepared as described in United States Patent 5,232,929 which issued on August 3, 1993, United States Patent Application 800,667, filed November 27, 1991 and PCT Patent Application PCT/US 92/00065, which designates the United States, was filed in the United States Receiving Office on January 14, 1992 and was published as WO 92/17449 on October 15, 1992. These applications are incorporated herein by reference in their entirety.

When R³ is a group of the formula VIII, the starting materials of the formula NH₂R³ that are used in the preparation of compounds of the formulae Ia, Ib, Ic, Id and Ie may be prepared as described in PCT Patent Application PCT/US 91/05776, which designates the United States, was filed in the United States Receiving Office on August 20, 1991 and was published as WO 92/06079 on April 16, 1992, United States Patent Application 800,667, filed November 27, 1991, PCT Patent Application PCT/US 92/00065, which designates the United States, was filed in the United States Receiving Office on January 14, 1992 and was published as WO 92/17449 on October 15, 1992 and United States Patent Application 924,773 which was filed August 4, 1992 . These applications are incorporated herein by reference in their entirety.

When R³ is a group of the formula IX, the starting materials of the formula NH₂R³ that are used in the preparation of compounds of the formulae Ia, Ib, Ic, Id and Ie may be prepared as described in United States Patent Application Serial No. 719,884, filed June 21, 1991 and PCT Patent Application PCT/US 92/04697, which designates the United States and was filed in the United States Receiving Office on June 11, 1992 and was published as WO93/00330 on January 7, 1993. These applications are incorporated herein by reference in their entirety.

Compounds of the formula X may be prepared as described in PCT Patent Application PCT/US 92/04002, which designates the United States, was filed in the United States Receiving Office on May 19, 1992 and was published as WO 92/20676 on November 26, 1992. This application is incorporated herein by reference in its entirety.

Compounds of the formula XI may be prepared as described in PCT Patent Application PCT/US 92/04697, which designates the United States, was filed in the United States Receiving Office on June 11, 1992 and was published as WO93/00330 on January 7,1993. This application is incorporated herein by reference in its entirety.

Compounds of the formula XII may be prepared as described in PCT Patent Application PCT/US 92/07730, which designates the United States and was filed in the United States Receiving Office on September 18, 1992 and was published as WO93/10073 on May 27, 1993. This application is incorporated herein by reference in its entirety.

Compounds of the formula XIII may be prepared as described in PCT Patent Application PCT/US 92/06819, which designates the United States and was filed in the United States Receiving Office on August 20, 1992 and published as WO93/06099 on April 1, 1993. This application is incorporated herein by reference in its entirety.

Compounds of the formula XIV may be prepared as described in United States Patent Application 885,110, which was filed on May 18, 1992 and PCT Patent Application PCT/US93/01429 filed on February 23, 1993. This application is incorporated herein by reference in its entirety.

Compounds of the formula XV may be prepared by the procedure described in Japanese Patent Application 065337/92, which was filed on March 23, 1992 and PCT Patent Application PCT/US93/01810 filed on March 5, 1993. These procedures are depicted in Schemes 1, 2 and 3 and discussed below. In the reaction schemes and discussion that follow, A, X¹, X², Ar¹ and Ar² are defined as in the definition of compounds of the formula XV above.

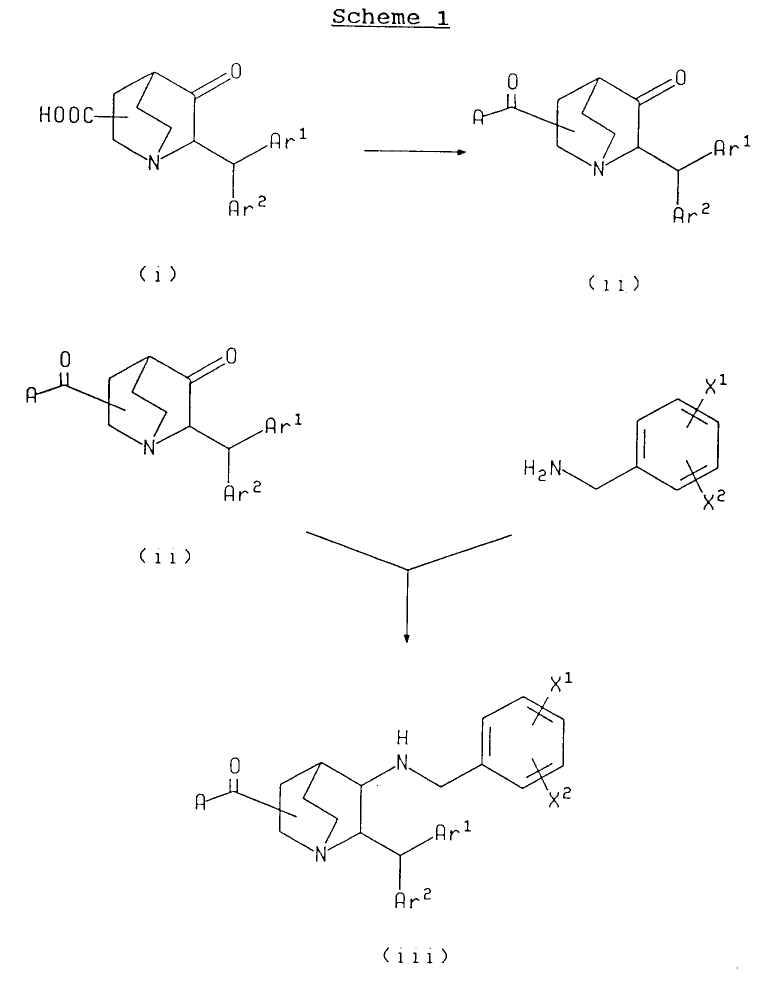

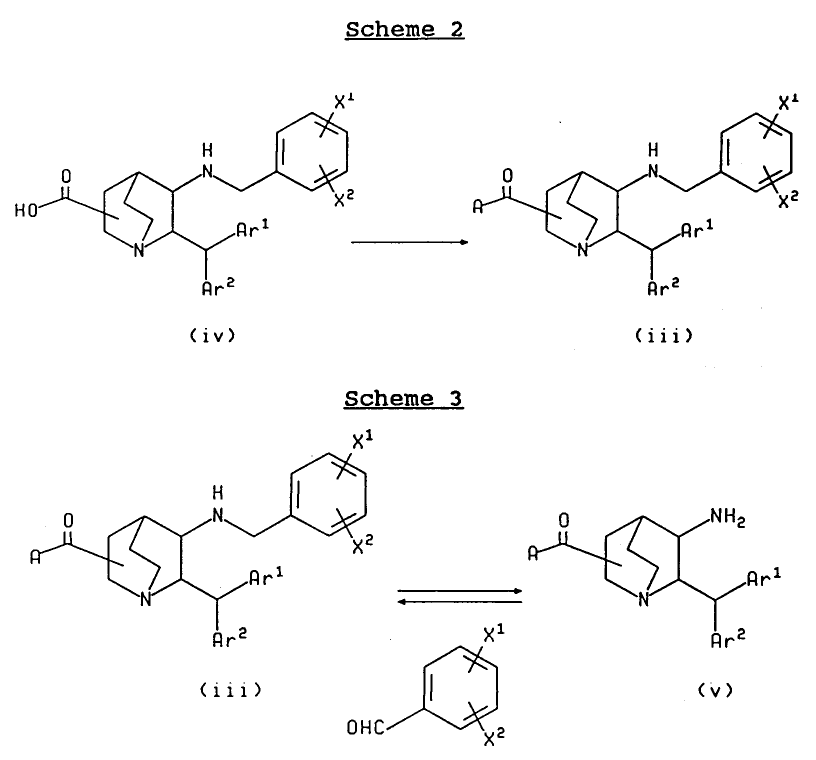

Referring to Scheme 1, the starting materials of the formula (i) may be prepared by the procedures described in PCT Patent Application PCT/US 92/04002, which was published as WO 92/15585 on September 17, 1992. This application is incorporated herein by reference in its entirety.

Introduction of a protected amino acid into a compound of the formula (i) to give a compound of the formula (ii) can be carried out by a variety of conventional methods for peptide synthesis, as described in "Peptide synthesis, the basis and experiments", edited by N. Izumiya, 1985 (Maruzen).

Such methods include an activated ester method that employs an acid chloride or mixed acid anhydride, and a condensation method that employs an appropriate condensing agent selected from dicyclohexylcarbodiimide (DCC), water soluble carbodiimide, 2-ethoxy-N-ethoxycarbonyl-1,2-dihydroquinoline, Bop agent, diethylcyanophosphonic acid and diphenylphospolylazide.

If necessary, addition of a tertiary amide such as triethylamine can promote the condensation reaction. N-hydroxysuccinimide, N-hydroxybenzotriazole or 3,4-dihydro-3-hydroxy-4-oxo-1,2,3-benzotriazine may be employed to prevent racemization.

Typically, a compound of the formula (ii) can be prepared from a compound of the formula (i) and an amino acid or its salt, which is protected by an amino group or a mono- or dialkyl substituted amino group, by the use of a peptide coupling reagent such as DCC or diethylcyanophosphonic acid in a reaction inert solvent such as methylene chloride, THF or DMF, in the presence of triethylamine.

The resulting compound of the formula (ii) may be converted into a compound of the formula (iii) by reductive amination. This route involves direct introduction of the appropriate benzylamino group at the 3-position of the quinuclidine, and is typically conducted in two steps. In the first step, the imine formation from the compound of formula (ii) and the benzylamine is carried out by heating the reactants at the reflux temperature in a reaction inert solvent such as toluene or benzene, in the presence of catalytic amount of acid (e.g., p-toluenesulfonate or camphorsulfonic acid (CSA)) under dehydrolytic conditions. Alternatively, a Lewis acid such as aluminum chloride or titanium tetrachloride can be used as the acid catalyst. Under such catalytic conditions, and at temperatures from about -78°C to about room temperature, it is preferable to use an acetonitrile or methylene chloride solvent together with a dehydrating agent such as molecular sieves.

In the second step, the imine is reduced to afford the compound of the formula (iii). This reduction can be carried out by either catalytic hydrogenation, or by reaction with a suitable hydride reagent such as a borohydride, borane or aluminum hydride. Typically, a reagent such as NaBH₄, NaBH₃CN or NaBH(OAc)₃ in the presence of acetic acid is used.

The above two reaction steps can be carried out simultaneously. In such cases, the reaction is preferably carried out using NaBH₃CN in methanol in the presence of acetic acid.

The compound of the formula (iii) can be converted into the corresponding carboxylic of formula (iv) acid by acidic hydrolysis in an inorganic acid such as hydrochloric acid at a temperature from about room temperature to about the reflux temperature for about 30 minutes to several hours.

The resulting carboxylic acid can be converted to the corresponding ester by heating it in an alcoholic solvent in the presence of an acid catalyst.

Alternatively, the compound of formula (iii) can be prepared by the procedure illustrated in Scheme 2. Referring to Scheme 2, the compound of formula (iii) can be prepared by peptide condensation from a compound of the formula (iv) and an amino acid which is protected at its carboxyl moiety. The generic synthetic condition for various peptide synthetic methods described in the above discussion of Scheme 1 can be used in this reaction.

The procedure illustrated in Scheme 3 can also be used to prepare compounds of the formula (iii). Using this procedure, compounds of the formula (iii) can be prepared by reductive amination of 3-amino quinuclidines of the formula (v) having an amino acid as the substituent A with the corresponding substituted benzaldehyde. This reductive amination reaction proceeds easily under standard reaction conditions because it goes by way of a stable imine intermediate. Use of a borane reducing agent (e.g. NaBH₃CN or NaBH(Oac)₃ etc.) is preferred.

The starting material of formula (v) can be obtained by debenzylation of the compound of formula (iii). The debenzylation is preferably accomplished by hydrogenolysis with a palladium catalyst (e.g., palladium or palladium hydroxide) which scarcely affects the other functional groups in the compound of formula (v).

The compounds of formula (iii) prepared by the above methods can be isolated and purified by conventional procedures, such as recrystallization or chromatography.

Compounds of the formula XVI may be prepared as described in United States Patent Application 026,382, which was filed on April 7, 1993. This application is incorporated herein by reference in its entirety.

The compounds of the formulae Ia, Ib, Ic, Id, Ie X, XI, XII, XIII, XIV, XV and XVI and other substance P receptor antagonists (hereinafter collectively referred to as the "therapeutic agents") and the pharmaceutically acceptable salts thereof are useful as substance P receptor antagonists, i.e., they possess the ability to antagonize the effects of tachykinins at the substance P receptor site in mammals, and therefore they are able to function as therapeutic agents in the treatment and prevention of sunburn in an afflicted mammal.

The therapeutic agents that are basic in nature are capable of forming a wide variety of different salts with various inorganic and organic acids. Examples of acids that form suitable pharmaceutically acceptable salts for use in this invention are those that form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [i.e., 1,1'-methylene-bis-(2-hydroxy-3-naphthoate)]salts.

Although such salts must be pharmaceutically acceptable for administration to animals, it is often desirable in practice to initially isolate a therapeutic agent from the reaction mixture as a pharmaceutically unacceptable salt and then simply convert the latter back to the free base compound by treatment with an alkaline reagent and subsequently convert the latter free base to a pharmaceutically acceptable acid addition salt. The acid addition salts of the base therapeutic agents of this invention are readily prepared by treating the base compound with a substantially equivalent amount of the chosen mineral or organic acid in an aqueous solvent medium or in a suitable organic solvent, such as methanol or ethanol. Upon careful evaporation of the solvent, the desired solid salt is readily obtained.

Those therapeutic agents of this invention that are also acidic in nature are capable of forming base salts with various pharmacologically acceptable cations. The chemical bases that are used as reagents to prepare the pharmaceutically acceptable base salts of the therapeutic agents are those that form non-toxic base salts with the acidic therapeutic agents. Such non-toxic base salts include those derived from such pharmacologically acceptable cations as sodium, potassium, calcium and magnesium, etc. These salts can easily be prepared by treating the corresponding acidic compounds with an aqueous solution containing the desired pharmacologically acceptable cations, and then evaporating the resulting solution to dryness, preferably under reduced pressure. Alternatively, they may also be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum yields of the desired final product.

The therapeutic agents and their pharmaceutically acceptable salts exhibit substance P receptor-binding activity and therefore are of value in the treatment and prevention of sunburn in mammals, including humans.

The therapeutic agents and the pharmaceutically acceptable salts thereof can be administered via either the oral, topical, rectal or parenteral routes.

In general, when these, compounds are administered orally, parenterally or rectally, they are most desirably administered in dosages ranging from about 1 mg up to about 10 mg per day, although variations will necessarily occur depending upon the weight and condition of the subject being treated and the particular route of administration chosen. However, a dosage level that is in the range of about 0.2 mg to about 0.6 mg per kg of body weight per day is most desirably employed. Variations may nevertheless occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day.

Typical unit dosage forms for topical administration will contain about 0.5 wt. % to about 10 wt. %, preferably about 1 wt. % to about 3.5 wt. %, most preferably about 2.5 wt. % to about 3.5 wt. % of the substance P receptor antagonist, based on the entire weight of the composition per topical unit dose application. If the composition is intended for sustained release such as by using microcapsules or microspheres, much larger amounts of the active ingredient would of course be incorporated into an individual unit.

The therapeutic agents and their pharmaceutically acceptable salts may be administered alone or in combination with pharmaceutically acceptable carriers or diluents by any of the routes previously indicated, and such administration may be carried out in single or multiple doses. More particularly, the novel therapeutic agents of this invention can be administered in a wide variety of different dosage forms, i.e., they may be combined with various pharmaceutically acceptable inert carriers in the form of tablets, capsules, lozenges, troches, hard candies, suppositories, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the therapeutic compounds of this invention are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a therapeutic agent in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

For topical administration, solutions or suspensions of a therapeutic agent in clear or milky lotions, gels, creams, ointments, sprays, lip balm, clothwipe, impregnated bandages and other topical and transdermal delivery devices may be employed.

Suitable solvents or vehicles, for instance, for the topical substance P receptor anagonist composition of the present invention includes methanol, ethanol, propanol, acetone, n-butyl alcohol, isobutyl alcohol and the like.

The therapeutic agents used in the invention are active in the treatment and prevention of sunburn. Prevention of sunburn refers to minimizing the deleterious effects on human skin caused by excessive exposure to ultraviolet B (290 to 320 nm) and ultraviolet A (320 to 400 nm) radiation by administration of a composition of the invention before erythema can be detected. Treatment of sunburn refers to minimizing the response to excessive exposure to ultraviolet B (290 to 320 nm) and ultraviolet A (320 to 400 nm) radiation after exposure has occurred.

The therapeutic agents of this invention may be administered alone or in combination with another active agent that is effective in the treatment or prevention of sunburn. Such other active agents include sunscreen-sunburn preventive agents, sunscreen-suntanning agents, and sunscreen-opaque sunblock agents. The FDA advisory review panel has generically defined a sunscreen agent as follows: "An ideal sunscreen vehicle would be stable, neutral, non-greasy, nondegreasing, nonirritating, nondehydrating, nondrying, odorless, and efficient on all kinds of human skin. It should hold at least 50% water, be easily compounded of known chemicals, and have infinite stability during storage". ***Federal Register***, 43, 38218 (1978).

The primary uses of sunscreens are to prevent sunburn and aid in the development of a tan. Secondarily, they serve to protect exposed areas of the body in susceptible individuals from the long-term hazards of skin cancer and premature aging. In addition, sunscreens can be used to protect against drug-related ultraviolet-induced photosensitivity.

For purposes of the present invention, the term "sunscreen agent" shall refer to the use of a substance P receptor antagonist, alone or with another active agent, as a sunscreen-sunburn preventive agent, a sunscreen-suntanning agent and/or a sunscreen-opaque sunblock agent. Each of those type of agents has been defined by the FDA advisory review panel as nonprescription topical analgesic, antirheumatic, otic, burn and sunburn prevention and treatment drug products as follows:

A sunscreen-sunburn preventive agent contains an active ingredient that absorbs 95% or more of the radiation in the ultraviolet range at wavelengths from 290-320 nm and thereby removes the sunburning rays;

A sunscreen-suntanning agent contains an active ingredient that absorbs at least 85% of the radiation in the ultra-violet range at wavelengths from 290-320 nm, but transmits ultraviolet wavelengths longer than 320 nm (such agents permit tanning in the average individual and also permits some erythema without pain);

A sunscreen-opaque sunblock agent has an opaque agent that reflects or scatters all radiation in the ultra-violet and visible range from 290-777 nm and thereby prevents or minimizes suntan and sunburn.

Examples of excipients and dilutants used in pharmaceutically acceptable topical commercial sunscreen and sunblock compositions are:
titanium dioxide, petrolatum, red petrolatum, benzophenone-3, isopropyl myristate, aloe vera extract, synthetic beeswax, cetyl palmitate, ceresin, lanolin, cetyl alcohol, alcohol, oleth-3 phosphate, synthetic spermaceti, glycerin, mineral oil, lanolin alcohol, cetyl stearyl glycol, lanolin oil, triethanolamine, carbomer 934, benzyl alcohol, menthol, camphor, essential oils, acrylic-acrylate copolymer, ammonium hydroxide, carbomer 934P, dimethicone, quaternium-15, stearic acid, stearyl alcohol, water, xanthan gum, SD alcohol 40, animal protein derivative, hydroxyethyl cellulose, choleth-24, hydroxypropyl cellulose, PPG-15 stearyl ether, propylene glycol dioctanoate, stearic acid, ozokerite, PEG-4 dilaurate, propylparaben, dihydroxyacetone, hydrocarbon oil, ointment base zinc oxide, opaque base, water-repellent cream base, caramel, perfume and flavors.

It would be advantageous for the topical composition of the present invention to have sufficient substantivity to withstand exposure of the skin to swimming, high humidity and sweating.

Generally, sunscreens should be applied approximately 30 minutes before exposure to the sun. However, there are exceptions, for instance, aminobenzoic acid and its esters are more effective if applied two hours before exposure. Pre-application of the topical substance P compounds composition prior to sun exposure to the skin is advantageous because it allows the substance P compounds to penetrate and perhaps bind with the skin.

Topical sunscreens can fall within one of two categories: (1) chemical, and (2) physical sunscreens. Chemical sunscreens contain one or more UV-absorbing chemicals, and upon application of a thin and invisible film, act as filters and do not allow the penetration of ultraviolet radiation to the viable cells of the epidermis. Chemical sunscreens are usually colorless because they do not contain any visible light-absorbing chemicals are, therefore, cosmetically acceptable to most persons provided they are a nonirritant to the skin and eyes, non-photosensitizing, stable, nonvolatile, and nonstaining to skin and clothes. Most of the commercial topical sunscreens contain one or more ultraviolet B absorbing chemicals in a moisturizing base. More recently, many leading brand-name sunscreens also contain ultraviolet A absorbing chemicals, especially the different benzophenones. The most widely used chemical sunscreens contain para-aminobenzoic acid (PABA), PABA esters (amyldimethyl PABA and octyldimethyl PABA), benzophenones (oxybenzone and sulisobenzone), cinnamates (octylmethoxy cinnamate and cinoxate), salicylates (homomenthyl salicylate) and anthranilates. To date, more than 21 such chemicals have been declared by the U.S. FDA as safe, effective agents in protecting skin against sunburn and are listed under Category I (safe and approved).

Several European sunscreen manufacturers often use p-methoxy-2-ethylhexylcinnamate, 2-phenylbenzimidazole-5-sulfonic acid, 2-phenyl-5-methoxybenzophenone, and 4-tert-butyl-4'-methoxydibenzoylmethane as ultraviolet A and B absorbing filters. The recommended concentration for each chemical may vary and is based on not only the solubility of the chemical in a given vehicle, but also the anticipated use of the sunscreen product as a total or partial block for the prevention of sunburn or acquisition of suntan responses. The formulation base (vehicle) used include alcohol plus glycerol or glycol, oil-in-water or water-in-oil lotion, cream, or ointment. The vehicle in which the ultraviolet radiation absorbing chemical is incorporated can determine whether a sunscreen remains effective under the general use condition involving prolonged sunbathing, sweating (sporting activities), and swimming. This adherent property to skin, known as "substantivity," varies considerably among commercially available sunscreen formulations, some of which are retained on the skin and others of which are washed off easily after sweating or swimming.

Physical sunscreens are usually opaque formulations and contain ingredients particulate in nature that do not selectively absorb ultraviolet radiation, but, when applied as a thin film, primarily reflect and scatter ultraviolet and visible radiation because of the size of the particles and the thickness of the film. These include titanium dioxide (5% to 20%), talc (magnesium silicate), magnesium oxide, zinc oxide, kaolin, ferric chloride, and ichthyol (ichthammol). Zinc oxide appears to be the most effective. These formulations are cosmetically unpleasing, unacceptable to many patients, and are often occlusive and messy to use. Physical sunscreens are, however, essential for those patients who are unusually sensitive to ultraviolet radiation as well as visible radiation; these are usually applied to limited areas such as the nose, lips, or helix of the ear.

Further, the sunburn/sunscreen product of the present invention may include a burn or sunburn treatment component such as an anesthetic, antimicrobial or another ingredient.

The anesthetic component of commercial products presently include:
benzocaine, lidocaine hydrochloride, butamben picrate, dibucaine, tetracaine hydrochloride, tripelennamine, and menthol benzocaine.

The antimicrobial component of commercial products currently include:
benzethonium chloride, benzalkonium chloride, povidoneiodine, chloroxylenol, chlorobutanol, 8-hydroxyquinoline, phenol, 8-hydroxyquinoline sulfate, cresol-camphor complex, chlorothymol, methylbenzethonium chloride, triclosan, benzyl alcohol, and parahydracin.

The activity of the therapeutic agents as substance P receptor antagonists may be determined by their ability to inhibit the binding of substance P at its receptor sites in bovine caudate tissue, employing radioactive ligands to visualize the tachykinin receptors by means of autoradiography. The substance P antagonizing activity of the herein described compounds may be evaluated by using the standard assay procedure described by M. A. Cascieri et al., as reported in the Journal of Biological Chemistry, Vol. 258, p. 5158 (1983). This method essentially involves determining the concentration of the individual compound required to reduce by 50% the amount of radiolabelled substance P ligands at their receptor sites in said isolated cow tissues, thereby affording characteristic IC₅₀ values for each compound tested.

In this procedure, bovine caudate tissue is removed from a -70°C freezer and homogenized in 50 volumes (w./v.) of an ice-cold 50 mM Tris (i.e., trimethamine which is 2-amino-2-hydroxymethyl-1,3-propanediol) hydrochloride buffer having a pH of 7.7. The homogenate is centrifuged at 30,000 x G for a period of 20 minutes. The pellet is resuspended in 50 volumes of Tris buffer, rehomogenized and then recentrifuged at 30,000 x G for another twenty- minute period. The pellet is then resuspended in 40 volumes of ice-cold 50 mM Tris buffer (pH 7.7) containing 2 mM of calcium chloride, 2 mM of magnesium chloride, 4 µg/ml of bacitracin, 4µg/ml of leupeptin, 2µg of chymostatin and 200 g/ml of bovine serum albumin. This step completes the production of the tissue preparation.

The radioligand binding procedure is then carried out in the following manner, viz., by initiating the reaction via the addition of 100 µl of the test compound made up to a concentration of 1 µM, followed by the addition of 100 µl of radioactive ligand made up to a final concentration 0.5 mM and then finally by the addition of 800 µl of the tissue preparation produced as described above. The final volume is thus 1.0 ml, and the reaction mixture is next vortexed and incubated at room temperature (ca. 20°C) for a period of 20 minutes. The tubes are then filtered using a cell harvester, and the glass fiber filters (Whatman GF/B) are washed four times with 50 mM of Tris buffer (pH 7.7), with the filters having previously been presoaked for a period of two hours prior to the filtering procedure. Radioactivity is then determined in a Beta counter at 53% counting efficiency, and the IC₅₀ values are calculated by using standard statistical methods.

The ability of the therapeutic agents to inhibit substance P induced effects in vivo may be determined by the following procedures "a" through "c". (Procedures "a" through "c" are described in Nagahisa et al., European Journal of Pharmacology, 217, 191-5 (1992), which is incorporated herein by reference in its entirety.)

### a. Plasma extravasation in the skin

Plasma extravasation is induced by intradermal administration of substance P (50 µl, 0.01% BSA-saline solution) in dorsal skin of pentobarbital (25 mg/kg i.p.) anesthetized male Hartley guinea pigs weighing 450-500 g. The compound to be tested is dissolved in 0.1% methyl cellulose-water (MC) and dosed p.o. 1 hour before substance P challenge (3 pmol/site). Evans blue dye (30 mg/kg) is administered intravenously 5 minutes before challenge. After 10 minutes, the animals are sacrificed, the dorsal skin is removed, and the blue spots are punched out using a cork borer (11.5 mm oral dose (o.d.)). Tissue dye content is quantitated after overnight formamide extraction at 600 nm absorbance.

### b. Capsaicin-induced plasma extravasation

Plasma extravasation is induced by intraperitoneal injection of capsaicin (10 ml of 30 µM solution in 0.1% BSA/saline) into pentobarbital anesthetized (25 mg/kg i.p.) guinea pigs. The compound to be tested is dissolved in 0.1% MC and dosed p.o. 1 hour before capsaicin challenge. Evans blue dye (30 mg/kg) is administered i.v. 5 minutes before challenge. After 10 minutes, the animals are sacrificed, and both right and left ureters are removed. Tissue dye content is quantitated as in "a" above.

### c. Acetic acid-induced abdominal stretching

Male ddY mice (SLC, Japan), weighing 14-18 g, were fasted overnight. The compound to be tested is dissolved in 0.1% MC and dosed p.o. 0.5 hour before acetic acid (AA) injection (0.7%, 0.16 ml/10 g body weight). The animals are placed in clear beakers (1 per beaker) and the stretching response is counted 10 to 20 minutes after the AA injection (10 minute interval).

The anti-sunburn activity of compounds that are substance P receptor antagonists may be determined by evaluating the compounds according to the following ultraviolet (UV) Erythema Model.

Male Hartley guinea pigs (5 weeks old) may be used for these experiments. The dorsal hair of the animals is removed 2 days before treatment with an electric shaver and hair remover cream (EBA cream Tokyo Tanabe Pharmaceuticals). The animals are then fasted over night.

The dorsal skin of the guinea pigs is then exposed to UV light (1650-1670 Lux, 60 sec) to induce a sunburn-like inflammation.

Erythema may be determined by visual scoring (0: none, 1: slightly and 2: clear) or by plasma extravasation. Plasma extravasation may be determined by Evans blue dye method (Guinea pigs are anesthetized with 25mg/kg i.p. of pentobarbitol and 10 minutes later, the dye 30 mg/kg is injected intravenous). The scoring and plasma extravasation may be determined at 2, 5, 18 and 24 hours after the UV-irradiation.

## Claims

1. The use
(a) of a compound of the formula 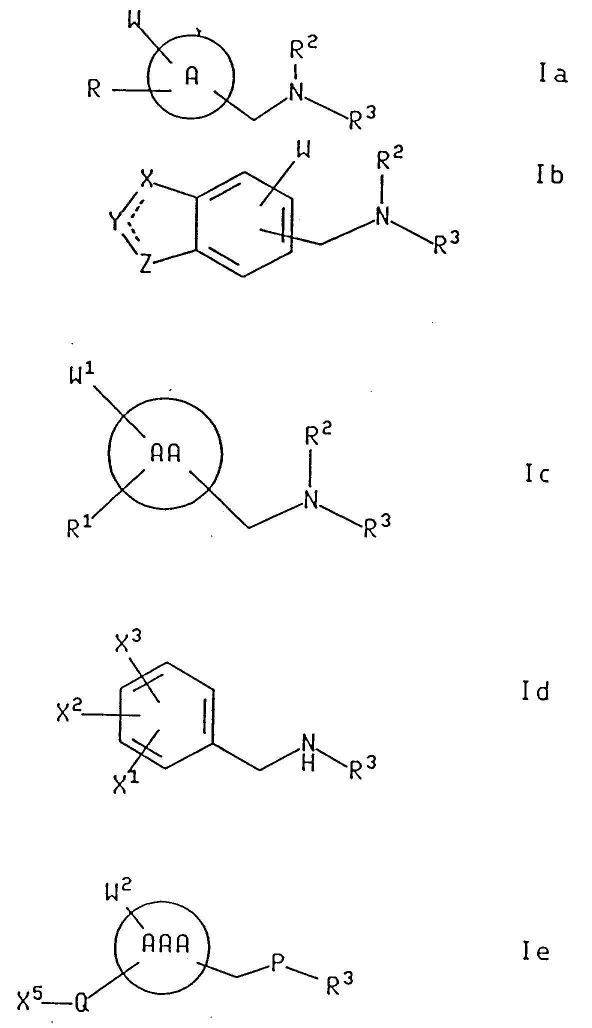 wherein A is a ring system selected from phenyl, naphthyl, thienyl, quinolinyl and indolinyl, and wherein the sidechain containing NR²R³ is attached to a carbon atom of ring system A;
AA is an aryl group selected from phenyl, naphthyl, thienyl, dihydroquinolinyl and indolinyl, and wherein the sidechain containing NR²R³ is attached to a carbon atom of AA;
AAA is an aryl group selected from phenyl, naphthyl, thienyl, dihydroquinolinyl and indolinyl, and wherein the - CH₂PR³ sidechain is attached to a carbon atom of ring A;
P is NR², O, S, SO or SO₂;
Q is SO₂, NH, 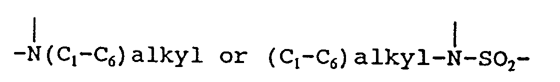 wherein the point of attachment of said 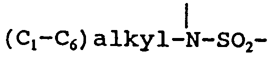 to ring A is the nitrogen atom and the point of attachment to R¹ is the sulfur atom;
W² is hydrogen, (C₁-C₆)alkyl, S-(C₁-C₃)alkyl, halo or (C₁-C₆)alkoxy optionally substituted with from one to three fluorine atoms;
W¹ is hydrogen, halo or; (C₁-C₆) alkyl, S-(C₁-C₃)alkyl, halo or (C₁-C₆) alkoxy optionally substituted with from one to three fluorine atoms;
W is hydrogen, (C₁-C₆)alkyl optionally substituted with from one to three fluorine atoms, -S(O)ᵥ-(C₁-C₆) alkyl wherein v is zero, one or two, halo or (C₁-C₆)alkoxy optionally substituted with from one to three fluorine atoms;
R is a 4, 5 or 6 membered heterocyclic ring containing from one to three heteroatoms selected from oxygen, nitrogen and sulfur (e.g., thiazolyl, azetidinyl, pyrrolyl, pyrazolyl, 1,2,3-triazolyl, 1,2,4-triazolyl, isothiazolyl, imidazolyl, isoxazolyl, or oxazolyl) wherein said heterocyclic ring may contain from zero to three double bonds and may optionally be substituted with one or more substituents, preferably one or two substituents, independently selected from (C₁-C₆) alkyl optionally substituted with from one to three fluorine atoms and (C₁-C₆) alkoxy optionally substituted with from one to three fluorine atoms;
R¹ is selected from amino, (C₁-C₆)alkylamino, di-(C₁-C₆)alkylamino, -S(O)ᵥ-(C₁-C₁₀)-alkyl wherein v is zero, one or two, -S(O)ᵥ-aryl wherein v is zero, one or two, -O-aryl, -SO₂NR⁴R⁵ wherein each of R⁴ and R⁵ is, independently, (C₁-C₆)alkyl, or R⁴ and R⁵, together with the nitrogen to which they are attached, form a saturated ring containing one nitrogen and from 3 to 6 carbons, 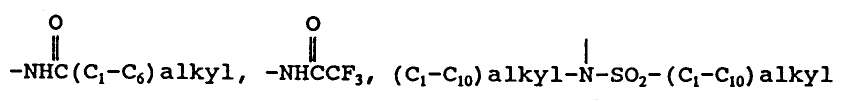 wherein one or both of the alkyl moieties may optionally be substituted with from one to three fluorine atoms, -N(SO₂-(C₁-C₁₀)alkyl)₂ and 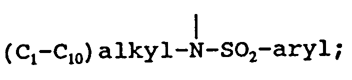 and wherein the aryl moieties of said -S(O)ᵥ-aryl, -O-aryl and 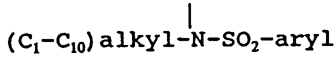 are independently selected from phenyl and benzyl and may optionally be substituted with from one to three substituents independently selected from (C₁-C₄)alkyl, (C₁-C₄)alkoxy and halo;
or R¹ is a group having the formula 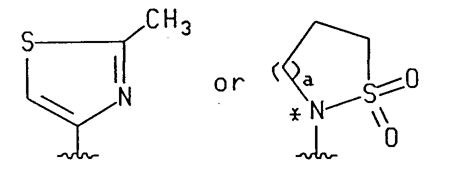 wherein a is 0, 1 or 2 and the asterisk represents a position meta to the R²R³NCH₂ side chain;
the dotted lines in formula Ib represent that one of the X-Y and Y-Z bonds may optionally be a double bond;
X is selected from =CH-, -CH₂-, -O-, -S-, -SO-, -SO₂-, -N(R⁴)-, -NH-, =N-, -CH[(C₁-C₆)alkyl]-, =C[(C₁-C₆)alkyl]-, -CH(C₆H₅)- and =C(C₆H₅)-;
wherein X¹ is hydrogen, (C₁-C₁₀)alkoxy optionally substituted with from one to three fluorine atoms or (C₁-C₁₀)alkyl optionally subsituted with from one to three fluorine atoms;
X² and X³ are independently selected from hydrogen, halo, nitro, (C₁-C₁₀)alkyl optionally substituted with from one to three fluorine atoms, (C₁-C₁₀)alkoxy optionally substituted with from one to three fluorine atoms, trifluoromethyl, hydroxy, phenyl, cyano, amino, hydroxy(C₁-C₄)alkyl, (C₁-C₄)alkoxy (C₁-C₄)alkyl,
(C₁-C₆)alkylamino, di-(C₁-C₆)alkylamino, 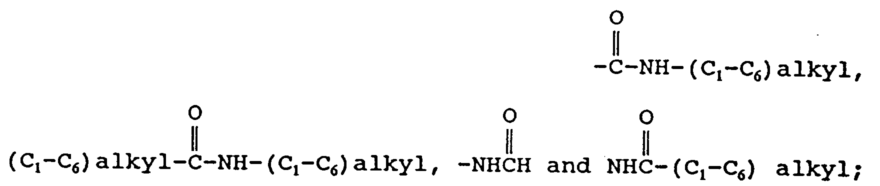 X⁵ is a four to six membered heterocyclic ring containing from one to three heteroatoms selected from sulfur, nitrogen and oxygen (e.g., thiazolyl, pyrrolyl, thienyl, triazolyl, oxazolyl, oxadiazolyl, thiadiazolyl or imidazolyl), wherein said heterocyclic ring may optionally be susbtituted with from one to three substituents, preferably with from zero to two substituents, independently selected from phenyl, (C₁-C₆)alkyl optionally substituted with from one to three fluorine atoms, (C₁-C₆)alkoxy optionally substituted with from one to three fluorine atoms and halo;
Y is selected from C=O, C=NR⁴, C=S, =CH-, -CH₂-, =C[(C₁-C₆)alkyl]-, -CH[(C₁-C₆)alkyl]-, =C(C₆H₅)-, -CH(C₆H₅)-, =N-, -NH-, -N(R⁴)-, =C(halo)-, =C(OR⁴)-, =C(SR⁴)-, =C(NR⁴)-, -O-, -S- and SO₂, wherein the phenyl moieties of said =C(C₆H₅)- and -CH(C₆H₅)- may optionally be substituted with from one to three substituents independently selected from trifluoromethyl and halo, and wherein the alkyl moieties of said =[(C₁-C₆)alkyl]- and -CH[C₁-C₆)alkyl]- may optionally be substituted with from one to three fluorine atoms;
Z is selected from =CH-, -CH₂-, =N-, -NH-, -S-, -N(R⁴)-, =C(C₆H₅)-, -CH(C₆H₅)-, =C[(C₁-C₆) alkyl]- and -CH[(C₁-C₆)alkyl]-;
or X, Y and Z, together with the two carbon atoms shared between the benzo ring and the XYZ ring, form a fused pyridine or pyrimidine ring;
R⁴ is (C₁-C₆) alkyl or phenyl;
R² is hydrogen or -CO₂(C₁-C₁₀)alkyl;
R³ is selected from 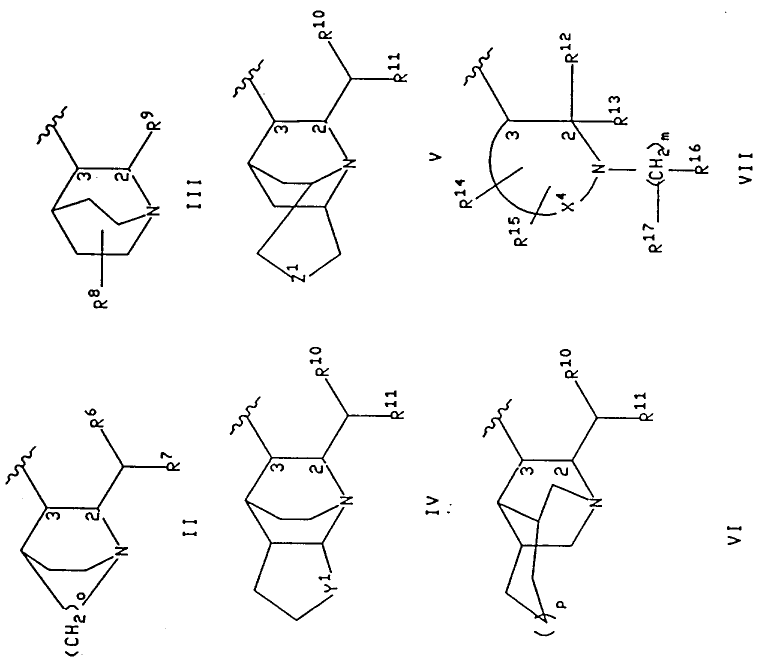 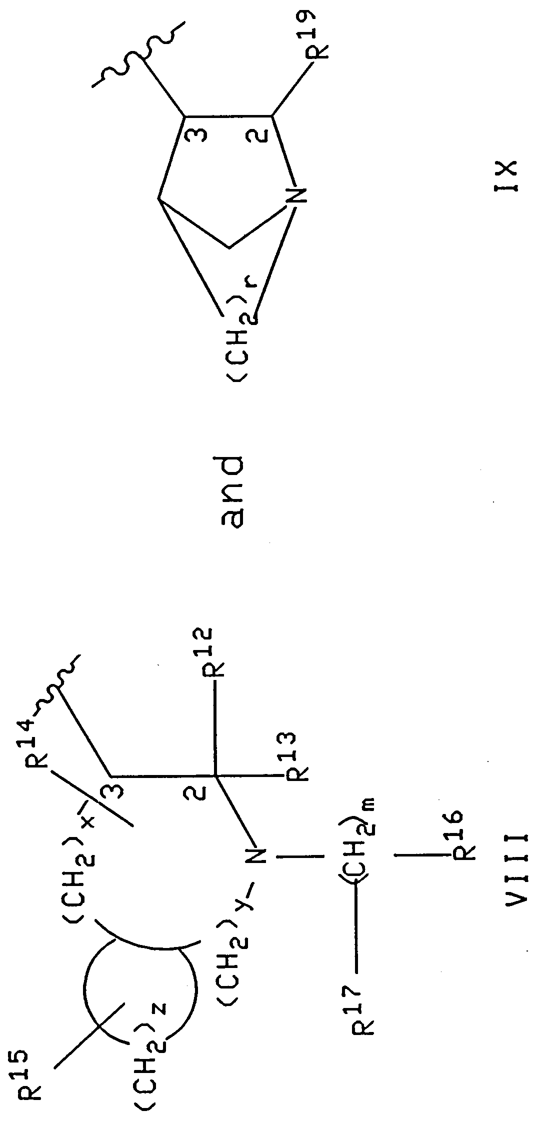 wherein R⁶ and R¹⁰ are independently selected from furyl, thienyl, pyridyl, indolyl, biphenyl and phenyl, wherein said phenyl may optionally be substituted with one or two substituents independently selected from halo, (C₁-C₁₀) alkyl optionally substituted with from one to three fluorine atoms, (C₁-C₁₀) alkoxy optionally substituted with from one to three fluorine atoms, carboxy, benzyloxycarbonyl and (C₁-C₃) alkoxy-carbonyl;
R⁷ is selected from (C₃-C₄) branched alkyl, (C₅-C₆) branched alkenyl, (C₅-C₇) cycloalkyl, and the radicals named in the definition of R⁶;
R⁸ is hydrogen or (C₁-C₆) alkyl;
R⁹ and R¹⁹ are independently selected from phenyl, biphenyl, naphthyl, pyridyl, benzhydryl, thienyl and furyl, and R⁹ and R¹⁹ may optionally be substituted with from one to three substituents independently selected from halo, (C₁-C₁₀) alkyl optionally substituted with from one to three fluorine atoms and (C₁-C₁₀) alkoxy optionally substituted with from one to three fluorine atoms;
Y¹ is (CH₂)ₗ wherein l is an integer from one to three, or Y¹ is a group of the formula 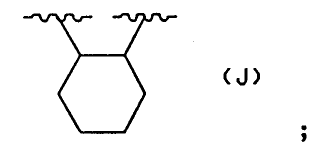 Z¹ is oxygen, sulfur, amino, (C₁-C₃)alkylamino or (CH₂)ₙ wherein n is zero, one or two;
x is an integer from zero to four;
y is an integer from zero to four;
z is an integer from one to six, wherein the ring containing (CH₂)_{z} may contain from zero to three double bonds, and one of the carbons of (CH₂)_{z} may optionally be replaced by oxygen, sulfur or nitrogen;
o is two or three;
p is zero or one;
r is one, two or three;
R¹¹ is thienyl, biphenyl or phenyl optionally substituted with one or two substituents independently selected from halo, (C₁-C₁₀) alkyl optionally substituted with from one to three fluorine atoms and (C₁-C₁₀) alkoxy optionally substituted with from one to three fluorine atoms;
X⁴ is (CH₂)_{q} wherein q is an integer from 1 to 6, and wherein any one of the carbon-carbon single bonds in said (CH₂)_{q} may optionally be replaced by a carbon-carbon double bond, and wherein any one of the carbon atoms of said (CH₂)_{q} may optionally be substituted with R¹⁴, and wherein any one of the carbon atoms of said (CH₂)_{q} may optionally be substituted with R¹⁵;
m is an integer from 0 to 8, and any one of the
carbon-carbon single bonds of (CH₂)ₘ, wherein both carbon atoms of such bond are bonded to each other and to another carbon atom in the (CH₂)ₘ chain, may optionally be replaced by a carbon-carbon double bond or a carbon-carbon triple bond, and any one of the carbon atoms of said (CH₂)ₘ may optionally be substituted with R¹⁷;
R¹² is a radical selected from hydrogen, (C₁-C₆) straight or branched alkyl, (C₃-C₇)cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl-(C₂-C₆)alkyl, benzhydryl and benzyl, wherein the point of attachment on R¹² is a carbon atom unless R¹² is hydrogen, and wherein each of said aryl and heteroaryl groups and the phenyl moieties of said benzyl, phenyl-(C₂-C₆)alkyl and benzhydryl may optionally be substituted with one or more substituents independently selected from halo, nitro, (C₁-C₁₀)alkyl optionally substituted with from one to three fluorine atoms, (C₁-C₁₀)alkoxy optionally substituted with from one to three fluorine atoms, amino, hydroxy-(C₁-C₆)alkyl, (C₁-C₆)alkoxy-(C₁-C₆)alkyl, (C₁-C₆)-alkylamino, 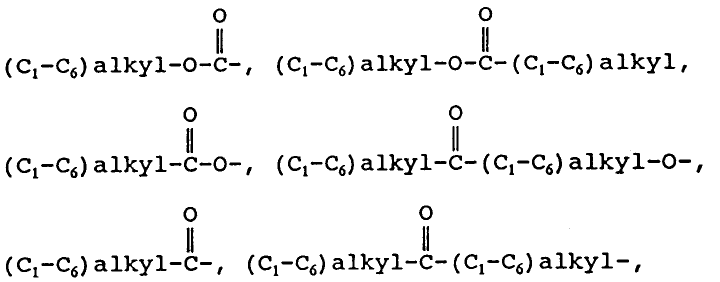 di-(C₁-C₆)alkylamino, 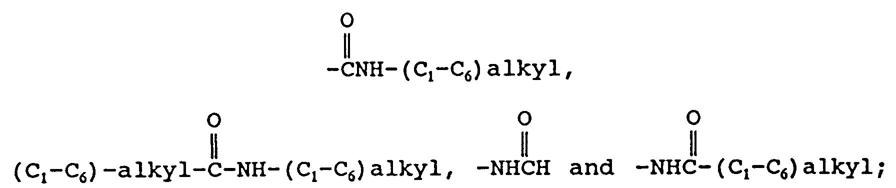 and wherein one of the phenyl moieties of said benzhydryl may optionally be replaced by naphthyl, thienyl, furyl or pyridyl;
R¹³ is hydrogen, phenyl or (C₁-C₆)alkyl;
or R¹² and R¹³, together with the carbon to which they are attached, form a saturated carbocyclic ring having from 3 to 7 carbon atoms wherein one of said carbon atoms that is neither the point of attachment of the spiro ring nor adjacent to it may optionally be replaced by oxygen, nitrogen or sulfur;
R¹⁴ and R¹⁵ are each independently selected from hydrogen, hydroxy, halo, amino, oxo (=O), cyano, hydroxy-(C₁-C₆)alkyl, (C₁-C₆)alkoxy-(C₁-C₆)alkyl, (C₁-C₆)alkylamino, di-(C₁-C₆)alkylamino, (C₁-C₆)alkoxy, 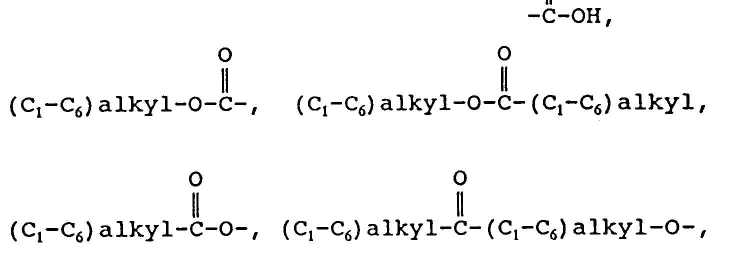 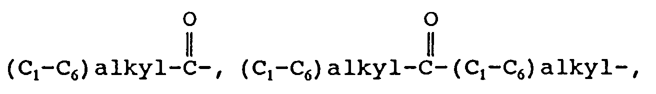 and the radicals set forth in the definition of R¹²;
R¹⁶ is 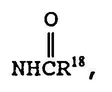 NHCH₂R¹⁸, SO₂R¹⁸, GR²⁰ CO₂H or one of the radicals set forth in any of the definitions of R¹², R¹⁴ and R¹⁵;
R¹⁷ is oximino (=NOH) or one of the radicals set forth in any of the definitions of R¹², R¹⁴ and R¹⁵; and
R¹⁸ is (C₁-C₆)alkyl, hydrogen, phenyl or phenyl (C₁-C₆)alkyl;
G is selected from the group consisting of CH₂, nitrogen, oxygen, sulfur and carbonyl;
R²⁰ is a monocyclic or bicyclic heterocycle selected from the group consisting of pyrimidinyl, benzoxazolyl, 2,3-dihydro-3-oxobenzisosulfonazol-2-yl, morpholin-1-yl, thiomorpholin-1-yl, benzofuranyl, benzothienyl, indolyl, isoindolyl, isoquinolinyl, furyl, pyridyl, isothiazolyl, oxazolyl, triazolyl, tetrazolyl, quinolyl, thiazolyl, thienyl, and groups of the formulae 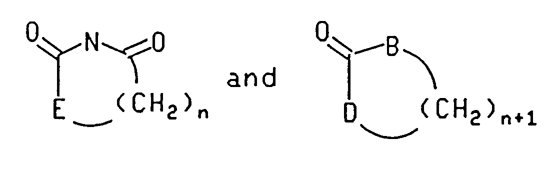 wherein B and D are selected from carbon, oxygen, and nitrogen, and at least one of B and D is other than carbon; E is carbon or nitrogen; n is an integer from 1 to 5; and any one of the carbons of the (CH₂)ₙ or (CH₂)ₙ₊₁ may be optionally substituted with (C₁-C₆)alkyl or (C₂-C₆) spiroalkyl, and either any two of the carbon atoms of said (CH₂)ₙ and (CH₂)ₙ₊₁ may be bridged by a one or two carbon atom linkage, or any one pair of adjacent carbons of said (CH₂)ₙ and (CH₂)ₙ₊₁ may form, together with from one to three carbon atoms that are not members of the carbonyl containing ring, a (C₃-C₅) fused carbocyclic ring;
with the proviso that (a) when m is 0, one of R¹⁶ and R¹⁷ is absent and the other is hydrogen, (b) when R³ is a group of the formula VIII, R¹⁴ and R¹⁵ cannot be attached to the same carbon atom, (c) when R¹⁴ and R¹⁵ are attached to the same carbon atom, then either each of R¹⁴ and R¹⁵ is independently selected from hydrogen, fluoro, (C₁-C₆)alkyl, hydroxy-(C₁-C₆)alkyl and (C₁-C₆)alkoxy-(C₁-C₆)alkyl, or R¹⁴ and R¹⁵, together with the carbon to which they are attached, form a (C₃-C₆) saturated carbocyclic ring that forms a Spiro compound with the nitrogen-containing ring to which they are attached; (d) R¹² and R¹³ cannot both be hydrogen; (e) when R¹⁴ or R¹⁵ is attached to a carbon atom of X⁴ or (CH₂)_{y} that is adjacent to the ring nitrogen, then R¹⁴ or R¹⁵, respectively, must be a substituent wherein the point of attachment is a carbon atom; (f) neither R¹⁴, R¹⁵, R¹⁶ nor R¹⁷ can form a ring with R¹³; and (g) the compound of formula Ia cannot be (2S,3S)-N-(2-methoxyphenyl)methyl-2-diphenylmethyl-1-azabicyclo[2.2.2]octan-3-amine;
or a pharmaceutically acceptable salt thereof, or
(b) of a compound having the formula 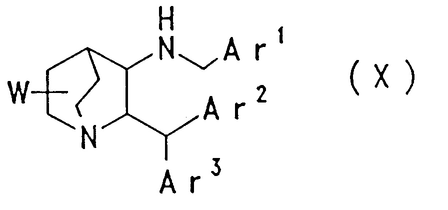 wherein W is Y or X(CH₂)ₙ;
Y is optionally substituted (C₁-C₆)alkyl, optionally substituted (C₂-C₆)alkenyl or optionally substituted (C₃-C₈)cycloalkyl;
X is optionally substituted (C₁-C₆)alkoxy, hydroxy, CONR¹R², CO₂R¹, CHR¹OR², CHR¹NR²R³, COR¹, CONR¹OR² or optionally substituted aryl, wherein said aryl is selected from phenyl, naphthyl, pyridyl, quinolyl, thienyl, furyl, phenoxyphenyl, oxazolyl, tetrazolyl, thiazolyl, imidazolyl and pyrazolyl; and n is an integer from zero to six;
Ar¹, Ar² and Ar³ are each, independently, optionally substituted aryl, wherein said aryl is selected from phenyl, naphthyl, pyridyl, quinolyl, thienyl, furyl, phenoxyphenyl, oxazolyl, tetrazolyl, thiazolyl, imidazolyl and pyrazolyl;
and R¹, R² and R³ are independently selected from hydrogen, optionally substituted (C₁-C₆)alkyl, optionally substituted (C₁-C₆) alkoxy, optionally substituted (C₃-C₈)cycloalkyl, optionally substituted aryl, wherein said aryl is selected from phenyl, naphthyl, pyridyl, quinolyl, thienyl, furyl, phenoxyphenyl, oxazolyl, tetrazolyl, thiazolyl, imidazolyl and pyrazolyl; and optionally substituted (C₁-C₅)heterocyclic groups, wherein said heterocyclic groups are selected from pyrrolidino, piperidino, morpholino, piperazinyl and thiamorpholino;
and wherein the substituents on the foregoing substituted alkyl, alkenyl, cycloalkyl and alkoxy groups are independently selected from halo, nitro, amino, (C₁-C₄)alkyl, (C₁-C₄)alkoxy, trifluoromethyl and trifluoromethoxy;
and wherein the substituents on the foregoing substituted (C₁-C₅) heterocyclic groups are attached to a sulfur or nitrogen atom on the ring and are independently selected from oxygen, di-oxygen and (C₁-C₄)alkyl when attached to a ring sulfur atom and are independently selected from oxygen and (C₁-C₅)alkyl when attached to a ring nitrogen atom;
and wherein the substituents on said substituted Ar¹ groups are independently selected from (C₁-C₆)alkyl optionally substituted with from one to three halo groups, (C₁-C₆)alkoxy optionally substituted with from one to three halo groups, (C₁-C₆)alkylsulfinyl, (C₂-C₆)alkenyl, (C₁-C₆)alkylthio, (C₁-C₆)alkylsulfonyl, (C₁-C₆)alkylsulfonylamino, and di-(C₁-C₆)alkylamino wherein one or both of the alkyl groups may be optionally substituted with a (C₁-C₆)alkylsulfonyl, or (C₁-C₆)alkylsulfinyl group;
and wherein the substituents on said substituted Ar² and Ar³ groups are independently selected from (C₁-C₄)alkyl, (C₁-C₄)alkoxy, (C₁-C₄)alkylthio, (C₁-C₄)alkylsulfinyl, di-(C₁-C₄)alkylamino, trifluoromethyl and trifluoromethoxy; with the proviso that when Y is unsubstituted or is substituted with (C₁-C₄)alkyl, it is attached to the 4- or 6-position of the quinuclidine ring;
or a pharmaceutically acceptable salt thereof, or
(c) of a compound having the formula 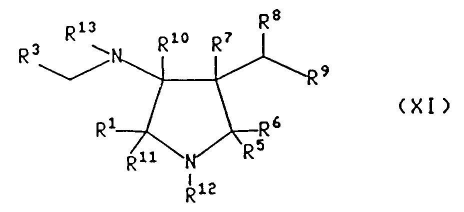 wherein R¹ is selected from hydrogen, (C₁-C₆) straight or branched alkyl, (C₃-C₇) cycloalkyl wherein one of the carbon atoms may optionally be replaced by nitrogen, oxygen or sulfur; aryl selected from phenyl, biphenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents independently selected from halo, nitro, (C₁-C₆) alkyl optionally substituted with from one to three fluorine atoms, (C₁-C₆) alkoxy, amino, trihaloalkoxy (e.g., trifluoromethoxy),
(C₁-C₆)alkylamino, 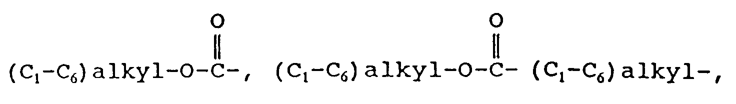 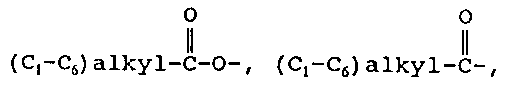 (C₁-C₆)alkyl-O-, 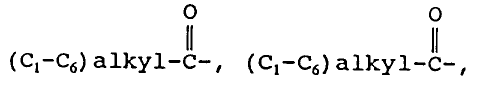 (C₁-C₆)alkyl-, di-(C₁-C₆)alkylamino, 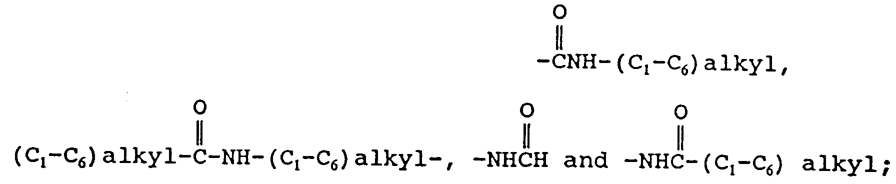 and wherein one of the phenyl moieties of said benzhydryl may optionally be replaced by naphthyl, thienyl, furyl or pyridyl;
R³ is aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; and cycloalkyl having 3 to 7 carbon atoms wherein one of said carbon atoms may optionally be replaced by nitrogen, oxygen or sulfur; wherein each of said aryl and heteroaryl groups may optionally be substituted with one or more substituents, and said (C₃-C₇) cycloalkyl may optionally be substituted with one or two substituents, each of said substituents being independently selected from halo, nitro, (C₁-C₆) alkyl optionally substituted with from one to three fluorine atoms, (C₁-C₆) alkoxy substituted with from one to three fluorine atoms, amino, phenyl, trihaloalkoxy, (C₁-C₆) alkylamino, 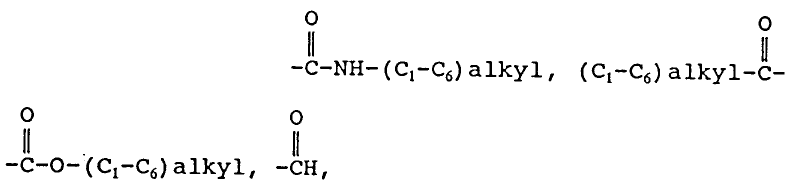 -CH₂OR¹³, NH(C₁-C₆)alkyl-, 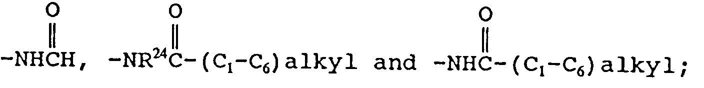 one of R⁵ and R⁶ is hydrogen and the other is selected from hydroxymethyl, hydrogen, (C₁-C₃)alkyl, (C₁-C₈)acyloxy-(C₁-C₃)alkyl, (C₁-C₈)alkoxymethyl and benzyloxymethyl;
R⁷ and R⁸ are independently selected from hydrogen, (C₁-C₃)alkyl and phenyl;
R⁹ is selected from methyl, hydroxymethyl, 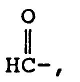 R¹⁴R¹⁵NCO₂CH₂-, R¹⁶OCO₂CH₂-, (C₁-C₄)alkyl-CO₂CH₂-, -CONR¹⁷R¹⁸, R¹⁷R¹⁸NCO₂-, R¹⁹OCO₂-, C₆H₅CH₂CO₂CH₂-, C₆H₅CO₂CH₂-, (C₁-C₄)alkyl-CH(OH)-, C₆H₅CH(OH)-, C₆H₅CH₂CH(OH)-, CH₂halo, R²⁰SO₂OCH₂, -CO₂R¹⁶ and R²¹CO₂-;
R¹⁰ and R¹¹ are independently selected from hydrogen, (C₁-C₃) alkyl and phenyl;
R¹² is hydrogen, benzyl or a group of the formula 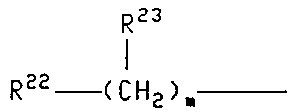 wherein m is an integer from zero to twelve, and any one of the carbon-carbon single bonds of (CH₂)ₘ, wherein both carbon atoms of such bond are bonded to each other and to another carbon atom of the (CH₂)ₘ chain, may optionally be replaced by a carbon-carbon double or triple bond, and any one of the carbon atoms of (CH₂)ₘ may optionally be substituted with R²³;
R¹³, R¹⁴, R¹⁵, R¹⁶, R¹⁷, R¹⁸, R¹⁹, R²⁰, R²¹ and R²⁴ are independently selected from hydrogen, (C₁-C₃)alkyl and phenyl;
R²² and R²³ are independently selected from hydrogen, hydroxy, halo, amino, carboxy, carboxy(C₁-C₆) alkyl, (C₁-C₆)alkylamino, di-(C₁-C₆)alkylamino, (C₁-C₆)alkoxy, 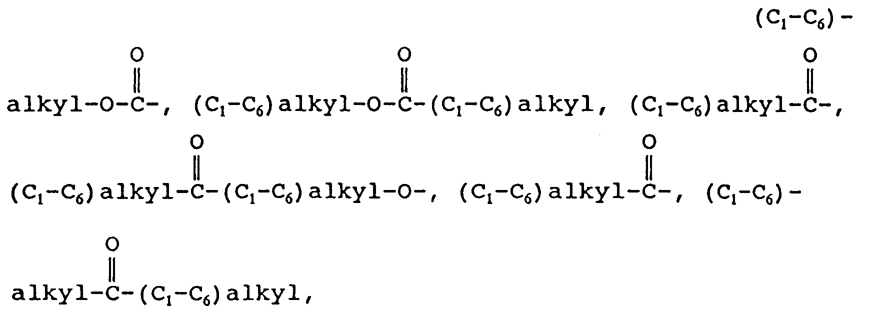 (C₁-C₆) straight or branched alkyl, (C₃-C₇) cycloalkyl wherein one of the carbon atoms may optionally be replaced by nitrogen, oxygen or sulfur; aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; phenyl-(C₂-C₆)alkyl, benzhydryl and benzyl, wherein each of said aryl and heteroaryl groups and the phenyl moieties of said benzyl, phenyl-(C₂-C₆)alkyl and benzhydryl may optionally be substituted with one or two substituents independently selected from halo, nitro, (C₁-C₆)alkyl optionally substituted with from one to three fluorine atoms, (C₁-C₆)alkoxy optionally substituted with from one to three fluorine atoms,
trifluoromethyl, amino, (C₁-C₆)-alkylamino, 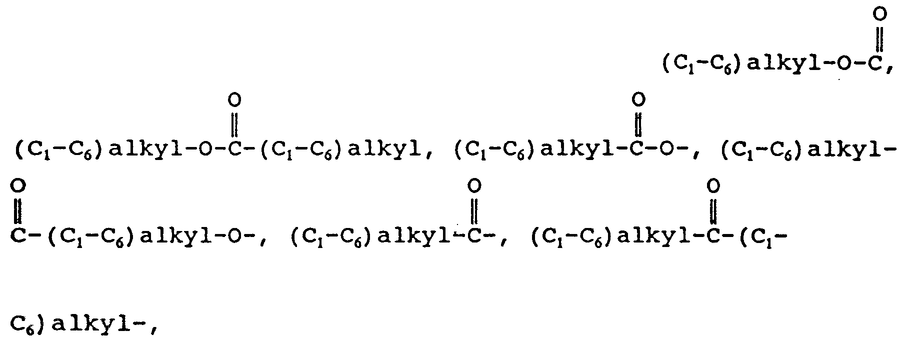 di-(C₁-C₆)alkylamino, 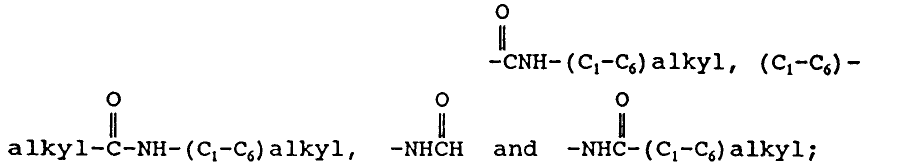 and wherein one of the phenyl moieties of said benzhydryl may optionally be replaced by naphthyl, thienyl, furyl or pyridyl;
or R⁹, together with the carbon to which it is attached, the nitrogen of the pyrrolidine ring, the carbon to which R⁷ is attached and the carbon to which R⁵ and R⁶ are attached form a second pyrrolidine ring; with the proviso that when R⁹, together with the carbon to which it is attached, the nitrogen of the pyrrolidine ring, the carbon to which R⁷ is attached and the carbon to which R⁵ and R⁶ are attached, form a second pyrrolidine ring (thus forming a bicyclic structure containing a bridgehead nitrogen), either R¹² is absent or R¹² is present and the nitrogen of the second pyrrolidine ring is positively charged;
or a pharmaceutically acceptable salt thereof, or
(d) of a compound of the formula 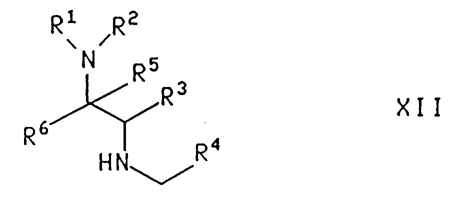 wherein R¹ is hydrogen, (C₁-C₈) alkyl, a saturated (C₆-C₁₀) carbocyclic ring system containing two fused rings, a saturated (C₆-C₁₀) carbocyclic bridged ring system containing two rings, or benzyl wherein the phenyl moiety of said benzyl may optionally be substituted with one or more substituents independently selected from halo, (C₁-C₆) alkyl optionally substituted with from one to three fluorine atoms and (C₁-C₈) alkoxy optionally substituted with from one to three fluorine atoms;
R² is hydrogen, benzyl or a group of the formula 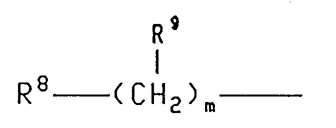 wherein m is an integer from zero to twelve, and any one of the carbon-carbon single bonds of (CH₂)ₘ, wherein both carbon atoms of such bond are bonded to each other and to another carbon atom of the (CH₂)ₘ chain, may optionally be replaced by a carbon-carbon double or triple bond, and any one of the carbon atoms of (CH₂)ₘ may optionally be substituted with R⁹;
R⁸ and R⁹ are independently selected from hydrogen, hydroxy, halo, amino, carboxy, carboxy(C₁-C₆)alkyl, (C₁-C₆)alkylamino, di-(C₁-C₆)alkylamino, (C₁-C₆)alkoxy, 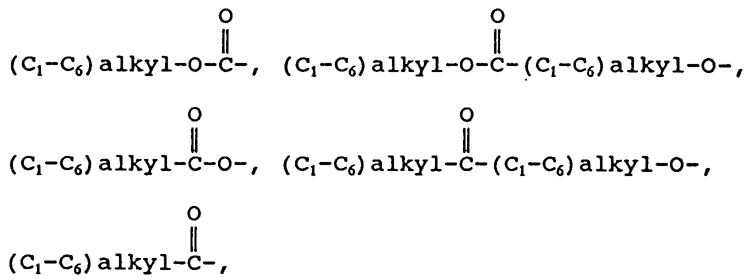 (C₁-C₆) straight or branched alkyl, (C₃-C₇) cycloalkyl wherein one of the carbon atoms may optionally be replaced by nitrogen, oxygen or sulfur; aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; phenyl-(C₂-C₆)alkyl, benzhydryl and benzyl, wherein each of said aryl and heteroaryl groups and the phenyl moieties of said benzyl, phenyl-(C₂-C₆)alkyl and benzhydryl may optionally be substituted with one or two substituents independently selected from halo, nitro, (C₁-C₆)alkyl optionally substituted with from one to three fluorine atoms, (C₁-C₆)alkoxy optionally substituted with from one to three fluorine atoms,
trifluoromethyl, amino, (C₁-C₆)-alkylamino, 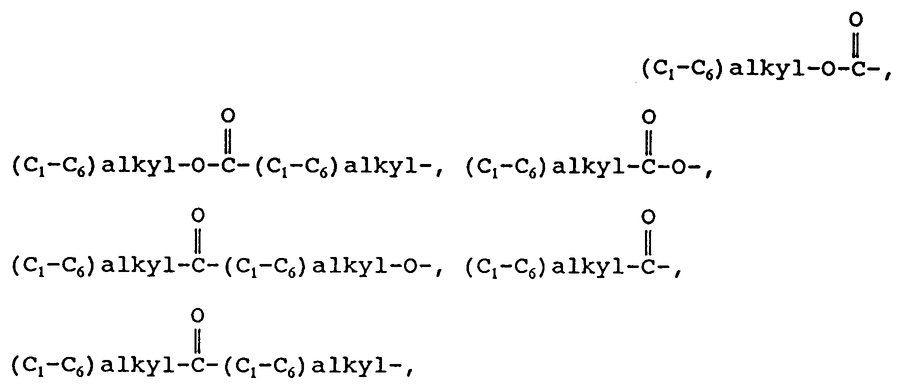 di-(C₁-C₆)alkylamino, 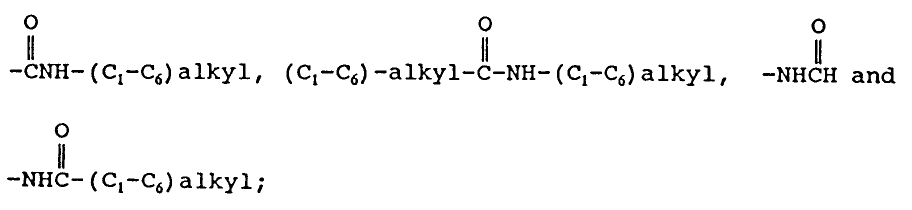 and wherein one of the phenyl moieties of said benzhydryl may optionally be replaced by naphthyl, thienyl, furyl or pyridyl;
or R¹ and R², together with the nitrogen to which they are attached, form a saturated or unsaturated monocyclic ring containing from three to eight carbon atoms, a fused bicyclic ring containing from six to ten carbon atoms, or a saturated bridged ring system containing from six to ten carbon atoms;
R⁴ is aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; and cycloalkyl having from three to seven carbon atoms wherein one of said carbon atoms may optionally be replaced by nitrogen, oxygen or sulfur; wherein each of said aryl and heteroaryl groups may optionally be substituted with one or more substituents, and said (C₃-C₇) cycloalkyl may optionally be substituted with one, two or three substituents, each of said substituents being independently selected from halo, nitro, (C₁-C₆) alkyl optionally substituted with from one to three fluorine atoms, (C₁-C₆) alkoxy optionally substituted with from one to three fluorine atoms, phenyl,
amino, (C₁-C₆) alkylamino, 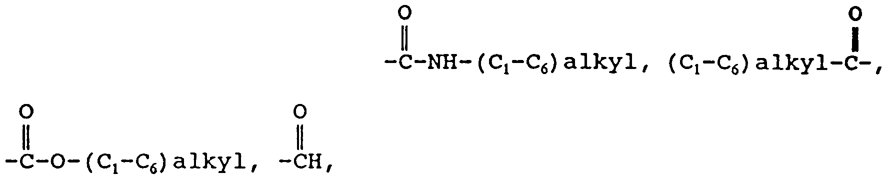 -CH₂OR¹², NH₂(C₁-C₆)alkyl-, 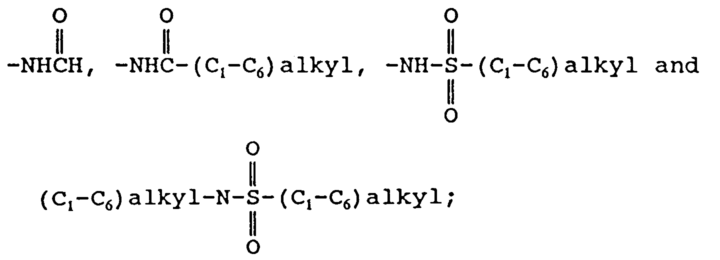 R³ is hydrogen, (C₃-C₈)cycloalkyl, (C₁-C₆) straight or branched alkyl or phenyl optionally substituted with one or more substituents independently selected from halo, (C₁-C₆)alkyl optionally substituted with from one to three fluorine atoms, and (C₁-C₆)alkoxy optionally substituted with from one to three fluorine atoms;
R⁵ is hydrogen, (C₁-C₆)alkyl, or phenyl optionally substituted with one or more substituents independently selected from halo, (C₁-C₆) alkyl optionally substituted with from one to three fluorine atoms and (C₁-C₆)alkoxy optionally substituted with from one to three fluorine atoms;
R⁶ is selected from hydrogen, (C₁-C₆) straight or branched alkyl, (C₃-C₇) cycloalkyl wherein one of the carbon atoms may optionally be replaced by nitrogen, oxygen or sulfur; aryl selected from phenyl, biphenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents independently selected from halo, nitro, (C₁-C₆) alkyl optionally substituted with from one to three fluorine atoms, (C₁-C₆) alkoxy, trifluoromethyl, amino, trihaloalkoxy (e.g.,trifluoromethoxy), (C₁-C₆)alkylamino, 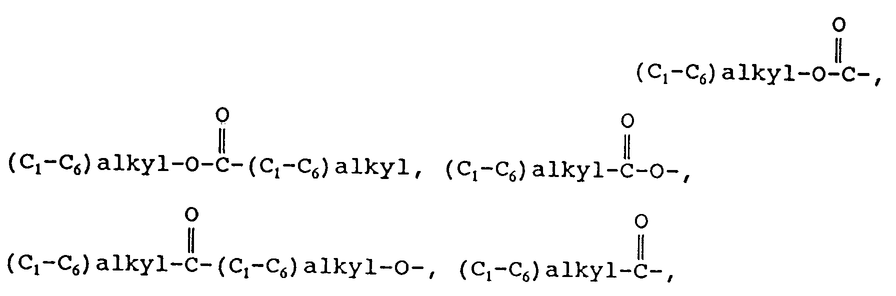 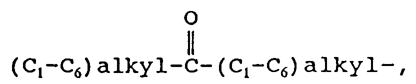 di-(C₁-C₆)alkylamino, 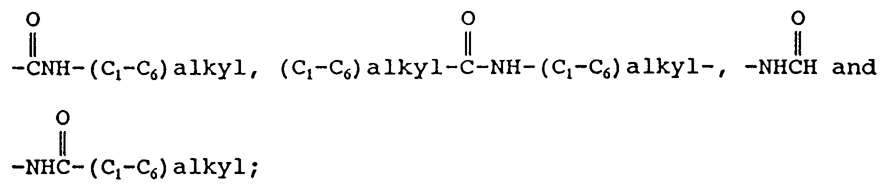 and wherein one of the phenyl moieties of said benzhydryl may optionally be replaced by naphthyl, thienyl, furyl or pyridyl; and
R¹² is hydrogen, (C₁-C₃)alkyl or phenyl;
or a pharmaceutically acceptable salt thereof, or
(e) of a compound of the formula 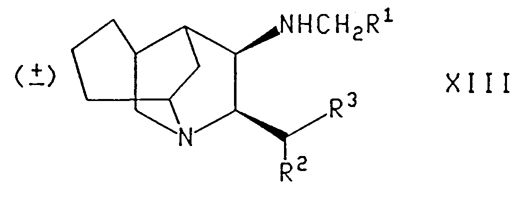 wherein R¹ is cycloalkyl having from five to seven carbon atoms, pyrrolyl, thienyl, pyridyl, phenyl or substituted phenyl, wherein said substituted phenyl is substituted with from one to three substituents independently selected from fluorine, chlorine, bromine, trifluoromethyl, alkyl having from one to three carbon atoms, alkoxy having from one to three carbon atoms, carboxy, alkoxycarbonyl having from one to three carbon atoms in the alkoxy moiety and benzyloxycarbonyl;
R² is furyl, thienyl, pyridyl, indolyl, biphenyl, phenyl or substituted phenyl, wherein said substituted phenyl is substituted with one or two substituents independently selected from fluorine, chlorine, bromine, trifluoromethyl, alkyl having from one to three carbon atoms, alkoxy having from one to three carbon atoms, carboxy, alkoxycarbonyl having from one to three carbon atoms in the alkoxy moiety and benzyloxycarbonyl; and
R³ is thienyl, phenyl, fluorophenyl, chlorophenyl or bromophenyl;
or a pharmaceutically acceptable salt thereof, or
(f) of a compound of the formula 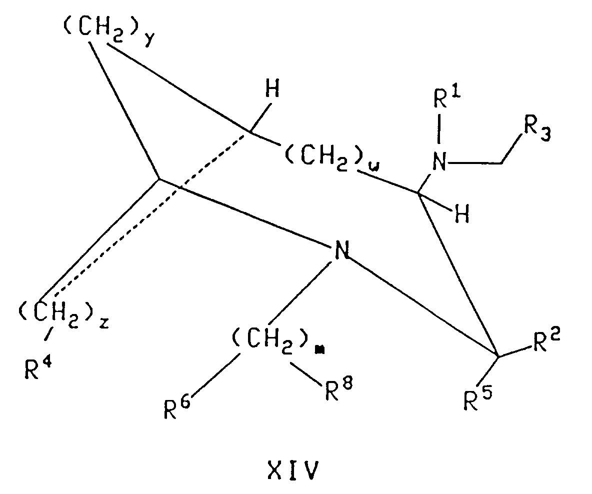 wherein m is an integer from 0 to 8, and any one of the carbon-carbon single bonds of (CH₂)ₘ, wherein both carbon atoms of such bond are bonded to each other and to another carbon in the (CH₂)ₘ chain, may optionally be replaced by a carbon-carbon double bond or a carbon-carbon triple bond, and any one of the carbon atoms of said (CH₂)ₘ may optionally be substituted with R⁸;
w is an integer from 0 to 2;
y is an integer from 1 to 4;
z is an integer from 1 to 4, and wherein any one of the carbon atoms of said (CH₂)_{z} may optionally be substituted with R⁴;
R¹ is hydrogen or (C₁-C₈) alkyl optionally substituted with hydroxy, alkoxy or fluoro;
R² is a group selected from hydrogen, (C₁-C₆)straight or branched alkyl, (C₃-C₇)cycloalkyl wherein one of the carbon atoms may optionally be replaced by nitrogen, oxygen or sulfur; aryl selected from phenyl, indanyl, and naphthyl; heteroaryl selected from thienyl, furyl, pyridyl, thiazolyl, isothiazolyl, oxazolyl, isoxazolyl, triazolyl, tetrazolyl and quinolyl; phenyl(C₂-C₆)alkyl, benzhydryl and benzyl, wherein one of the phenyl moieties of said benzhydryl may optionally be replaced by naphthyl, thienyl, furyl or pyridyl and wherein each of said aryl and heteroaryl groups and the phenyl moieties of said benzyl, phenyl(C₂-C₆)alkyl and benzhydryl may optionally be substituted with one or more substituents independently selected from halo, nitro, (C₁-C₆)alkyl, (C₁-C₆)alkoxy, trifluoromethyl, amino, (C₁-C₆)-alkylamino, 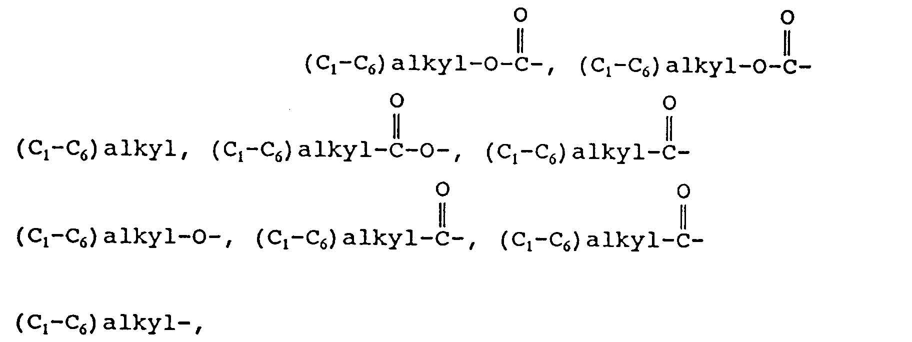 di-(C₁-C₆)alkylamino, 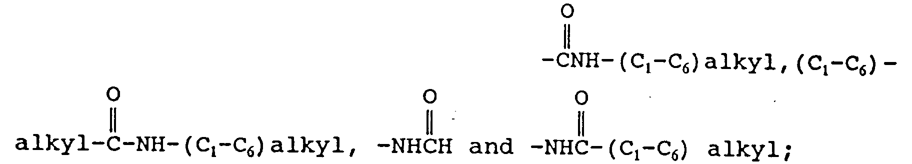 R⁵ is hydrogen, phenyl or (C₁-C₆)alkyl;
or R² and R⁵, together with the carbon to which they are attached, form a saturated carbocyclic ring having from 3 to 7 carbon atoms wherein one of said carbon atoms may optionally be replaced by oxygen, nitrogen or sulfur;
R³ is aryl selected from phenyl, indanyl, and naphthyl; heteroaryl selected from thienyl, furyl, pyridyl, thiazolyl, isothiazolyl, oxazolyl, isoxazolyl, triazolyl, tetrazolyl and quinolyl; and cycloalkyl having 3 to 7 carbon atoms wherein one of said carbon atoms may optionally be replaced by nitrogen, oxygen or sulfur; wherein each of said aryl and heteroaryl groups may optionally be substituted with one or more substituents, and said (C₃-C₇) cycloalkyl may optionally be substituted with one or two substituents, each of said substituents being independently selected from halo, nitro, (C₁-C₆)alkyl optionally substituted with from one to three fluorine atoms, (C₁-C₆)alkoxy optionally substituted with from one to three fluorine atoms, trifluoromethyl, phenyl, amino, (C₁-C₆)alkylamino, (C₁-C₆)dialkyl amino, 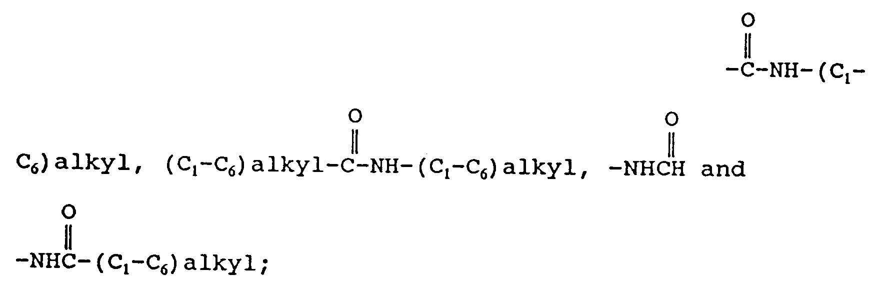 R⁴ is independently selected from hydrogen, hydroxy, halo, amino, oxo (=O), nitrile,
(C₁-C₆)alkylamino, di-(C₁-C₆)alkylamino, (C₁-C₆)alkoxy, 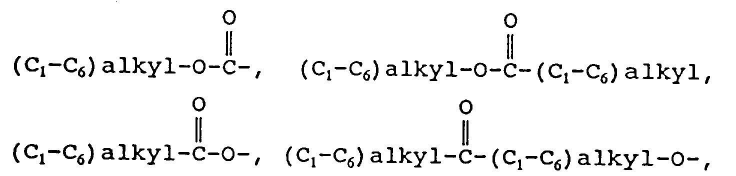 hydroxy-(C₁-C₆)alkyl, (C₁-C₆)alkoxy(C₁-C₆)alkyl, 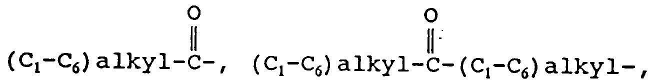 and the groups set forth in the definition of R²;
R⁶ is 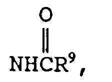 NHCH₂R⁹, NHSO₂R⁹ or one of the groups set forth in any of the definitions of R², and R⁴;
R⁸ is oximino (=NOH) or one of the groups set forth in any of the definitions of R², and R⁴;
R⁹ is (C₁-C₆) alkyl, hydrogen, phenyl or phenyl (C₁-C₆)alkyl;
with the proviso that (a) when m is 0, R⁸ is absent and R⁶ is hydrogen, (b) neither R⁴, R⁶, nor R⁸ can form, together with the carbon to which it is attached, a ring with R⁵, and (c) the sum of y and z must be less than 7;
or a pharmaceutically acceptable salt thereof, or
(g) of a compound of the formula 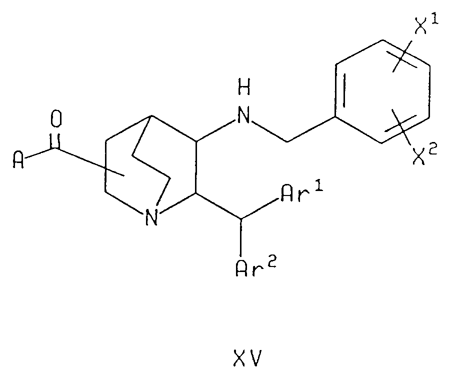 wherein X¹ is (C₁-C₅) alkoxy or halosubstituted (C₁-C₅)alkoxy;
X² is hydrogen, halogen, (C₁-C₅)alkyl, (C₂-C₅)alkenyl, (C₂-C₅)alkynyl, (C₁-C₅)alkoxy, (C₁-C₅)alkylthio, (C₁-C₅)alkylsulfinyl, (C₁-C₅)alkylsulfonyl, halosubstituted (C₁-C₅)alkyl, halosubstituted (C₁-C₅)alkoxy, (C₁-C₅)alkylamino, dialkylamino having from 1 to 5 carbon atoms in each alkyl moiety, (C₁-C₅)alkylsulfonylamino (which may be substituted by halogen), 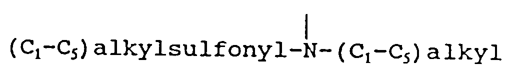 (which may be substituted by halogen in the alkylsulfonyl moiety), (C₁-C₅)alkanoylamino (which may be substituted by halogen) or 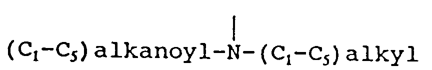 (which may be substituted by halogen in the alkanoyl moiety);
Ar¹ and Ar₂ are each, independently, thienyl, phenyl, fluorophenyl, chlorophenyl or bromophenyl;
A is Y-(CH₂)ₘ-CH(R²)-(CH₂)ₙ-NR¹-;
R¹ is hydrogen, (C₁-C₅)alkyl, benzyl or -(CH₂)ₚ-Y;
R² is hydrogen, (C₁-C₅)alkyl (which may be substituted by a substituent selected from the group consisting of hydroxy, amino, methylthio and mercapto), benzyl, 4-hydroxybenzyl, 3-indolylmethyl or -(CH₂)ₚ-Y;
Y is -CN, -CH₂Z or -COZ;
Z is hydroxy, amino, (C₁-C₅)alkoxy, (C₁-C₅)alkylamino or dialkylamino having from 1 to 5 carbon atoms in each alkyl moiety;
m, n and p are each, independently, 0, 1, 2 or 3; and
R¹ and R² may be connected to form a ring;
or a pharmaceutically acceptable salt thereof, or
(h) of a compound of the formula 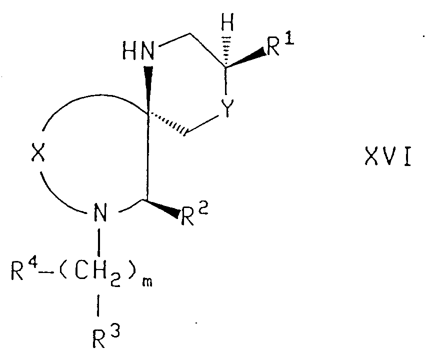 wherein R¹ is phenyl optionally substituted with one or more substituents, preferably with from one to three substituents, independently selected from hydrogen, halo, nitro, (C₁-C₁₀) alkyl optionally substituted with from one to three fluorine atoms, (C₁-C₁₀) alkoxy optionally substituted with from one to three fluorine atoms, trifluoromethyl, hydroxy, phenyl, cyano, amino, (C₁-C₆)-alkylamino, di-(C₁-C₆)alkylamino, 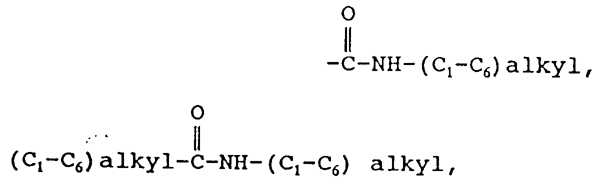 hydroxy(C₁-C₄)alkyl, 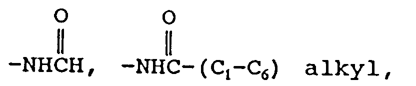 (C₁-C₄)alkoxy(C₁-C₄)alkyl, -S(O)ᵥ-(C₁-C₁₀)-alkyl wherein v is zero, one or two, -S(O)ᵥ-aryl wherein v is zero, one or two, -O-aryl, -SO₂NR⁴R⁵ wherein each of R⁴ and R⁵ is, independently, (C₁-C₆)alkyl, or R⁴ and R⁵, together with the nitrogen to which they are attached, form a saturated ring containing one nitrogen and from 3 to 6 carbons, 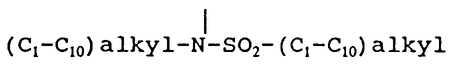 wherein one or both of the alkyl moieties may optionally be substituted with from one to three fluorine atoms, -N(SO₂-(C₁-C₁₀)alkyl)₂ and 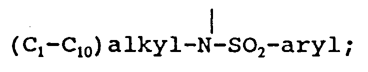 and wherein the aryl moieties of said -S(O)ᵥ-aryl, -O-aryl and 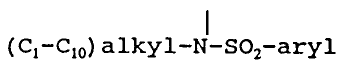 are independently selected from phenyl and benzyl and may optionally be substituted with from one to three substituents independently selected from (C₁-C₄)alkyl, (C₁-C₄)alkoxy and halo;
or R¹ is phenyl substituted with a group having the formula 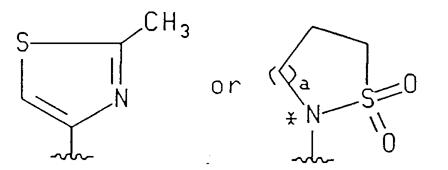 wherein a is 0, 1 or 2 and the asterisk represents a position meta to the point of attachment of R¹;
R² is selected from (C₁-C₆) straight or branched alkyl, (C₃-C₇) cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents, preferably with from one to three substituents, independently selected from halo, nitro, (C₁-C₁₀) alkyl optionally substituted with from one to three fluorine atoms, (C₁-C₁₀) alkoxy optionally substituted with from one to three fluorine atoms, amino, hydroxy-(C₁-C₆)alkyl, (C₁-C₆)alkoxy-(C₁-C₆)alkyl, (C₁-C₆)-alkylamino, 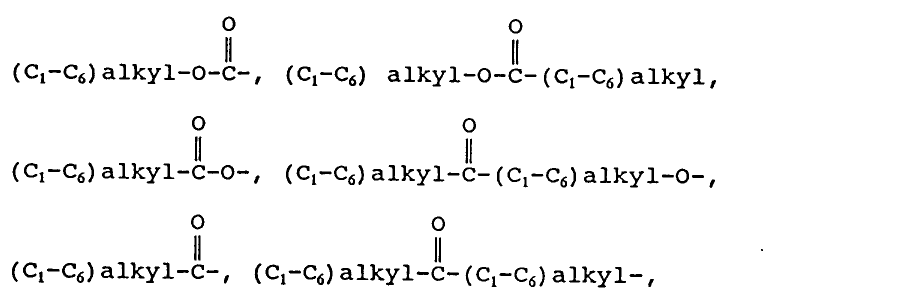 di-(C₁-C₆)alkylamino, 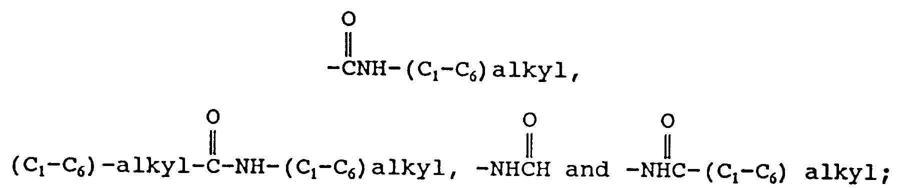 and wherein one of the phenyl moieties of said benzhydryl may optionally be replaced by naphthyl, thienyl, furyl or pyridyl;
m is an integer from 0 to 8, and any one of the carbon-carbon single bonds of (CH₂)ₘ, wherein both carbon atoms of such bond are bonded to each other and to another carbon atom in the (CH₂)ₘ chain, may optionally be replaced by a carbon-carbon double bond or a carbon-carbon triple bond, and any one of the carbon atoms of said (CH₂)ₘ may optionally be substituted with R⁴;
R³ is selected from 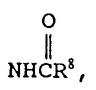 NHCH₂R⁸, SO₂R⁸, AR⁹, CO₂H and the radicals set forth in the definitions of R², R⁶ and R⁷;
A is CH₂, nitrogen, oxygen, sulfur or carbonyl;
R⁸ is (C₁-C₆) alkyl, hydrogen, phenyl or phenyl (C₁-C₆)alkyl;
R⁴ is selected from oximino (=NOH) and the radicals set forth in the definitions of R², R⁶ and R⁷;
R⁹ is a monocyclic or bicyclic heterocycle selected from the group consisting of pyrimidinyl, benzoxazolyl, 2,3-dihydro-3-oxobenzisosulfonazol-2-yl, morpholin-1-yl, thiomorpholin-1-yl, benzofuranyl, benzothienyl, indolyl, isoindolyl, isoquinolinyl, furyl, pyridyl, isothiazolyl, oxazolyl, triazolyl, tetrazolyl, quinolyl, thiazolyl, thienyl, and groups of the formulae 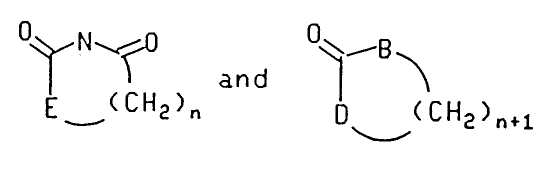 wherein B and D are selected from carbon, oxygen and nitrogen, and at least one of B and D is other than carbon; E is carbon or nitrogen; n is an integer from 1 to 5; any one of the carbon atoms of said (CH₂)ₙ and (CH₂)ₙ₊₁ may be optionally substituted with (C₁-C₆)alkyl or (C₂-C₆) spiroalkyl; and either any one pair of the carbon atoms of said (CH₂)ₙ and (CH₂)ₙ₊₁ may be bridged by a one or two carbon atom linkage, or any one pair of adjacent carbon atoms of said (CH₂)ₙ and (CH₂)ₙ₊₁ may form, together with from one to three carbon atoms that are not members of the carbonyl containing ring, a (C₃-C₅) fused carbocyclic ring;
X is (CH₂)_{q} wherein q is two or three and wherein one of the carbon-carbon single bonds in said (CH₂)_{q} may optionally be replaced by a carbon-carbon double bond, and wherein any one of the carbon atoms of said (CH₂)_{q} may optionally be substituted with R⁶, and wherein any one of the carbon atoms of said (CH₂)_{q} may optionally be substituted with R⁷;
R⁶ and R⁷ are independently selected from hydrogen, hydroxy, halo, amino, oxo (=O), cyano, hydroxy-(C₁-C₆)alkyl, (C₁-C₆)alkoxy-(C₁-C₆)alkyl, (C₁-C₆)alkylamino, di-(C₁-C₆)alkylamino, (C₁-C₆)alkoxy, 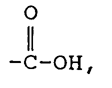 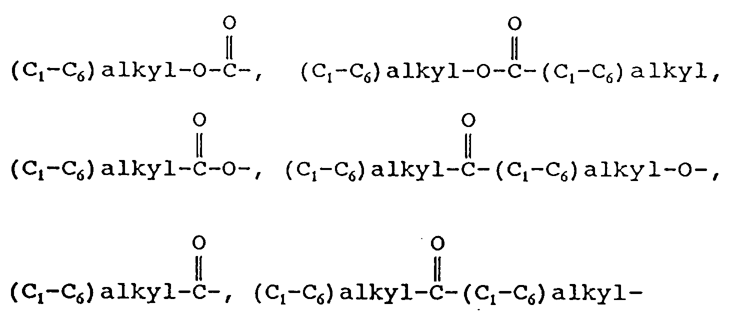 and the radicals set forth in the definition of R²; and
Y is (CH₂)_{z} wherein z is zero or one;
with the proviso that: (a) when A is -(CH₂)- or carbonyl, R⁹ cannot be furyl, pyridyl, isothiazolyl, oxazolyl, triazolyl, tetrazolyl, quinolyl, thiazolyl or thienyl; (b) when m is zero, one of R³ and R⁴ is absent and the other is hydrogen; and (c) when R⁶ or R⁷ is attached to a carbon atom of X that is adjacent to the ring nitrogen, then R⁶ or R⁷, respectively, must be a substituent wherein the point of attachment is a carbon atom;
or a pharmaceutically acceptable salt thereof, for the manufacture of a medicament for the treatment or prevention of sunburn in a mammal.

2. The use according to claim 1, wherein the compound is of the formula 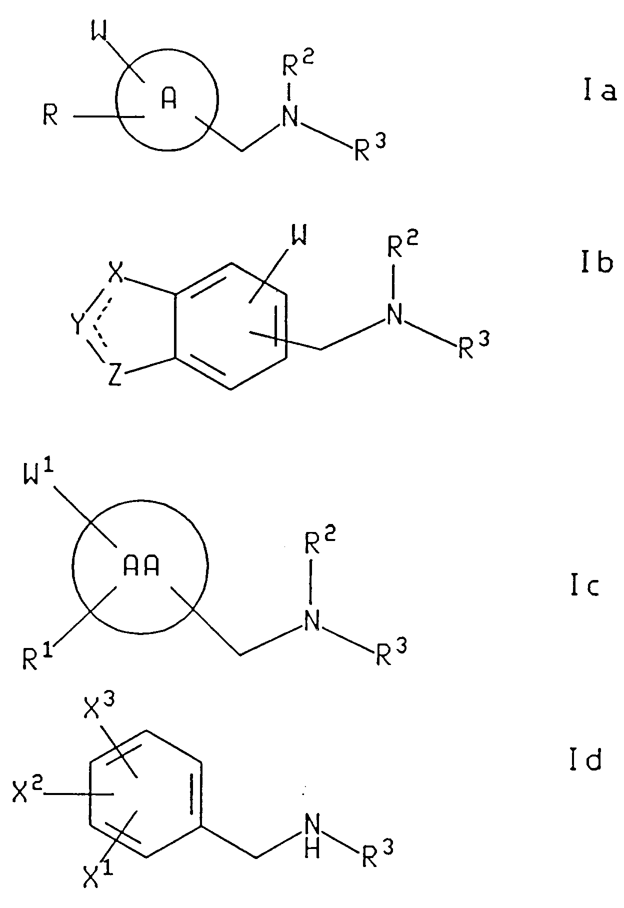 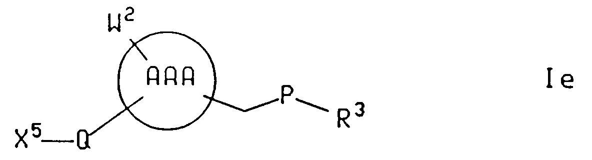 wherein A is a ring system selected from phenyl, naphthyl, thienyl, quinolinyl and indolinyl, and wherein the sidechain containing NR²R³ is attached to a carbon atom of ring system A;
AA is an aryl group selected from phenyl, naphthyl, thienyl, dihydroquinolinyl and indolinyl, and wherein the sidechain containing NR²R³ is attached to a carbon atom of AA;
AAA is an aryl group selected from phenyl, naphthyl, thienyl, dihydroquinolinyl and indolinyl, and wherein the - CH₂PR³ sidechain is attached to a carbon atom of ring A;
P is NR², O, S, SO or SO₂;
Q is SO₂, NH, 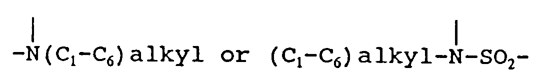 wherein the point of attachment of said 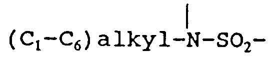 to ring A is the nitrogen atom and the point of attachment to R¹ is the sulfur atom;
W² is hydrogen, (C₁-C₆)alkyl, S-(C₁-C₃)alkyl, halo or (C₁-C₆)alkoxy optionally substituted with from one to three fluorine atoms;
W¹ is hydrogen, halo or (C₁-C₆) alkyl, S-(C₁-C₃)alkyl, halo or (C₁-C₆) alkoxy optionally substituted with from one to three fluorine atoms;
W is hydrogen, (C₁-C₆)alkyl optionally substituted with from One to three fluorine atoms, -S(O)ᵥ-(C₁-C₆) alkyl wherein v is zero, one or two, halo or (C₁-C₆)alkoxy optionally substituted with from one to three fluorine atoms;
R is a 4, 5 or 6 membered heterocyclic ring containing from one to three heteroatoms selected from oxygen, nitrogen and sulfur (e.g., thiazolyl, azetidinyl, pyrrolyl, pyrazolyl, 1,2,3-triazolyl, 1,2,4-triazolyl, isothiazolyl, imidazolyl, isoxazolyl, or oxazolyl) wherein said heterocyclic ring may contain from zero to three double bonds and may optionally be substituted with one or more substituents, preferably one or two substituents, independently selected from (C₁-C₆) alkyl optionally substituted with from one to three fluorine atoms and (C₁-C₆) alkoxy optionally substituted with from one to three fluorine atoms;
R¹ is selected from amino, (C₁-C₆) alkylamino, di-(C₁-C₆)alkylamino, -S(O)ᵥ-(C₁-C₁₀)-alkyl wherein v is zero, one or two, -S(O)ᵥ-aryl wherein v is zero, one or two, -O-aryl, -SO₂NR⁴R⁵ wherein each of R⁴ and R⁵ is, independently, (C₁-C₆)alkyl, or R⁴ and R⁵, together with the nitrogen to which they are attached, form a saturated ring containing one nitrogen and from 3 to 6
carbons, 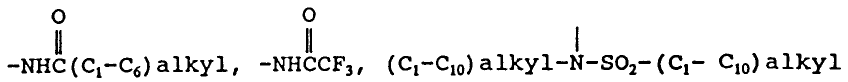 wherein one or both of the alkyl moieties may optionally be substituted with from one to three fluorine atoms, -N(SO₂-(C₁-C₁₀)alkyl)₂ and 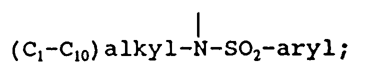 and wherein the aryl moieties of said -S(O)ᵥ-aryl, -O-aryl and 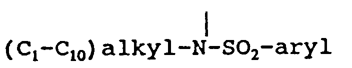 are independently selected from phenyl and benzyl and may optionally be substituted with from one to three substituents independently selected from (C₁-C₄)alkyl, (C₁-C₄)alkoxy and halo;
or R¹ is a group having the formula 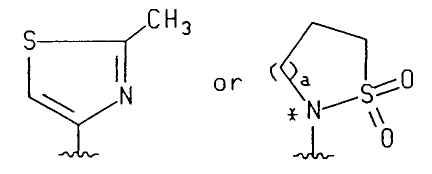 wherein a is 0, 1 or 2 and the asterisk represents a position meta to the R²R³NCH₂ side chain;
the dotted lines in formula Ib represent that one of the X-Y and Y-Z bonds may optionally be a double bond;
X is selected from =CH-, -CH₂-, -O-, -S-, -SO-, -SO₂-, -N(R⁴)-, -NH-, =N-, -CH[(C₁-C₆)alkyl]-, =C[(C₁-C₆)alkyl]-, -CH(C₆H₅)- and =C(C₆H₅)-;
X¹ is hydrogen, (C₁-C₁₀) alkoxy optionally substituted with from one to three fluorine atoms or (C₁-C₁₀)alkyl optionally subsituted with from one to three fluorine atoms;
X² and X³ are independently selected from hydrogen, halo, nitro, (C₁-C₁₀)alkyl optionally substituted with from one to three fluorine atoms, (C₁-C₁₀)alkoxy optionally substituted with from one to three fluorine atoms, trifluoromethyl, hydroxy, phenyl, cyano, amino, hydroxy (C₁-C₄)alkyl, (C₁-C₄)alkoxy, (C₁-C₄)alkyl, (C₁-C₆)alkylamino, di-(C₁-c₆)alkylamino, 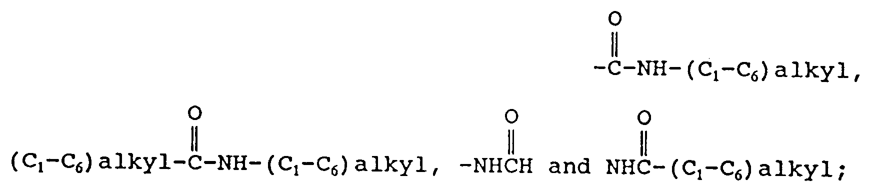 X⁵ is a four to six membered heterocyclic ring containing from one to three heteroatoms selected from sulfur, nitrogen and oxygen (e.g., thiazolyl, pyrrolyl, thienyl, triazolyl, oxazolyl, oxadiazolyl, thiadiazolyl or imidazolyl), wherein said heterocyclic ring may optionally be susbtituted with from one to three substituents, preferably with from zero to two substituents, independently selected from phenyl, (C₁-C₆)alkyl optionally substituted with from one to three fluorine atoms, (C₁-C₆)alkoxy optionally substituted with from one to three fluorine atoms and halo;
Y is selected from C=O, C=NR⁴, C=S, =CH-, -CH₂-, =C[(C₁-C₆)alkyl]-, -CH[(C₁-C₆)alkyl]-, =C(C₆H₅)-, -CH(C₆H₅)-, =N-, -NH-, -N(R⁴)-, =C(halo)-, =C(OR⁴)-, =C(SR⁴)-, =C(NR⁴)-, -O-, -S- and SO₂, wherein the phenyl moieties of said =C(C₆H₅)- and -CH(C₆H₅)- may optionally be substituted with from one to three substituents independently selected from trifluoromethyl and halo, and wherein the alkyl moieties of said =[(C₁-C₆)alkyl]- and -CH[C₁-C₆)alkyl]- may optionally be substituted with from one to three fluorine atoms;
Z is selected from =CH-, -CH₂-, =N-, -NH-, -S-, -N(R⁴)-, =C(C₆H₅)-, -CH(C₆H₅)-, =C[(C₁-C₆) alkyl]- and -CH[(C₁-C₆)alkyl]-;
or X, Y and Z, together with the two carbon atoms shared between the benzo ring and the XYZ ring, form a fused pyridine or pyrimidine ring;
R⁴ is (C₁-C₆) alkyl or phenyl;
R² is hydrogen or -CO₂(C₁-C₁₀)alkyl;
R³ is selected from 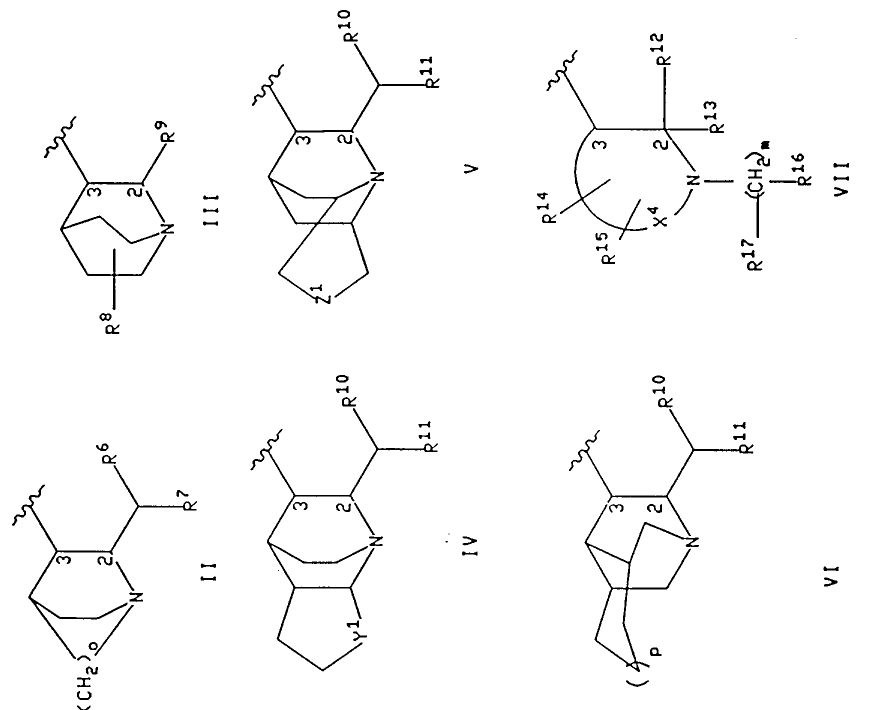 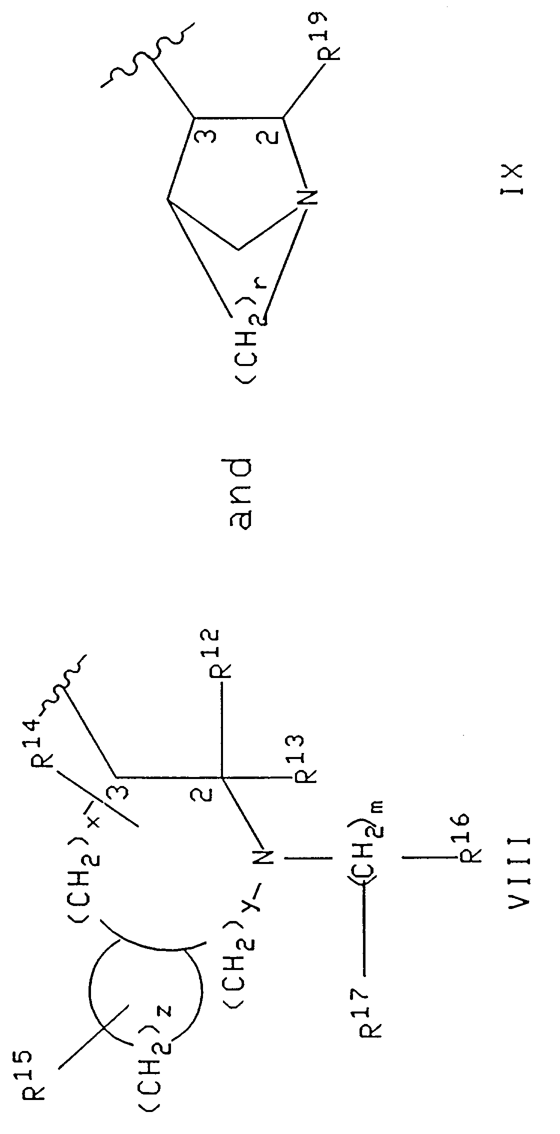 wherein R⁶ and R¹⁰ are independently selected from furyl, thienyl, pyridyl, indolyl, biphenyl and phenyl, wherein said phenyl may optionally be substituted with one or two substituents independently selected from halo, (C₁-C₁₀) alkyl optionally substituted with from one to three fluorine atoms, (C₁-C₁₀) alkoxy optionally substituted with from one to three fluorine atoms, carboxy, benzyloxycarbonyl and (C₁-C₃) alkoxy-carbonyl;
R⁷ is selected from (C₃-C₄) branched alkyl, (C₅-C₆) branched alkenyl, (C₅-C₇) cycloalkyl, and the radicals named in the definition of R⁶;
R⁸ is hydrogen or (C₁-C₆) alkyl;
R⁹ and R¹⁹ are independently selected from phenyl, biphenyl, naphthyl, pyridyl, benzhydryl, thienyl and furyl, and R⁹ and R¹⁹ may optionally be substituted with from one to three substituents independently selected from halo, (C₁-C₁₀) alkyl optionally substituted with from one to three fluorine atoms and (C₁-C₁₀) alkoxy optionally substituted with from one to three fluorine atoms;
Y¹ is (CH₂)ₗ wherein l is an integer from one to three, or Y¹ is a group of the formula 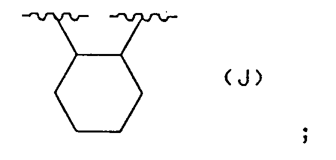 Z¹ is oxygen, sulfur, amino, (C₁-C₃)alkylamino or (CH₂)ₙ wherein n is zero, one or two;
x is an integer from zero to four;
y is an integer from zero to four;
z is an integer from one to six, wherein the ring containing (CH₂)_{z} may contain from zero to three double bonds, and one of the carbons of (CH₂)_{z} may optionally be replaced by oxygen, sulfur or nitrogen;
o is two or three;
p is zero or one;
r is one, two or three;
R¹¹ is thienyl, biphenyl or phenyl optionally substituted with one or two substituents independently selected from halo, (C₁-C₁₀) alkyl optionally substituted with from one to three fluorine atoms and (C₁-C₁₀) alkoxy optionally substituted with from one to three fluorine atoms;
X⁴ is (CH₂)_{q} wherein q is an integer from 1 to 6, and wherein any one of the carbon-carbon single bonds in said (CH₂)_{q} may optionally be replaced by a carbon-carbon double bond, and wherein any one of the carbon atoms of said (CH₂)_{q} may optionally be substituted with R¹⁴, and wherein any one of the carbon atoms of said (CH₂)_{q} may optionally be substituted with R¹⁵;
m is an integer from 0 to 8, and any one of the carbon-carbon single bonds of (CH₂)ₘ, wherein both carbon atoms of such bond are bonded to each other and to another carbon atom in the (CH₂)ₘ chain, may optionally be replaced by a carbon-carbon double bond or a carbon-carbon triple bond, and any one of the carbon atoms of said (CH₂)ₘ may optionally be substituted with R¹⁷;
R¹² is a radical selected from hydrogen, (C₁-C₆) straight or branched alkyl, (C₃-C₇)cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl-(C₂-C₆)alkyl, benzhydryl and benzyl, wherein the point of attachment on R¹² is a carbon atom unless R¹² is hydrogen, and wherein each of said aryl and heteroaryl groups and the phenyl moieties of said benzyl, phenyl-(C₂-C₆)alkyl and benzhydryl may optionally be substituted with one or more substituents independently selected from halo, nitro, (C₁-C₁₀)alkyl optionally substituted with from one to three fluorine atoms, (C₁-C₁₀)alkoxy optionally substituted with from one to three fluorine atoms, amino, hydroxy-(C₁-C₆)alkyl, (C₁-C₆)alkoxy-(C₁-C₆)alkyl, (C₁-C₆)-alkylamino, 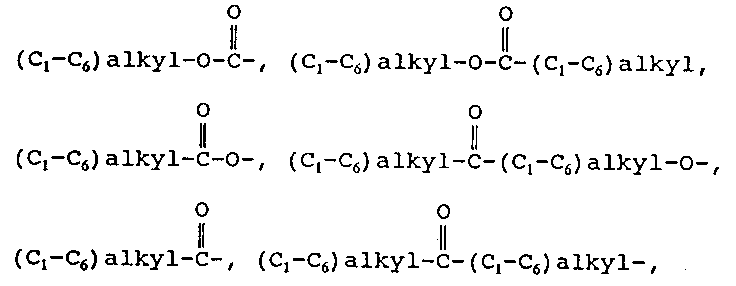 di-(C₁-C₆)alkylamino, 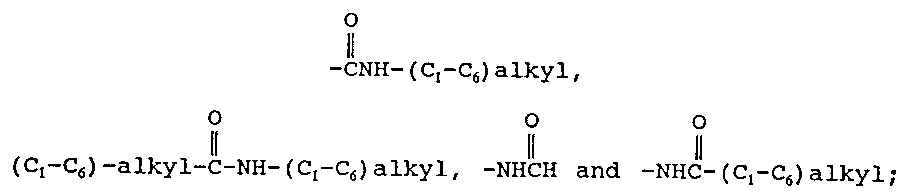 and wherein one of the phenyl moieties of said benzhydryl may optionally be replaced by naphthyl, thienyl, furyl or pyridyl;
R¹³ is hydrogen, phenyl or (C₁-C₆)alkyl;
or R¹² and R¹³, together with the carbon to which they are attached, form a saturated carbocyclic ring having from 3 to 7 carbon atoms wherein one of said carbon atoms that is neither the point of attachment of the spiro ring nor adjacent to it may optionally be replaced by oxygen, nitrogen or sulfur;
R¹⁴ and R¹⁵ are each independently selected from hydrogen, hydroxy, halo, amino, oxo (=O), cyano, hydroxy-(C₁-C₆)alkyl, (C₁-C₆)alkoxy-(C₁-C₆)alkyl, (C₁-C₆)alkylamino, di-(C₁-C₆)alkylamino, (C₁-C₆)alkoxy, 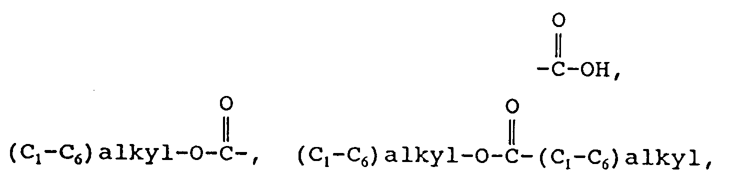 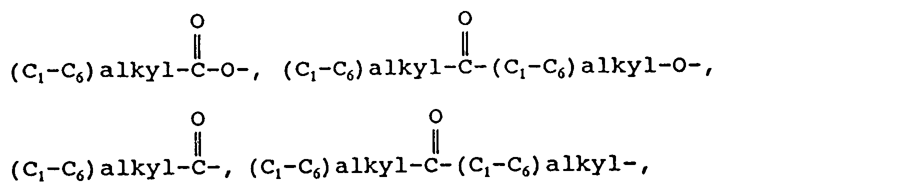 and the radicals set forth in the definition of R¹²;
R¹⁶ is 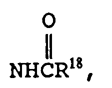 NHCH₂R¹⁸, SO₂R¹⁸, GR²⁰ CO₂H or one of the radicals set forth in any of the definitions of R¹², R¹⁴ and R¹⁵;
R¹⁷ is oximino (=NOH) or one of the radicals set forth in any of the definitions of R¹², R¹⁴ and R¹⁵; and
R¹⁸ is (C₁-C₆)alkyl, hydrogen, phenyl or phenyl (C₁-C₆)alkyl;
G is selected from the group consisting of CH₂, nitrogen, oxygen, sulfur and carbonyl;
R²⁰ is a monocyclic or bicyclic heterocycle selected from the group consisting of pyrimidinyl, benzoxazolyl, 2,3-dihydro-3-oxobenzisosulfonazol-2-yl, morpholin-1-yl, thiomorpholin-1-yl, benzofuranyl, benzothienyl, indolyl, isoindolyl, isoquinolinyl, furyl, pyridyl, isothiazolyl, oxazolyl, triazolyl, tetrazolyl, quinolyl, thiazolyl, thienyl, and groups of the formulae 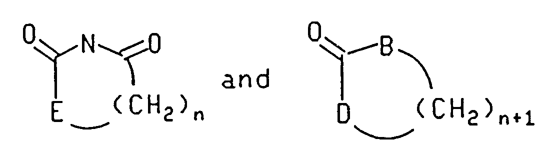 wherein B and D are selected from carbon, oxygen, and nitrogen, and at least one of B and D is other than carbon; E is carbon or nitrogen; n is an integer from 1 to 5; and any one of the carbons of the (CH₂)ₙ or (CH₂)ₙ₊₁ may be optionally substituted with (C₁-C₆) alkyl or (C₂-C₆) spiroalkyl, and either any two of the carbon atoms of said (CH₂)ₙ and (CH₂)ₙ₊₁ may be bridged by a one or two carbon atom linkage, or any one pair of adjacent carbons of said (CH₂)ₙ and (CH₂)ₙ₊₁ may form, together with from one to three carbon atoms that are not members of the carbonyl containing ring, a (C₃-C₅) fused carbocyclic ring;
with the proviso that (a) when m is 0, one of R¹⁶ and R¹⁷ is absent and the other is hydrogen, (b) when R³ is a group of the formula VIII, R¹⁴ and R¹⁵ cannot be attached to the same carbon atom, (c) when R¹⁴ and R¹⁵ are attached to the same carbon atom, then either each of R¹⁴ and R¹⁵ is independently selected from hydrogen, fluoro, (C₁-C₆)alkyl, hydroxy-(C₁-C₆)alkyl and (C₁-C₆)alkoxy-(C₁-C₆)alkyl, or R¹⁴ and R¹⁵, together with the carbon to which they are attached, form a (C₃-C₆) saturated carbocyclic ring that forms a spiro compound with the nitrogen-containing ring to which they are attached; (d) R¹² and R¹³ cannot both be hydrogen; (e) when R¹⁴ or R¹⁵ is attached to a carbon atom of X⁴ or (CH₂)_{y} that is adjacent to the ring nitrogen, then R¹⁴ or R¹⁵, respectively, must be a substituent wherein the point of attachment is a carbon atom; (f) neither R¹⁴, R¹⁵, R¹⁶ nor R¹⁷ can form a ring with R¹³; and (g) the compound of formula Ia cannot be (2S,3S)-N-(2-methoxyphenyl)methyl-2-diphenylmethyl-1-azabicyclo[2.2.2]octan-3-amine;
or a pharmaceutically acceptable salt thereof.

3. The use according to claim 1, wherein the compound is a compound having the formula 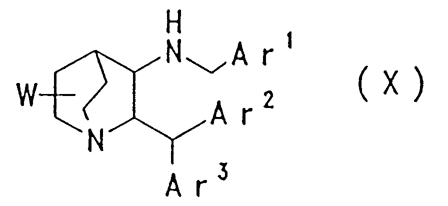 wherein W is Y or X(CH₂)ₙ;
Y is optionally substituted (C₁-C₆)alkyl, optionally substituted (C₂-C₆)alkenyl or optionally substituted (C₃-C₈)cycloalkyl;
X is optionally substituted (C₁-C₆)alkoxy, hydroxy, CONR¹R², CO₂R¹, CHR¹OR², CHR¹NR²R³, COR¹, CONR¹OR² or optionally substituted aryl, wherein said aryl is selected from phenyl, naphthyl, pyridyl, quinolyl, thienyl, furyl, phenoxyphenyl, oxazolyl, tetrazolyl, thiazolyl, imidazolyl and pyrazolyl; and n is an integer from zero to six;
Ar¹, Ar² and Ar³ are each, independently, optionally substituted aryl, wherein said aryl is selected from phenyl, naphthyl, pyridyl, quinolyl, thienyl, furyl, phenoxyphenyl, oxazolyl, tetrazolyl, thiazolyl, imidazolyl and pyrazolyl;
and R¹, R² and R³ are independently selected from hydrogen, optionally substituted (C₁-C₆)alkyl, optionally substituted (C₁-C₆)alkoxy, optionally substituted (C₃-C₈)cycloalkyl, optionally substituted aryl, wherein said aryl is selected from phenyl, naphthyl, pyridyl, quinolyl, thienyl, furyl, phenoxyphenyl, oxazolyl, tetrazolyl, thiazolyl, imidazolyl and pyrazolyl; and optionally substituted (C₁-C₅)heterocyclic groups, wherein said heterocyclic groups are selected from pyrrolidino, piperidino, morpholino, piperazinyl and thiamorpholino;
and wherein the substituents on the foregoing substituted alkyl, alkenyl, cycloalkyl and alkoxy groups are independently selected from halo, nitro, amino, (C₁-C₄)alkyl, (C₁-C₄)alkoxy, trifluoromethyl and trifluoromethoxy;
and wherein the substituents on the foregoing substituted (C₁-C₅) heterocyclic groups are attached to a sulfur or nitrogen atom on the ring and are independently selected from oxygen, di-oxygen and (C₁-C₄)alkyl when attached to a ring sulfur atom and are independently selected from oxygen and (C₁-C₄)alkyl when attached to a ring nitrogen atom;
and wherein the substituents on said substituted Ar¹ groups are independently selected from (C₁-C₆)alkyl optionally substituted with from one to three halo groups, (C₁-C₆)alkoxy optionally substituted with from one to three halo groups, (C₁-C₆)alkylsulfinyl, (C₂-C₆)alkenyl, (C₁-C₆)alkylthio, (C₁-C₆)alkylsulfonyl, (C₁-C₆)alkylsulfonylamino, and di-(C₁-C₆)alkylamino wherein one or both of the alkyl groups may be optionally substituted with a (C₁-C₆)alkylsulfonyl, or (C₁-C₆)alkylsulfinyl group;
and wherein the substituents on said substituted Ar² and Ar³ groups are independently selected from (C₁-C₄) alkyl, (C₁-C₄)alkoxy, (C₁-C₄)alkylthio, (C₁-C₄)alkylsulfinyl, di-(C₁-C₄)alkylamino, trifluoromethyl and trifluoromethoxy; with the proviso that when Y is unsubstituted or is substituted with (C₁-C₄)alkyl, it is attached to the 4- or 6-position of the quinuclidine ring;
or a pharmaceutically acceptable salt of such compound.

4. The use according to claim 1, wherein the compound is a compound having the formula 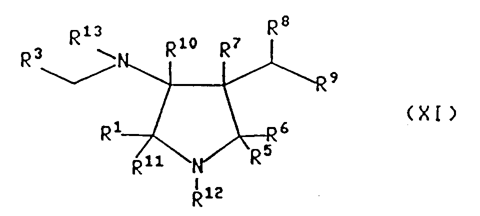 wherein R¹ is selected from hydrogen, (C₁-C₆) straight or branched alkyl, (C₃-C₇) cycloalkyl wherein one of the carbon atoms may optionally be replaced by nitrogen, oxygen or sulfur; aryl selected from phenyl, biphenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents independently selected from halo, nitro, (C₁-C₆) alkyl optionally substituted with from one to three fluorine atoms, (C₁-C₆) alkoxy, amino, trihaloalkoxy (e.g., trifluoromethoxy),
(C₁-C₆)alkylamino, 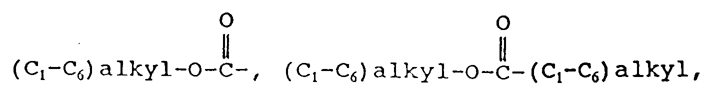 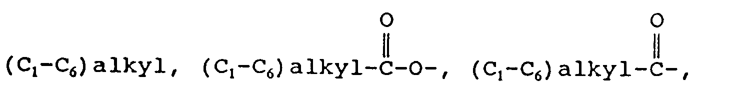 (C₁-C₆)alkyl-O-, 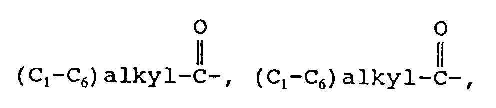 (C₁-C₆)alkyl-, di-(C₁-C₆)alkylamino, 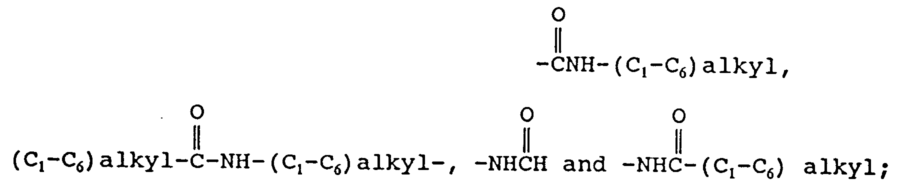 and wherein one of the phenyl moieties of said benzhydryl may optionally be replaced by naphthyl, thienyl, furyl or pyridyl;
R³ is aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; and cycloalkyl having 3 to 7 carbon atoms wherein one of said carbon atoms may optionally be replaced by nitrogen, oxygen or sulfur; wherein each of said aryl and heteroaryl groups may optionally be substituted with one or more substituents, and said (C₃-C₇) cycloalkyl may optionally be substituted with one or two substituents, each of said substituents being independently selected from halo, nitro, (C₁-C₆) alkyl optionally substituted with from one to three fluorine atoms, (C₁-C₆) alkoxy substituted with from one to three fluorine atoms, amino, phenyl, trihaloalkoxy, (C₁-C₆) alkylamino, 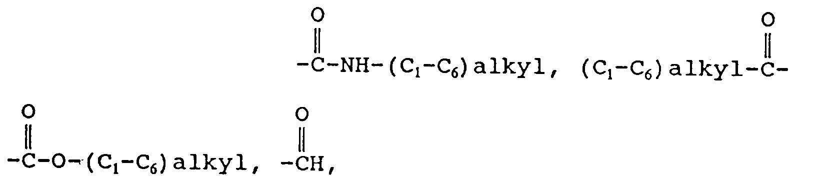 -CH₂OR¹³, NH(C₁-C₆)alkyl-, 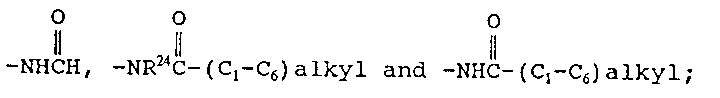 one of R⁵ and R⁶ is hydrogen and the other is selected from hydroxymethyl, hydrogen, (C₁-C₃)alkyl, (C₁-C₈)acyloxy-(C₁-C₃)alkyl, (C₁-C₈)alkoxymethyl and benzyloxymethyl;
R⁷ and R⁸ are independently selected from hydrogen, (C₁-C₃)alkyl and phenyl;
R⁹ is selected from methyl, hydroxymethyl, 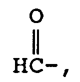 R¹⁴R¹⁵NCO₂CH₂-, R¹⁶OCO₂CH₂-, (C₁-C₄)alkyl-CO₂CH₂-, -CONR¹⁷R¹⁸, R¹⁷R¹⁸NCO₂-, R¹⁹OCO₂-, C₆H₅CH₂CO₂CH₂-, C₆H₅CO₂CH₂-, (C₁-C₄)alkyl-CH(OH)-, C₆H₅CH(OH)-, C₆H₅CH₂CH(OH)-, CH₂halo, R²⁰SO₂OCH₂, -CO₂R¹⁶ and R²¹CO₂-;
R¹⁰ and R¹¹ are independently selected from hydrogen, (C₁-C₃) alkyl and phenyl;
R¹² is hydrogen, benzyl or a group of the formula 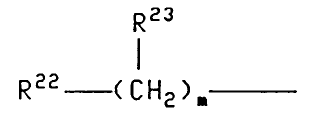 wherein m is an integer from zero to twelve, and any one of the carbon-carbon single bonds of (CH₂)ₘ, wherein both carbon atoms of such bond are bonded to each other and to another carbon atom of the (CH₂)ₘ chain, may optionally be replaced by a carbon-carbon double or triple bond, and any one of the carbon atoms of (CH₂)ₘ may optionally be substituted with R²³;
R¹³, R¹⁴, R¹⁵, R¹⁶, R¹⁷, R¹⁸, R¹⁹, R²⁰, R²¹ and R²⁴ are independently selected from hydrogen, (C₁-C₃)alkyl and phenyl;
R²² and R²³ are independently selected from hydrogen, hydroxy, halo, amino, carboxy, carboxy(C₁-C₆)alkyl, (C₁-C₆)alkylamino, di-(C₁-C₆)alkylamino, (C₁-C₆)alkoxy, 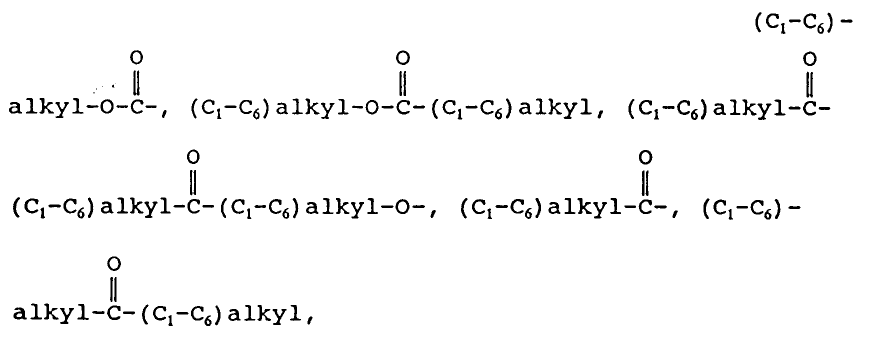 (C₁-C₆) straight or branched alkyl, (C₃-C₇) cycloalkyl wherein one of the carbon atoms may optionally be replaced by nitrogen, oxygen or sulfur; aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; phenyl-(C₂-C₆)alkyl, benzhydryl and benzyl, wherein each of said aryl and heteroaryl groups and the phenyl moieties of said benzyl, phenyl-(C₂-C₆)alkyl and benzhydryl may optionally be substituted with one or two substituents independently selected from halo, nitro, (C₁-C₆)alkyl optionally substituted with from one to three fluorine atoms, (C₁-C₆)alkoxy optionally substituted with from one to three fluorine atoms,
trifluoromethyl, amino, (C₁-C₆)-alkylamino, 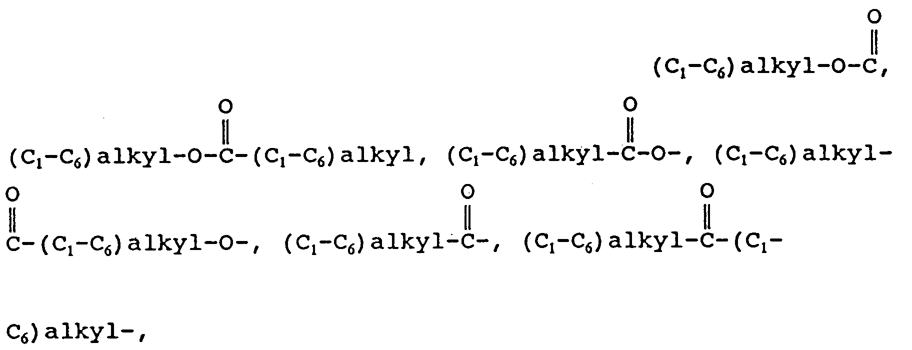 di-(C₁-C₆)alkylamino, 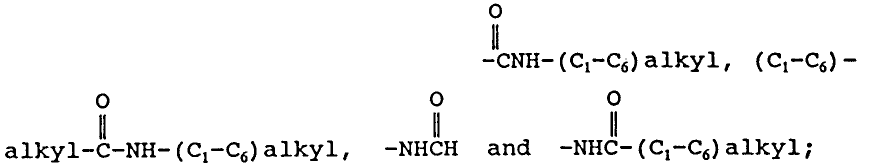 and wherein one of the phenyl moieties of said benzhydryl may optionally be replaced by naphthyl, thienyl, furyl or pyridyl;
or R⁹, together with the carbon to which it is attached, the nitrogen of the pyrrolidine ring, the carbon to which R⁷ is attached and the carbon to which R⁵ and R⁶ are attached form a second pyrrolidine ring; with the proviso that when R⁹, together with the carbon to which it is attached, the nitrogen of the pyrrolidine ring, the carbon to which R⁷ is attached and the carbon to which R⁵ and R⁶ are attached, form a second pyrrolidine ring (thus forming a bicyclic structure containing a bridgehead nitrogen), either R¹² is absent or R¹² is present and the nitrogen of the second pyrrolidine ring is positively charged;
or a pharmaceutically acceptable salt of such compound.

5. The use according to claim 1, wherein the compound is a compound of the formula 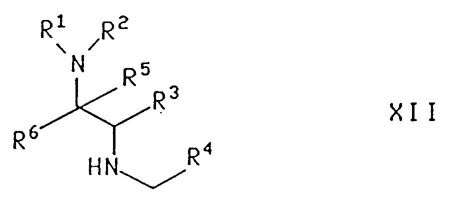 wherein R¹ is hydrogen, (C₁-C₈) alkyl, a saturated (C₆-C₁₀) carbocyclic ring system containing two fused rings, a saturated (C₆-C₁₀) carbocyclic bridged ring system containing two rings, or benzyl wherein the phenyl moiety of said benzyl may optionally be substituted with one or more substituents independently selected from halo, (C₁-C₆) alkyl optionally substituted with from one to three fluorine atoms and (C₁-C₈) alkoxy optionally substituted with from one to three fluorine atoms;
R² is hydrogen, benzyl or a group of the formula 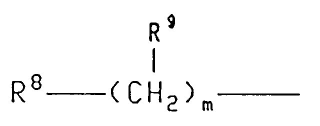 wherein m is an integer from zero to twelve, and any one of the carbon-carbon single bonds of (CH₂)ₘ, wherein both carbon atoms of such bond are bonded to each other and to another carbon atom of the (CH₂)ₘ chain, may optionally be replaced by a carbon-carbon double or triple bond, and any one of the carbon atoms of (CH₂)ₘ may optionally be substituted with R⁹;
R⁸ and R⁹ are independently selected from hydrogen, hydroxy, halo, amino, carboxy, carboxy (C₁-C₆) alkyl, (C₁-C₆)alkylamino, di-(C₁-C₆)alkylamino, (C₁-C₆)alkoxy, 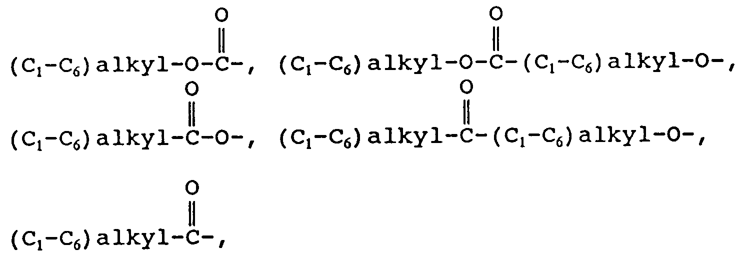 (C₁-C₆) straight or branched alkyl, (C₃-C₇) cycloalkyl wherein one of the carbon atoms may optionally be replaced by nitrogen, oxygen or sulfur; aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; phenyl-(C₂-C₆)alkyl, benzhydryl and benzyl, wherein each of said aryl and heteroaryl groups and the phenyl moieties of said benzyl, phenyl-(C₂-C₆)alkyl and benzhydryl may optionally be substituted with one or two substituents independently selected from halo, nitro, (C₁-C₆)alkyl optionally substituted with from one to three fluorine atoms, (C₁-C₆)alkoxy optionally substituted with from one to three fluorine atoms,
trifluoromethyl, amino, (C₁-C₆)-alkylamino, 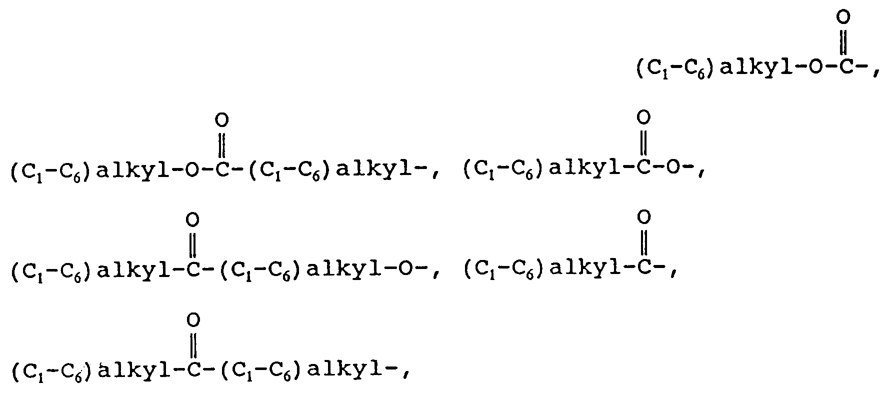 di-(C₁-C₆)alkylamino, 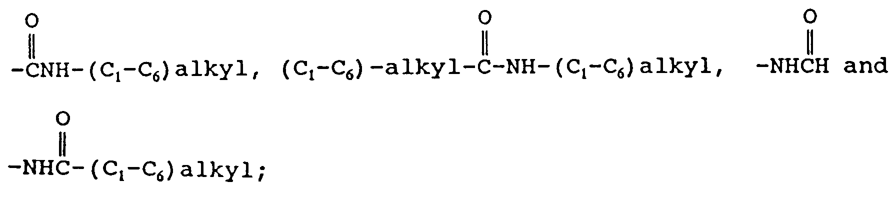 and wherein one of the phenyl moieties of said benzhydryl may optionally be replaced by naphthyl, thienyl, furyl or pyridyl;
or R¹ and R², together with the nitrogen to which they are attached, form a saturated or unsaturated monocyclic ring containing from three to eight carbon atoms, a fused bicyclic ring containing from six to ten carbon atoms, or a saturated bridged ring system containing from six to ten carbon atoms;
R⁴ is aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; and cycloalkyl having from three to seven carbon atoms wherein one of said carbon atoms may optionally be replaced by nitrogen, oxygen or sulfur; wherein each of said aryl and heteroaryl groups may optionally be substituted with one or more substituents, and said (C₃-C₇) cycloalkyl may optionally be substituted with one, two or three substituents, each of said substituents being independently selected from halo, nitro, (C₁-C₆) alkyl optionally substituted with, from one to three fluorine atoms, (C₁-C₆) alkoxy optionally substituted with from one to three fluorine atoms, phenyl,
amino, (C₁-C₆) alkylamino, 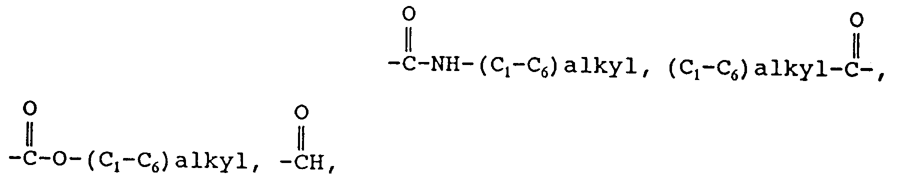 -CH₂OR¹²,NH₂(C₁-C₆)alkyl-, 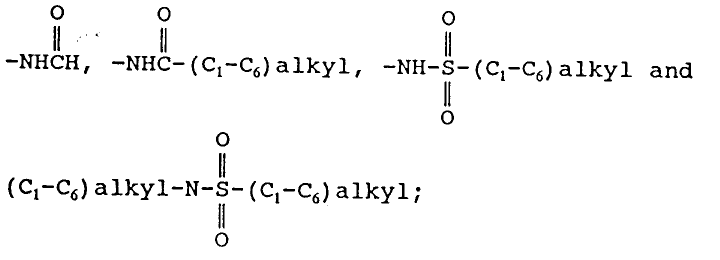 R³ is hydrogen, (C₃-C₈) cycloalkyl, (C₁-C₆) straight or branched alkyl or phenyl optionally substituted with one or more substituents independently selected from halo, (C₁-C₆)alkyl optionally substituted with from one to three fluorine atoms, and (C₁-C₆)alkoxy optionally substituted with from one to three fluorine atoms;
R⁵ is hydrogen, (C₁-C₆)alkyl, or phenyl optionally substituted with one or more substituents independently selected from halo, (C₁-C₆)alkyl optionally substituted with from one to three fluorine atoms and (C₁-C₆) alkoxy optionally substituted with from one to three fluorine atoms;
R⁶ is selected from hydrogen, (C₁-C₆) straight or branched alkyl, (C₃-C₇) cycloalkyl wherein one of the carbon atoms may optionally be replaced by nitrogen, oxygen or sulfur; aryl selected from phenyl, biphenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents independently selected from halo, nitro, (C₁-C₆) alkyl optionally substituted with from one to three fluorine atoms, (C₁-C₆) alkoxy, trifluoromethyl, amino, trihaloalkoxy (e.g.,trifluoromethoxy), (C₁-C₆)alkylamino, 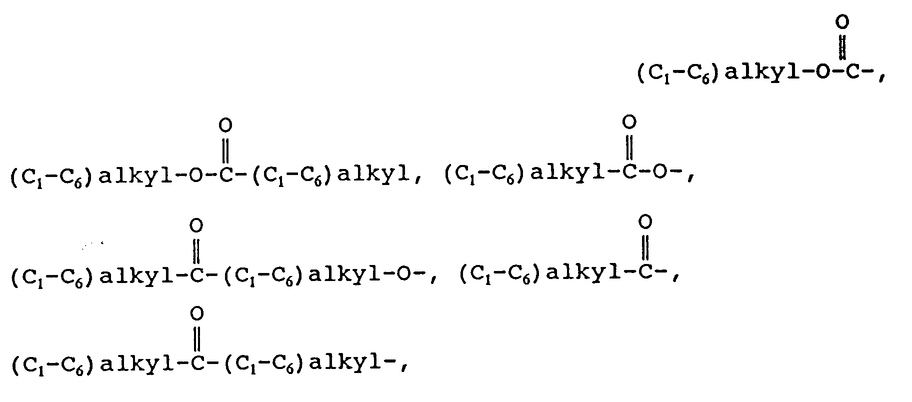 di-(C₁-C₆)alkylamino, 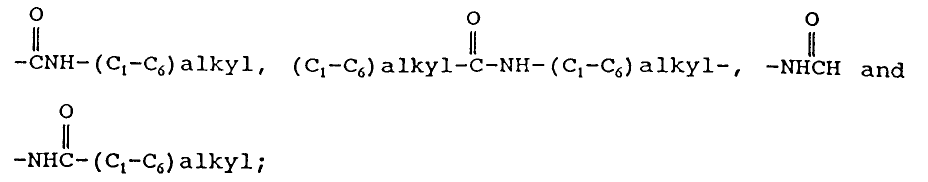 and wherein one of the phenyl moieties of said benzhydryl may optionally be replaced by naphthyl, thienyl, furyl or pyridyl; and
R¹² is hydrogen, (C₁-C₃)alkyl or phenyl;
or a pharmaceutically acceptable salt of such compound.

6. The use according to claim 1, wherein the compound is a compound of the formula 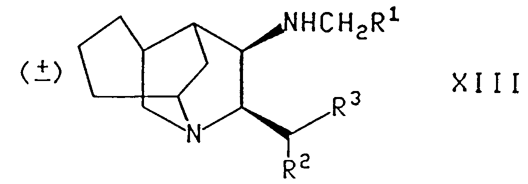 wherein R¹ is cycloalkyl having from five to seven carbon atoms, pyrrolyl, thienyl, pyridyl, phenyl or substituted phenyl, wherein said substituted phenyl is substituted with from one to three substituents independently selected from fluorine, chlorine, bromine, trifluoromethyl, alkyl having from one to three carbon atoms, alkoxy having from one to three carbon atoms, carboxy, alkoxycarbonyl having from one to three carbon atoms in the alkoxy moiety and benzyloxycarbonyl;
R² is furyl, thienyl, pyridyl, indolyl, biphenyl, phenyl or substituted phenyl, wherein said substituted phenyl is substituted with one or two substituents independently selected from fluorine, chlorine, bromine, trifluoromethyl, alkyl having from one to three carbon atoms, alkoxy having from one to three carbon atoms, carboxy, alkoxycarbonyl having from one to three carbon atoms in the alkoxy moiety and benzyloxycarbonyl; and
R³ is thienyl, phenyl, fluorophenyl, chlorophenyl or bromophenyl;
or a pharmaceutically acceptable salt of such compound.

7. The use according to claim 1, wherein the compound is a compound of the formula 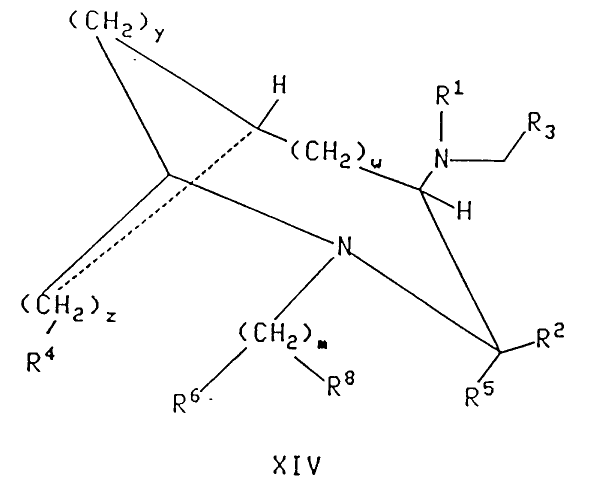 wherein m is an integer from 0 to 8, and any one of the carbon-carbon single bonds of (CH₂)ₘ, wherein both carbon atoms of such bond are bonded to each other and to another carbon in the (CH₂)ₘ chain, may optionally be replaced by a carbon-carbon double bond or a carbon-carbon triple bond, and any one of the carbon atoms of said (CH₂)ₘ may optionally be substituted with R⁸;
w is an integer from 0 to 2;
y is an integer from 1 to 4;
z is an integer from 1 to 4, and wherein any one of the carbon atoms of said (CH₂)_{z} may optionally be substituted with R⁴;
R¹ is hydrogen or (C₁-C₈) alkyl optionally substituted with hydroxy, alkoxy or fluoro;
R² is a group selected from hydrogen, (C₁-C₆)straight or branched alkyl, (C₃-C₇)cycloalkyl wherein one of the carbon atoms may optionally be replaced by nitrogen, oxygen or sulfur; aryl selected from phenyl, indanyl, and naphthyl; heteroaryl selected from thienyl, furyl, pyridyl, thiazolyl, isothiazolyl, oxazolyl, isoxazolyl, triazolyl, tetrazolyl and quinolyl; phenyl(C₂-C₆)alkyl, benzhydryl and benzyl, wherein one of the phenyl moieties of said benzhydryl may optionally be replaced by naphthyl, thienyl, furyl or pyridyl and wherein each of said aryl and heteroaryl groups and the phenyl moieties of said benzyl, phenyl(C₂-C₆)alkyl and benzhydryl may optionally be substituted with one or more substituents independently selected from halo, nitro, (C₁-C₆)alkyl, (C₁-C₆)alkoxy, trifluoromethyl, amino, (C₁-C₆)-alkylamino, 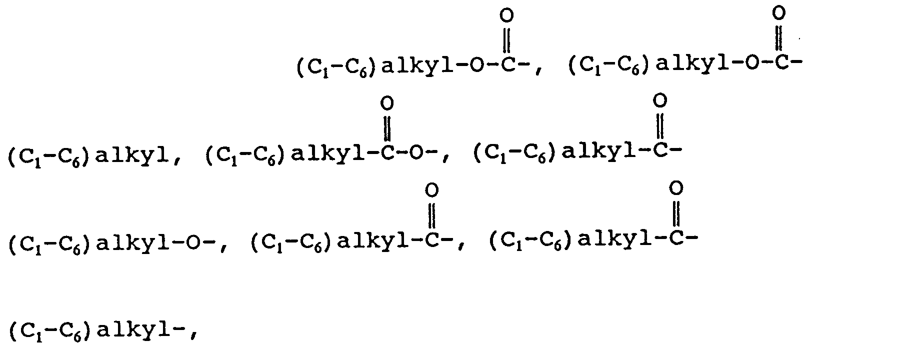 di-(C₁-C₆)alkylamino, 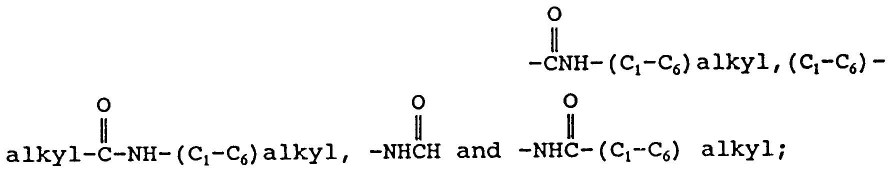 R⁵ is hydrogen, phenyl or (C₁-C₆)alkyl;
or R² and R⁵, together with the carbon to which they are attached, form a saturated carbocyclic ring having from 3 to 7 carbon atoms wherein one of said carbon atoms may optionally be replaced by oxygen, nitrogen or sulfur;
R³ is aryl selected from phenyl, indanyl, and naphthyl; heteroaryl selected from thienyl, furyl, pyridyl, thiazolyl, isothiazolyl, oxazolyl, isoxazolyl, triazolyl, tetrazolyl and quinolyl; and cycloalkyl having 3 to 7 carbon atoms wherein one of said carbon atoms may optionally be replaced by nitrogen, oxygen or sulfur; wherein each of said aryl and heteroaryl groups may optionally be substituted with one or more substituents, and said (C₃-C₇) cycloalkyl may optionally be substituted with one or two substituents, each of said substituents being independently selected from halo, nitro, (C₁-C₆)alkyl optionally substituted with from one to three fluorine atoms, (C₁-C₆)alkoxy optionally substituted with from one to three fluorine atoms, trifluoromethyl, phenyl, amino, (C₁-C₆)alkylamino, (C₁-C₆)dialkyl amino, 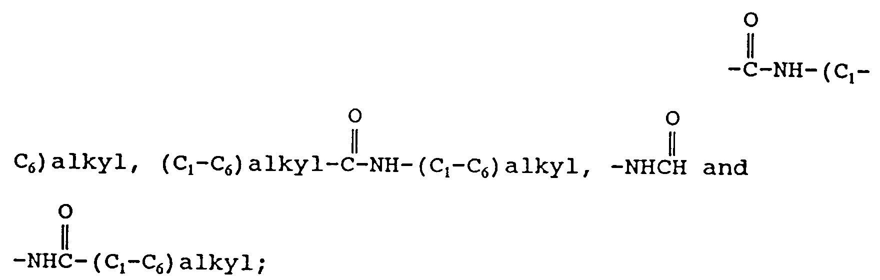 R⁴ is independently selected from hydrogen, hydroxy, halo, amino, oxo (=O), nitrile,
(C₁-C₆)alkylamino, di-(C₁-C₆)alkylamino, (C₁-C₆)alkoxy, 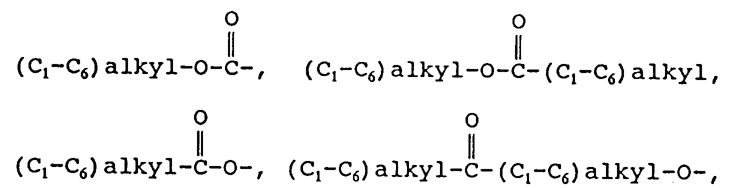 hydroxy-(C₁-C₆)alkyl, (C₁-C₆)alkoxy(C₁-C₆)alkyl, 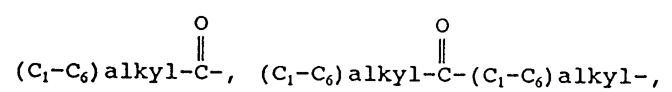 and the groups set forth in the definition of R²;
R⁶ is 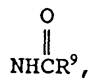 NHCH₂R⁹, NHSO₂R⁹ or one of the groups set forth in any of the definitions of R², and R⁴;
R⁸ is oximino (=NOH) or one of the groups set forth in any of the definitions of R², and R⁴;
R⁹ is (C₁-C₆)alkyl, hydrogen, phenyl or phenyl (C₁-C₆)alkyl;
with the proviso that (a) when m is 0, R⁸ is absent and R⁶ is hydrogen, (b) neither R⁴, R⁶, nor R⁸ can form, together with the carbon to which it is attached, a ring with R⁵, and (c) the sum of y and z must be less than 7;
or a pharmaceutically acceptable salt thereof.

8. The use according to claim 1, wherein the compound is a compound of the formula 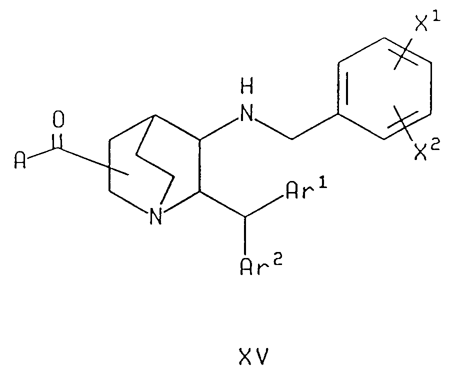 wherein X¹ is (C₁-C₅) alkoxy or halosubstituted (C₁-C₅) alkoxy;
X² is hydrogen, halogen, (C₁-C₅)alkyl, (C₂-C₅)alkenyl, (C₂-C₅)alkynyl, (C₁-C₅)alkoxy, (C₁-C₅)alkylthio, (C₁-C₅)alkylsulfinyl, (C₁-C₅)alkylsulfonyl, halosubstituted (C₁-C₅)alkyl, halosubstituted (C₁-C₅)alkoxy, (C₁-C₅)alkylamino, dialkylamino having from 1 to 5 carbon atoms in each alkyl moiety, (C₁-C₅)alkylsulfonylamino (which may be substituted by halogen), 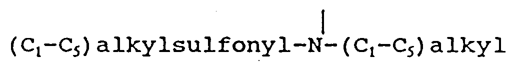 (which may be substituted by halogen in the alkylsulfonyl moiety), (C₁-C₅)alkanoylamino (which may be substituted by halogen) or 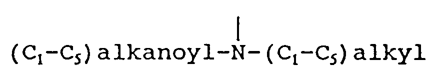 (which may be substituted by halogen in the alkanoyl moiety);
Ar¹ and Ar₂ are each, independently, thienyl, phenyl, fluorophenyl, chlorophenyl or bromophenyl;
A is Y-(CH₂)ₘ-CH(R²)-(CH₂)ₙ-NR¹-;
R¹ is hydrogen, (C₁-C₅)alkyl, benzyl or -(CH₂)ₚ-Y;
R² is hydrogen, (C₁-C₅)alkyl (which may be substituted by a substituent selected from the group consisting of hydroxy, amino, methylthio and mercapto), benzyl, 4-hydroxybenzyl, 3-indolylmethyl or -(CH₂)ₚ-Y;
Y is -CN, -CH₂Z or -COZ;
Z is hydroxy, amino, (C₁-C₅)alkoxy, (C₁-C₅)alkylamino or dialkylamino having from 1 to 5 carbon atoms in each alkyl moiety;
m, n and p are each, independently, 0, 1, 2 or 3; and
R¹ and R² may be connected to form a ring;
or a pharmaceutically acceptable salt thereof.

9. The use according to claim 1, wherein the compound is a compound of the formula 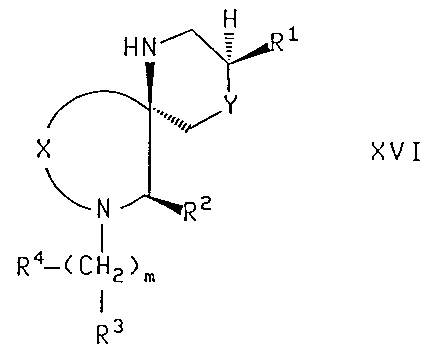 wherein R¹ is phenyl optionally substituted with one or more substituents, preferably with from one to three substituents, independently selected from hydrogen, halo, nitro, (C₁-C₁₀) alkyl optionally substituted with from one to three fluorine atoms, (C₁-C₁₀) alkoxy optionally substituted with from one to three fluorine atoms, trifluoromethyl, hydroxy, phenyl, cyano, amino, (C₁-C₆)-alkylamino, di-(C₁-C₆)alkylamino, 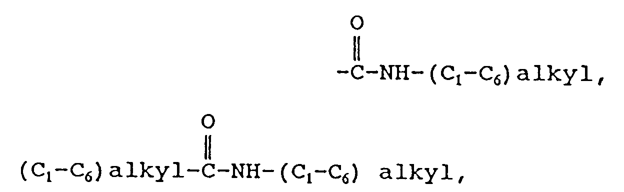 hydroxy(C₁-C₄)alkyl, 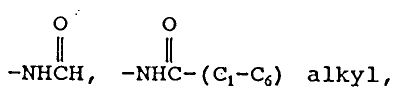 (C₁-C₄)alkoxy(C₁-C₄)alkyl, -S(O)ᵥ-(C₁-C₁₀)-alkyl wherein v is zero, one or two, -S(O)ᵥ-aryl wherein v is zero, one or two, -O-aryl, -SO₂NR⁴R⁵ wherein each of R⁴ and R⁵ is, independently, (C₁-C₆)alkyl, or R⁴ and R⁵, together with the nitrogen to which they are attached, form a saturated ring containing one nitrogen and from 3 to 6 carbons, 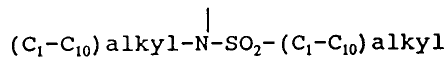 wherein one or both of the alkyl moieties may optionally be substituted with from one to three fluorine atoms, -N(SO₂-(C₁-C₁₀)alkyl)₂ and 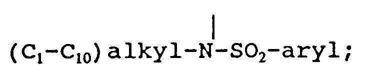 and wherein the aryl moieties of said -S(O)ᵥ-aryl, -O-aryl and 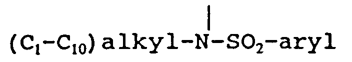 are independently selected from phenyl and benzyl and may optionally be substituted with from one to three substituents independently selected from (C₁-C₄)alkyl, (C₁-C₄)alkoxy and halo;
or R¹ is phenyl substituted with a group having the formula 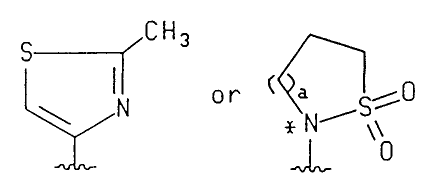 wherein a is 0, 1 or 2 and the asterisk represents a position meta to the point of attachment of R¹;
R² is selected from (C₁-C₆) straight or branched alkyl, (C₃-C₇) cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents, preferably with from one to three substituents, independently selected from halo, nitro, (C₁-C₁₀) alkyl optionally substituted with from one to three fluorine atoms, (C₁-C₁₀) alkoxy optionally substituted with from one to three fluorine atoms, amino, hydroxy-(C₁-C₆)alkyl, (C₁-C₆)alkoxy-(C₁-C₆)alkyl, (C₁-C₆)-alkylamino, 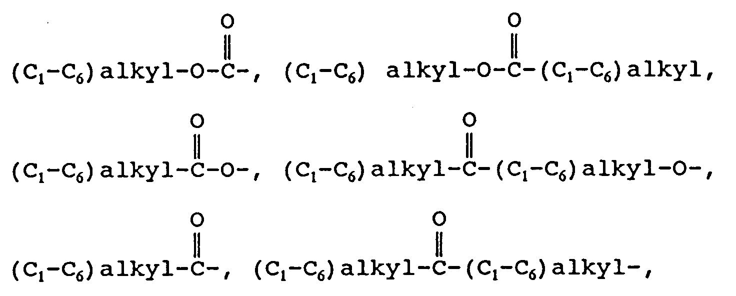 di-(C₁-C₆)alkylamino, 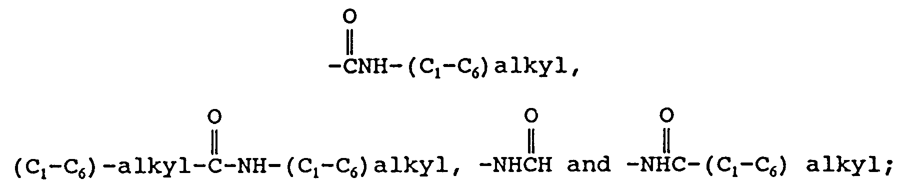 and wherein one of the phenyl moieties of said benzhydryl may optionally be replaced by naphthyl, thienyl, furyl or pyridyl;
m is an integer from 0 to 8, and any one of the carbon-carbon single bonds of (CH₂)ₘ, wherein both carbon atoms of such bond are bonded to each other and to another carbon atom in the (CH₂)ₘ chain, may optionally be replaced by a carbon-carbon double bond or a carbon-carbon triple bond, and any one of the carbon atoms of said (CH₂)ₘ may optionally be substituted with R⁴;
R³ is selected from 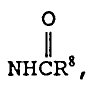 NHCH₂R⁸, SO₂R⁸, AR⁹, CO₂H and the radicals set forth in the definitions of R², R⁶ and R⁷;
A is CH₂, nitrogen, oxygen, sulfur or carbonyl;
R⁸ is (C₁-C₆)alkyl, hydrogen, phenyl or phenyl (C₁-C₆)alkyl;
R⁴ is selected from oximino (=NOH) and the radicals set forth in the definitions of R², R⁶ and R⁷;
R⁹ is a monocyclic or bicyclic heterocycle selected from the group consisting of pyrimidinyl, benzoxazolyl, 2,3-dihydro-3-oxobenzisosulfonazol-2-yl, morpholin-1-yl, thiomorpholin-1-yl, benzofuranyl, benzothienyl, indolyl, isoindolyl, isoquinolinyl, furyl, pyridyl, isothiazolyl, oxazolyl, triazolyl, tetrazolyl, quinolyl, thiazolyl, thienyl, and groups of the formulae 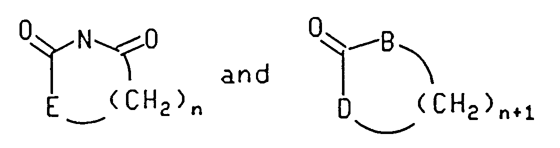 wherein B and D are selected from carbon, oxygen and nitrogen, and at least one of B and D is other than carbon; E is carbon or nitrogen; n is an integer from 1 to 5; any one of the carbon atoms of said (CH₂)ₙ and (CH₂)ₙ₊₁ may be optionally substituted with (C₁-C₆)alkyl or (C₂-C₆) spiroalkyl; and either any one pair of the carbon atoms of said (CH₂)ₙ and (CH₂)ₙ₊₁ may be bridged by a one or two carbon atom linkage, or any one pair of adjacent carbon atoms of said (CH₂)ₙ and (CH₂)ₙ₊₁ may form, together with from one to three carbon atoms that are not members of the carbonyl containing ring, a (C₃-C₅) fused carbocyclic ring;
X is (CH₂)_{q} wherein q is two or three and wherein one of the carbon-carbon single bonds in said (CH₂)_{q} may optionally be replaced by a carbon-carbon double bond, and wherein any one of the carbon atoms of said (CH₂)_{q} may optionally be substituted with R⁶, and wherein any one of the carbon atoms of said (CH₂)_{q} may optionally be substituted with R⁷;
R⁶ and R⁷ are independently selected from hydrogen, hydroxy, halo, amino, oxo (=O), cyano, hydroxy-(C₁-C₆)alkyl, (C₁-C₆)alkoxy-(C₁-C₆)alkyl, (C₁-C₆)alkylamino, di-(C₁-C₆)alkylamino, (C₁-C₆)alkoxy, 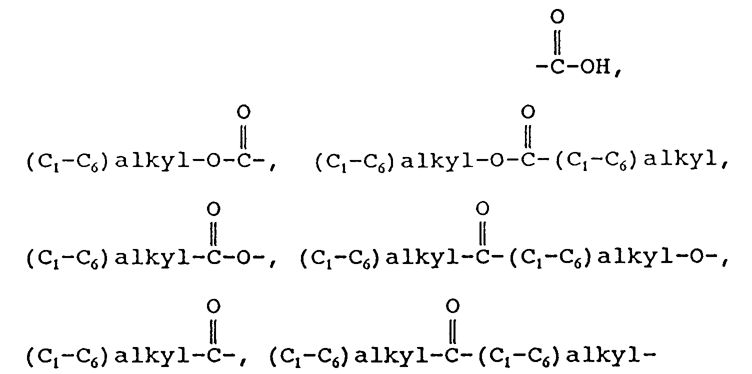 and the radicals set forth in the definition of R²; and
Y is (CH₂)_{z} wherein z is zero or one;
with the proviso that: (a) when A is -(CH₂)- or carbonyl, R⁹ cannot be furyl, pyridyl, isothiazolyl, oxazolyl, triazolyl, tetrazolyl, quinolyl, thiazolyl or thienyl; (b) when m is zero, one of R³ and R⁴ is absent and the other is hydrogen; and (c) when R⁶ or R⁷ is attached to a carbon atom of X that is adjacent to the ring nitrogen, then R⁶ or R⁷, respectively, must be a substituent wherein the point of attachment is a carbon atom;
or a pharmaceutically acceptable salt of such compound.

10. The use according to claim 2, wherein the compound is selected from the group consisting of:
(2S,3S)-3-(5-tert-butyl-2-methoxybenzyl)amino-2-(3-trifluoromethoxyphenyl)piperidine;
(2S,3S)-3-(2-isopropoxy-5-trifluoromethoxybenzyl)amino-2-phenyl-piperidine;
(2S,3S)-3-(2-ethoxy-5-trifluoromethoxybenzyl)amino-2-phenyl-piperidine;
(2S,3S)-3-(2-methoxy-5-trifluoromethoxybenzyl)-amino-2-phenylpiperidine;
(2S,3S)-3(-5-tert-butyl-2-trifluoromethoxybenzyl)amino-2-phenylpiperidine;
2-(diphenylmethyl)-N-(2-methoxy-5-trifluoromethoxyphenyl)methyl-1-azabicyclo[2.2.2]octan-3-amine;
(2S,3S)-3-[5-chloro-2-(2,2,2-trifluoroethoxy)benzyl]amino-2-phenylpiperidine;
(2S,3S)-3-(5-tert-butyl-2-trifluoromethoxybenzyl)amino-2-phenylpiperidine;
(2S,3S)-3-(2-isopropoxy-5-trifluoromethoxybenzyl)amino-2-phenylpiperidine;
(2S,3S)-3-(2-difluoromethoxy-5-trifluoromethoxybenzyl)-amino-2-phenylpiperidine;
(2S,3S)-2-phenyl-3-[2-(2,2,2-trifluoroethoxybenzyl)-aminopiperidine; and
(2S,3S)-2-phenyl-3-(2-trifluoromethoxybenzyl)]aminopiperidine.

11. The use according to claim 2, wherein the compound is selected from the group consisting of:
cis-3-(2-chlorobenzylamino)-2-phenylpiperidine;
cis-3-(2-trifluoromethylbenzylamino)-2-phenyl-piperidine;
cis-3-(2-methoxybenzylamino)-2-(2-fluorophenyl)-piperidine;
cis-3-(2-methoxybenzylamino)-2-(2-chlorophenyl)-piperidine;
cis-3-(2-methoxybenzylamino)-2-(2-methylphenyl)-piperidine;
cis-3-(2-methoxybenzylamino)-2-(3-methoxyphenyl)-piperidine;
cis-3-(2-methoxybenzylamino)-2-(3-fluorophenyl)-piperidine;
cis-3-(2-methoxybenzylamino)-2-(3-chlorophenyl)-piperidine;
cis-3-(2-methoxybenzylamino)-2-phenylpiperidine;
cis-3-(2-methoxybenzylamino)-2-(3-methylphenyl)-piperidine;
cis-3-(2-methoxybenzylamino)-2-(4-fluorophenyl)-piperidine;
cis-3-(2-methoxybenzylamino)-2-(3-thienyl)-piperidine;
cis-3-(2-methoxybenzylamino)-2-phenylazacyclo-heptane;
3-(2-methoxybenzylamino)-4-methyl-2-phenylpiperidine;
3-(2-methoxybenzylamino)-5-methyl-2-phenylpiperidine;
3-(2-methoxybenzylamino)-6-methyl-2-phenylpiperidine;
(2S,3S)-3-(2-methoxybenzylamino)-2-phenylpiperidine;
(2S,3S)-1-(5-carboethoxypent-1-yl)-3-(2-methoxybenzylamino)-2-phenylpiperidine;
(2S,3S)-1-(6-hydroxy-hex-1-yl)-3-(2-methoxybenzylamino)-2-phenylpiperidine;
(2S,3S)-1-(4-hydroxy-4-phenylbut-1-yl)-3-(2-methoxybenzylamino)-2-phenylpiperidine;
(2S,3S)-1-(4-oxo-4-phenylbut-1-yl)-3-(2-methoxybenzylamino)-2-phenylpiperidine;
(2S,3S)-1-(5,6-dihydroxyhex-1-yl)-3-(2-methoxybenzylamino)-2-phenylpiperidine;
cis-3-(5-fluoro-2-methoxybenzylamino)-2-phenylpiperidine;
(2S,3S)-1-[4-(4-fluorophenyl)-4-oxobut-1-yl]-3-(2-methoxybenzylamino)-2-phenylpiperidine;
(2S,3S)-1-[4-[4-fluorophenyl)-4-hydroxybut-1-yl]-3-(2-methoxybenzylamino)-2-phenylpiperidine;
cis-3-(2-methoxy-5-methylbenzylamino)-2-phenylpiperidine;
(2S,3S)-1-(4-benzamidobut-1-yl)-3-(2-methoxybenzylamino)-2-phenylpiperidine;
cis-3-(2-methoxynaphth-1-yl-methylamino)-2-phenylpiperidine;
(2S,3S)-3-(2-methoxybenzylamino)-1-(5-N-methylcarboxamidopent-1-yl)-2-phenylpiperidine;
(2S,3S)-1-(4-cyanobut-1-yl)-3-(2-methoxybenzylamino)-2-phenylpiperidine;
(2S,3S)-1-[4(2-naphthamido)but-1-yl]-3-(2-methoxybenzylamino)-2-phenylpiperidine;
(2S,3S)-1-(5-benzamidopent-1-yl)-3-(2-methoxybenzylamino)-2-phenylpiperidine;
(2S,3S)-1-(5-aminopent-1-yl)-3-(2-methoxybenzylamino)-2-phenylpiperidine;
(2S,3S)-3-(5-chloro-2-methoxybenzylamino)-2-phenylpiperidine;
(2S,3S)-3-(2,5-dimethoxybenzylamino)-2-phenylpiperidine;
cis-3-(3,5-difluoro-2-methoxybenzylamino)-2-phenylpiperidine;
cis-3-(4,5-difluoro-2-methoxybenzylamino)-2-phenylpiperidine;
cis-3-(2,5-dimethoxybenzylamino)-1-[4-(4-fluorophenyl)-4-oxobut-1-yl]-2-phenylpiperidine;
cis-3-(5-chloro-2-methoxybenzylamino)-1-(5,6-dihydroxyhex-1-yl)-2-phenylpiperidine;
cis-1-(5,6-dihydroxyhex-1-yl)-3-(2,5-dimethoxybenzylamino)-2-phenylpiperidine;
cis-2-phenyl-3-[-2(prop-2-yloxy)benzylamino]piperidine;
cis-3-(2,5-dimethoxybenzyl)amino-2-(3-methoxyphenyl)piperidine hydrochloride;
cis-3-(5-chloro-2-methoxybenzyl)amino-2-(3-methoxyphenyl)piperidine dihydrochloride;
cis-3-(5-chloro-2-methoxybenzyl)amino-2-(3-chlorophenyl)piperidine dihydrochloride;
3-(2-methoxybenzylamino)-2,4-diphenylpiperidine;
cis-3-(2-methoxybenzylamino)-2-phenylpyrrolidine;
(2S,3S)-3-(5-ethyl-2-methoxybenzyl)amino-2-phenylpiperidine;
(2S,3S)-3-(5-n-butyl-2-methoxybenzyl)amino-2-phenylpiperidine;
(2S,3S)-3-(2-methoxy-5-n-propylbenzyl)amino-2-phenylpiperidine;
(2S,3S)-3-(5-isopropyl-2-methoxybenzyl)amino-2-phenylpiperidine;
(2S,3S)-3-(5-s-butyl-2-methoxybenzyl)amino-2-phenylpiperidine;
(2S,3S)-3-(5-t-butyl-2-methoxybenzyl)amino-2-phenylpiperidine; and
(2S,3S)-3-(2-methoxy-5-phenylbenzyl)amino-2-phenylpiperidine.

12. The use according to claim 2, wherein the compound is selected from the group consisting of:
(2S,3S)-N-(5-isopropyl-2-methoxyphenyl)methyl-2-diphenylmethyl-1-azabicyclo[2.2.2]octan-3-amine;
(2S,3S)-N-(5-tert-butyl-2-methoxyphenyl)methyl-2-diphenylmethyl-1-azabicyclo[2.2.2]octan-3-amine;
(2S,3S)-N-(5-methyl-2-methoxyphenyl)methyl-2-diphenylmethyl-1-azabicyclo[2.2.2]octan-3-amine;
(2S,3S)-N-(5-ethyl-2-methoxyphenyl)methyl-2-diphenylmethyl-1-azabicyclo[2.2.2]octan-3-amine;
(2S,3S)-N-(5-isopropyl-2-methoxyphenyl)methyl-2-diphenylmethyl-1-azabicyclo[2.2.2]octan-3-amine;
(2S,3S)-N-(5-sec-butyl-2-methoxyphenyl)methyl-2-diphenylmethyl-1-azabicyclo[2.2.2]octan-3-amine; and
(2S,3S)-N-(5-n-propyl-2-methoxyphenyl)methyl-2-diphenylmethyl-1-azabicyclo[2.2.2]octan-3-amine,
2,4-dimethylthiazole-5-sulfonic acid [4-methoxy-3-((2S,3S)-2-phenylpiperidin-3-ylaminomethyl)phenyl]-methylamide;
N-(4,5-dimethylthiazol-2-yl)-N-[4-methoxy-3-((2S,3S)-2-phenylpiperidin-3-yl-aminomethyl)phenyl]-methanesulfonamide;
{5-[(4,5-dimethylthiazol-2-yl)methylamino]-2-methoxybenzyl)-((2S,3S)-2-phenylpiperidin-3-yl)amine;
{5-(4,5-dimethylthiazol-2-ylamino)-2-methoxybenzyl}-((2S,3S)-2-phenylpiperidin-3-ylamine;
4,5-dimethylthiazole-2-sulfonic acid methyl-[3-((2S,3S)-2-phenylpiperidin-3-ylaminomethyl)-4-trifluoromethoxyphenyl]-amide;
2,4-dimethylthiazole-5-sulfonic acid [4-isopropoxy-3-((2S,3S)-2-phenylpiperidin-3-ylaminomethyl)phenyl]-methylamide;
2,4-dimethylthiazole-5-sulfonic acid [4-isopropoxy-3-((2S,3S)-2-phenylpiperidin-3-ylaminomethyl)phenyl]-isopropylamide;
2,4-dimethylthiazole-5-sulfonic acid [4-methoxy-3-((2S,3S)-2-phenylpiperidin-3-ylaminomethyl)phenyl]-isopropylamide;
2,4-dimethylthiazole-5-sulfonic acid [4-methoxy-3-((2S,3S)-2-phenylpiperidin-3-ylaminomethyl)phenyl]-isobutylamide; and
2,4-dimethylthiazole-5-sulfonic acid [4-isopropoxy-3-((2S,3S)-2-phenylpiperidin-3-ylaminomethyl)phenyl]-isobutylamide.
or a pharmaceutically acceptable salt thereof.

13. The use according to claim 3, wherein the compound is selected from the group consisting of:
(3R,4S,5S,6S)-N,N-diethyl-5-(5-isopropyl-2-methoxybenzylamino)-6-diphenylmethyl-1-azabicyclo[2.2.2]octane-3-carboxamide;
(3R,4S,5S,6S)-N,N-diethyl-5-(2,5-dimethoxybenzylamino)-6-diphenylmethyl-1-azabicyclo[2.2.2]octane-3-carboxamide;
(3R,4S,5S,6S)-5-(5-isopropyl-2-methoxybenzylamino)-6-diphenylmethyl-1-azabicyclo[2.2.2]octane-3-carboxaylic acid;
(3R,4S,5S,6S)-5-(2-methoxy-2-methylthiobenzylamino)-6-diphenylmethyl-1-azabicyclo[2.2.2]octane-3-carboxylic acid;
(3R,4S,5S,6S)-5-(2,5-dimethoxybenzylamino)-6-diphenylmethyl-1-azabicyclo-[2.2.2]octane-3-carboxylic acid;
(3R,4S,5S,6S)-5-(2-methoxy-5-methylbenzylamino)-6-diphenylmethyl-1-azabicyclo[2.2.2]octane-3-carboxylic acid;
(3R,4S,5S,6S)-5-(5-ethyl-2-methoxybenzylamino)-6-diphenylmethyl-1-azabicyclo[2.2.2]octane-3-carboxylic acid;
(3R,4S,5S,6S)-5-(2-methoxyl-5-n-propylbenzylamino)-6-diphenylmethyl-1-azabicyclo[2.2.2]octane-3-carboxylic acid;
(3R,4S,5S,6S)-5-(5-sec-butyl-2-methoxybenzylamino)-6-diphenylmethyl-1-azabicyclo[2.2.2]octane-3-carboxylic acid;
(3R,4S,5S,6S)-5-(5-N-methyl-methanesulfonylamino-2-methoxy-benzylamino)-6-diphenylmethyl-1-azabicyclo[2.2.2]octane-3-carboxylic acid;
(3R,4S,5S,6S)-5-(2-methoxy-5-methylsulfinylbenzylamino)-6-diphenylmethyl-1-azabicyclo[2.2.2]octane-3-carboxylic acid;
(3R,4S,5S,6S)-5-(2-methoxy-5-trifluoromethoxybenzylamino)-6-diphenylmethyl-1-azabicyclo[2.2.2]octane-3-carboxylic acid;
(3R,4S,5S,6S)-5-(2-methoxy-5-methylsulfonylbenzylamino)-6-diphenylmethyl-1-azabicyclo[2.2.2]octane-3-carboxylic acid;
(3R,4S,5S,6S)-5-(5-dimethylamino-2-methoxybenzylamino)-6-diphenylmethyl-1-azabicyclo[2.2.2]octane-3-carboxylic acid;
(3R,4S,5S,6S)-5-(5-isopropyl-2-methoxybenzylamino)-6-diphenylmethyl-1-azabicyclo[2.2.2]octane-2-carboxylic acid;
(3R,4S,5S,6S)-5-(2-methoxy-5-methylthiobenzylamino)-6-diphenylmethyl-1-azabicyclo[2.2.2]octane-2-carboxylic acid;
(3R,4S,5S,6S)-5-(2,5-dimethoxybenzylamino)-6-diphenylmethyl-1-azabicyclo[2.2.2]octane-2-carboxylic acid;
(3R,4S,5S,6S)-5-(2-methoxy-5-methylbenzylamino)-6-diphenylmethyl-1-azabicyclo[2.2.2]octane-2-carboxylic acid;
(3R,4S,5S,6S)-5-(5-ethyl-2-methoxybenzylamino)-6-diphenylmethyl-1-azabicyclo[2.2.2]octane-2-carboxylic acid;
(3R,4S,5S,6S)-5-(2-methoxyl-5-n-propylbenzylamino)-6-diphenylmethyl-1-azabicyclo[2.2.2]octane-2-carboxylic acid;
(3R,4S,5S,6S)-5-(5-sec-butyl-2-methoxybenzylamino)-6-diphenylmethyl-1-azabicyclo[2.2.2]octane-2-carboxylic acid;
(3R,4S,5S,6S)-5-(5-N-methyl-methanesulfonylamino-2-methoxybenzyl-amino)-6-diphenylmethyl-1-azabicyclo[2.2.2]octane-2-carboxylic acid;
(3R,4S,5S,6S)-5-(2-methoxy-5-methylsulfinylbenzylamino)-6-diphenylmethyl-1-azabicyclo[2.2.2]octane-2-carboxylic acid;
(3R,4S,5S,6S)-5-(2-methoxy-5-trifluoromethoxybenzylamino)-6-diphenylmethyl-1-azabicyclo[2.2.2]octane-2-carboxylic acid;
(3R,4S,5S,6S)-5-(2-methoxy-5-methylsulfonylbenzylamino)-6-diphenylmethyl-1-azabicyclo[2.2.2]octane-2-carboxylic acid; and
(3R,4S,5S,6S)-5-(5-dimethylamino-2-methoxybenzylamino)-6-diphenylmethyl-1-azabicyclo[2.2.2]octane-2-carboxylic acid.

14. The use of a compound that is a substance P receptor antagonist, except (2S,3S)-N-(2-methoxyphenyl)methyl-2-diphenylmethyl-1-azabicyclo[2.2.2]octan-3-amine, or a pharmaceutically acceptable salt thereof, for the manufacture of a medicament for the treatment or prevention of sunburn in a mammal.

15. A topical pharmaceutical composition comprising a compound according to any one of claims 1 to 14 and a pharmaceutically acceptable carrier for the manufacture of a medicament for the treatment or prevention of sunburn in a mammal.
